# EUROPEAN PATENT APPLICATION

(11) **EP 4 424 381 A2**
(43) Date of publication of application: **04.09.2024**
(21) Application number: 24175051.2
(22) Date of filing: 16.10.2020
(51) Int. Cl.: A61P 37/02

(54) **SAFE AND EFFECTIVE METHOD OF TREATING ULCERATIVE COLITIS WITH ANTI-IL12/IL23 ANTIBODY**

(30) Priority: 18.10.2019 US 201962916984 P
(62) Divisional of application: 20877088.3
(71) Applicant: Janssen Biotech, Inc., Horsham, PA 19044 (US)
(72) Inventor: ADEDOKUN, Omoniyi, Spring House, 19477 (US); JOHANNS, Jewel, Spring House, 19477 (US); LI, Katherine, Spring House, 19477 (US); MARANO, Colleen, Spring House, 19477 (US); O'BRIEN, Christopher, Lafayette Hill, 19444 (US); SHIELDS-TUTTLE, Kimberly, Spring House, 19477 (US); STRAUSS, Richard, Spring House, 19477 (US); ZHANG, Hongyan, Wayne, 19087 (US)
(74) Representative: Carpmaels & Ransford LLP

(57) **Abstract**

Described are methods and compositions for clinically proven safe and effective treatment of ulcerative colitis according to the product label described herein, particularly moderately to severely active ulcerative colitis in patients who have had an inadequate response to or are intolerant of a conventional or existing therapy by intravenous and/or subcutaneous administration of an anti-IL-12/IL-23p40 antibody.

## Description

### REFERENCE TO SEQUENCE LISTING SUBMITTED ELECTRONICALLY

This application contains a sequence listing, which is submitted electronically via EFS-Web as an ASCII formatted sequence listing with a file name
"JBI6165WOPCT1SequenceListing.txt" creation date of October 15, 2020, and is 15 kilobytes in size. The sequence listing submitted via EFS-Web is part of the specification and is herein incorporated by reference in its entirety.

### FIELD OF THE INVENTION

The invention relates to methods of providing a clinically proven safe and clinically proven effective treatment of ulcerative colitis, particularly moderately to severely active ulcerative colitis in patients who have had an inadequate response to or are intolerant of a conventional or existing therapy by intravenous and/or subcutaneous administration of an anti-IL-12/IL-23p40 antibody.

### BACKGROUND OF THE INVENTION

Inflammatory bowel diseases (IBDs), including ulcerative colitis (UC), are chronic relapsing disorders characterized by destructive inflammation and epithelial injury in the gastrointestinal (GI) tract (Baumgart and Sandborn, J Clin Invest. 98:1010-1020 (1996); Danese and Fiocchi, N Engl J Med. 365:1715-1725 (2011)). The incidence of UC in the United States is estimated to be between 9 and 12 per 100,000 persons with a prevalence of 205 to 240 per 100,000 persons (Tally et al., Am J Gastroenterol. 106 Suppl 1: S2-S25 (2011)). The estimate of the prevalence of UC in Europe is approximately 1 million people (Loftus, Gastroenterology. 126(6):1504-1517 (2004); Loftus, Gastoenterol Clin N Am.31:1-20 (2002)). The etiology of UC is unknown. However, abnormal immune responses to contents in the gut, including intestinal microbes, are thought to drive disease in genetically predisposed individuals (Geremia et al., Autoimmun Rev. 13:3-10 (2014)). Dysregulated innate and adaptive immune pathways contribute to aberrant intestinal inflammation in IBD, and cytokines, including interleukin (IL)-12, interferon-gamma (IFNy), and IL-23 have been implicated in the pathogenesis of UC (Geremia et al., Autoimmune Rev. 2014; 13:3-10; Neurath, Nat Rev Immunol. 14(5):329-42 (2014)).

The involvement of the IL-12/23 pathway in the pathogenesis of IBD is well established, and an important role for IL-12/IL-23 pathway in intestinal inflammation has been elucidated in colitis (Ahern et al., Immunity. 33(2):279-288 (2010); Investigator's Brochure: STELARA^{®} (ustekinumab), edition 18. Janssen Research & Development, LLC (2017); Uhlig et al., Immunity. 25:309 318 (2006); Yen et al., J Clin Invest. 116(5):1310-1316 (2006)). Early studies showed that treatment with anti-IFNy (Berg et al., J Clin Invest. 98: 1010-1020 (1996); Davidson et al., J Immunol. 161:3143-3149 (1998)) or anti-IL-12p40 monoclonal antibodies (mAb) prevented disease in experimental colitis models, suggesting an important role for type 1 T helper (Th-1) cells in promoting intestinal inflammation (Neurath et al., J Exp Med. 182(5):1281-1290 (1995)). Genome-wide association studies have implicated several genetic loci in humans in the IL-12/23 pathway that are associated with increased susceptibility to UC, including IL-23R and IL-12B (Anderson et al., Nat Genet. 43(3):246-252 (2011); Brant et al., Clin Gastroenterol Hepatol. 11(1):22-26 (2013)). Subjects with active UC were shown to have significantly more IL-23, IL-22, IL-22R1 and p-STAT3-positive cells than subjects with inactive UC and normal controls (Yu et al., World J Gastroenterol. 19(17):2638-2649 (2013)).

Biologic therapies currently approved for the treatment of UC are either tumor necrosis factor (TNF) or integrin inhibitors (Colombel et al., Gastroenterology. 132:52-65 (2007); Hanauer et al., Lancet. 359:1541-1549 (2002); Sandborn et al., N Engl J Med. 369:711-721 (2013); Sandborn et al., Gastroenterology. 142:257-265 (2012)). However, only 1 therapy of all currently approved treatments, vedolizumab, has demonstrated efficacy in subjects who have had an inadequate response to (i.e., primary nonresponse or secondary loss of response) or are intolerant of anti-TNFs (Feagan et al., N Engl J Med. 369:699 710 (2013)). Anti-TNFs have safety risks associated with immunosuppression and not all subjects adequately respond to such therapy. Furthermore, as was observed with the anti-TNFs, inadequate response, and intolerance has been identified in subjects receiving vedolizumab for the treatment of their UC. Therefore, there remains an unmet need for novel therapies with alternative mechanisms of action.

When tested, biologic therapies that are currently approved for the treatment of UC have also demonstrated efficacy in Crohn's disease (Sandborn et al., Gastroenterology. 135(4):1130-1141 (2008)). Multiple lines of evidence suggest that inflammatory bowel disease (UC and Crohn's disease) is mediated by Th1 or Th17 cells with strong contribution from the proinflammatory cytokines, IL-12, and IL-23. Ustekinumab (STELARA^{®}) is a fully human immunoglobulin G1 mAb to human IL-12/23p40 that prevents IL-12 and IL-23 bioactivity by inhibiting their interaction with their cell surface IL-12Rβ1 receptor protein (Investigator's Brochure: STELARA^{®} (ustekinumab), edition 18. Janssen Research & Development, LLC (2017)). Through this mechanism of action, ustekinumab effectively neutralizes IL-12 (Th1)- and IL-23 (Th17)-mediated cellular responses. Ustekinumab has received marketing approval globally, including countries in North America, Europe, South America, and the Asia-Pacific region, for the treatment of adult subjects with moderately to severely active Crohn's disease (the first approval for Crohn's disease was received on 11 November 2016), moderate to severe plaque psoriasis, or active psoriatic arthritis, as well as for pediatric subjects (12 to 17 years old) with moderate to severe plaque psoriasis.

The efficacy and safety of intravenous (IV) ustekinumab as induction therapy in Crohn's disease have been evaluated in clinical studies CRD3001 and CRD3002. In study CRD3001, subjects with demonstrated prior failure or intolerance to one or more TNF antagonists were evaluated, and in CRD3002 subjects with history of inadequate response to or intolerance of corticosteroids or immunomodulators, but without a history of an inadequate response or intolerance to TNF antagonists were evaluated. In these studies, two IV doses were evaluated: a 130 mg IV fixed dose (-2 mg/kg on a mg/kg basis) was chosen for the low-dose group, while body-weight range based doses approximating ~6 mg/kg IV (weight ≤55 kg: ustekinumab 260 mg; weight >55 and ≤85 kg: ustekinumab 390 mg; weight >85 kg: ustekinumab: 520 mg) were chosen as the high-dose group. In both studies, ustekinumab demonstrated clinically significant efficacy compared with placebo and was well-tolerated with a favorable safety profile.

Prior to the present invention, no studies had been conducted with ustekinumab for UC. there is a need in the art for improved methods of treating UC, particularly moderately to severely active UC, in subjects who had previously failed or were intolerant of a biologic therapy or other conventional therapy, or subjects who had demonstrated corticosteroid dependence.

### BRIEF SUMMARY OF THE INVENTION

The present application relates to clinically proven safe and clinically proven effective methods and compositions for treatment of moderately to severely active ulcerative colitis (UC), particularly in subjects who have had an inadequate response to or are intolerant of a conventional or existing therapy, by administration of an anti-IL-12/IL-23p40 antibody to subjects, thereby addressing a clear unmet medical need in this subject population.

In an embodiment of the invention, a pharmaceutical composition comprises an antibody comprising: (i) a heavy chain variable region and a light chain variable region, the heavy chain variable region comprising: a complementarity determining region heavy chain 1 (CDRH1) amino acid sequence of SEQ ID NO:1; a CDRH2 amino acid sequence of SEQ ID NO:2; and a CDRH3 amino acid sequence of SEQ ID NO:3; and the light chain variable region comprising: a complementarity determining region light chain 1 (CDRL1) amino acid sequence of SEQ ID NO:4; a CDRL2 amino acid sequence of SEQ ID NO:5; and a CDRL3 amino acid sequence of SEQ ID NO:6; (ii) a heavy chain variable region of the amino acid sequence of SEQ ID NO:7 and a light chain variable region of the amino acid sequence of SEQ ID NO:8; or (iii) a heavy chain of the amino acid sequence of SEQ ID NO: 10 and a light chain of the amino acid sequence of SEQ ID NO:11; and packaging comprising one or more drug product label elements disclosed in Annex I including data from a randomized, double-blind, placebo-controlled, clinical study in adult men and women with moderately to severely active ulcerative colitis (UC).

In one general aspect, the application relates to a clinically proven safe and clinically proven effective method of treating moderately to severely active ulcerative colitis (UC) in a subject in need thereof, comprising administering to the subject a pharmaceutical composition comprising a safe and effective amount of an anti-IL-12/IL-23p40 antibody, comprising: (i) a heavy chain variable region and a light chain variable region, the heavy chain variable region comprising: a complementarity determining region heavy chain 1 (CDRH1) amino acid sequence of SEQ ID NO:1; a CDRH2 amino acid sequence of SEQ ID NO:2; and a CDRH3 amino acid sequence of SEQ ID NO:3; and the light chain variable region comprising: a complementarity determining region light chain 1 (CDRL1) amino acid sequence of SEQ ID NO:4; a CDRL2 amino acid sequence of SEQ ID NO:5; and a CDRL3 amino acid sequence of SEQ ID NO:6; (ii) a heavy chain variable region of the amino acid sequence of SEQ ID NO:7 and a light chain variable region of the amino acid sequence of SEQ ID NO: 8; or (iii) a heavy chain of the amino acid sequence of SEQ ID NO:10 and a light chain of the amino acid sequence of SEQ ID NO:11; and packaging comprising one or more drug product label elements disclosed in Annex I including data from a randomized, double-blind, placebo-controlled, clinical study in adult men and women with moderately to severely active ulcerative colitis (UC).In certain embodiments, the anti-IL-12 and/or anti-IL-23 antibody is administered intravenously to the subject, preferably at week 0, at a dosage of about 6.0 mg/kg body weight of the subject or 130 mg per administration.

In certain embodiments, the anti-IL-12 and/or anti-IL-23 antibody is administered intravenously or subcutaneously to the subject, preferably at week 8, at a dosage of about 6.0 mg/kg body weight of the subject or 90 mg per administration, respectively.

Preferably, the subject treated by methods according to embodiments of the application has had an inadequate response to or are intolerant of a conventional or existing therapy. In some embodiments, the subject had previously failed or were intolerant of a biologic therapy, such as an anti-TNF and/or vedolizumab. In some embodiments, the subject had previously failed or were intolerant of a non-biologic therapy, such as a treatment with corticosteroids, azathioprine (AZA), and/or 6 mercaptopurine (6 MP). In some embodiments, the subject had demonstrated corticosteroid dependence.

In another general aspect, the application relates to a clinically proven safe and clinically proven effective method of treating moderately to severely active ulcerative colitis (UC) in a subject in need thereof, comprising:
intravenously administering to the subject a pharmaceutical composition comprising an anti-IL-12/IL-23p40 antibody at a dosage of about 6.0 mg/kg body weight of the subject or 130 mg of the antibody per administration at week 0 of the treatment, and
subcutaneously administering to the subject a pharmaceutical composition comprising the anti-IL-12/IL-23p40 antibody at a dosage of 90 mg of the antibody per administration at week 8 of the treatment,
wherein the antibody comprises a heavy chain variable region and a light chain variable region, the heavy chain variable region comprising: a complementarity determining region heavy chain 1 (CDRH1) amino acid sequence of SEQ ID NO:1; a CDRH2 amino acid sequence of SEQ ID NO:2; and a CDRH3 amino acid sequence of SEQ ID NO:3; and the light chain variable region comprising: a complementarity determining region light chain 1 (CDRL1) amino acid sequence of SEQ ID NO:4; a CDRL2 amino acid sequence of SEQ ID NO:5; and a CDRL3 amino acid sequence of SEQ ID NO:6; and
wherein the subject had previously failed or were intolerant of at least one therapy selected from the group consisting of: an anti-TNF, vedolizumab, corticosteroids, azathioprine (AZA), and 6 mercaptopurine (6 MP), or the subject had demonstrated corticosteroid dependence

In certain embodiments, methods of the present application comprise intravenously (IV) and/or subcutaneously (SC) administering to the subject a pharmaceutical composition comprising an anti-IL-12 and/or anti-IL-23 antibody or antigen binding fragment comprising: (i) a heavy chain variable domain amino acid sequence of SEQ ID NO:7; and (ii) a light chain variable domain amino acid sequence of SEQ ID NO:8.

In certain embodiments, methods of the present application comprise intravenously (IV) and/or subcutaneously (SC) administering to the subject a pharmaceutical composition comprising the anti-IL-12/23p40 antibody ustekinumab, which comprises: (i) a heavy chain amino acid sequence of SEQ ID NO:10; and (ii) a light chain amino acid sequence of SEQ ID NO:11.

In certain embodiments, the IV dose at week 0 is about 6.0 mg/kg. For example, the IV dose is 260 mg for subjects with body weight ≥35 kg and ≤55 kg, 390 mg for subjects with body weight >55 kg and ≤85 kg, and 520 mg for subjects with body weight >85 kg.

In certain embodiments, the subject is a responder to a treatment of a method according to an embodiment of the application and is identified as having at least one of: (1) a clinical remission based on at least one of the global submissions and the US submissions; (2) an endoscopic healing; (3) a clinical response; (4) a change from baseline in Inflammatory Bowel Disease Questionnaire (IBDQ) score; (5) a mucosal healing; (6) a decrease from baseline in Mayo score; and (7) a normalization of one or more biomarkers selected from the group consisting of C-reactive protein, fecal lactoferrin and fecal calprotectin. Preferably, at least one of (1) to (7) above is identified from the subject by week 16, more preferably by week 8 or week 4, and most preferably by week 2 of the treatment.

In certain embodiments, the present invention provides a clinically proven safe and clinically proven effective method of treating moderately to severely active UC in a subject, wherein the subject is a responder to the treatment with the antibody and is identified as having a statistically significant improvement in disease activity as determined by endoscopic healing with a Mayo endoscopy subscore of 0 or 1 by week 8 of treatment with the antibody.

In other embodiments, the present invention provides a clinically proven safe and clinically proven effective method of treating moderately to severely active UC in a subject, wherein the subject is a responder to the treatment with the antibody and is identified as having a statistically significant improvement in disease activity as determined by an Ulcerative Colitis Endoscopic Index of Severity (UCEIS) score of ≤4 by week 8 of treatment with the antibody.

In certain embodiments, the subject is in clinical response as determined by a decrease from baseline in the Mayo score by ≥30% and ≥3 points and a decrease from baseline in the rectal bleeding subscore ≥1 points or a rectal bleeding subscore of 0 or 1 by week 8 of treatment with the antibody.

In other embodiments, a maintenance dose of the anti-IL-12/IL-23p40 antibody is administered every 8 weeks after the treatment at week 8 or every 12 weeks after the treatment at week 8 and clinical response is maintained by the subject for at least 44 weeks.

In certain embodiments, the present invention provides a clinically proven safe and clinically proven effective method of treating moderately to severely active UC in a subject, wherein a subject identified as a non-responder to an initial treatment is administered a second treatment, preferably with an administration route different from the initial treatment. For example, a subject identified as a non-responder to an initial treatment with an IV administration of an antibody or antibody binding fragment can be treated with a subsequent subcutaneous administration of the antibody or antibody binding fragment according to embodiments of the invention.

In certain embodiments, the present application provides for a method of treating moderately to severely active UC in a subject, wherein an anti-IL-12 and/or anti-IL-23 antibody for use with IV administration is in a pharmaceutical composition comprising a solution comprising 10 mM L-histidine, 8.5% (w/v) sucrose, 0.04% (w/v) polysorbate 80, 0.4 mg/mL L methionine, and 20 µg/mL EDTA disodium salt, dehydrate, at pH 6.0.

In certain embodiments, the present application provides for a clinically proven safe and clinically proven effective method of treating moderately to severely active UC in a subject, wherein an anti-IL-12 and/or anti-IL-23 antibody for use with subcutaneous administration is in a pharmaceutical composition comprising a solution comprising 6.7 mM L-histidine, 7.6% (w/v) sucrose, 0.004% (w/v) polysorbate 80, at pH 6.0.

In certain embodiments, the present application provides a method further comprising administering to the subject one or more additional drugs used to treat UC. In a preferred embodiment, the additional drug is selected from the group consisting of: oral 5-aminosalicylate (5-ASA) compounds, oral corticosteroids, immunomodulators, 6-mercaptopurine (6-MP), azathioprine (AZA), or methotrexate (MTX).

Other aspects of the application include pharmaceutical compositions comprising an anti-IL-12 and/or anti-IL-23 antibody for use in a clinically proven safe and clinically proven effective method of treating moderately to severely active UC in a subject, as well as methods of preparing the compositions and kits comprising the pharmaceutical compositions.

In certain embodiments, a kit useful for a method of the invention comprises at least one of a pharmaceutical composition for intravenous administration of the invention and pharmaceutical composition for subcutaneous administration of the invention. In other embodiments, the kit comprises both a pharmaceutical composition for intravenous administration and a pharmaceutical composition for subcutaneous administration of the invention.

### BRIEF DESCRIPTION OF THE DRAWINGS

The foregoing summary, as well as the following detailed description of the invention, will be better understood when read in conjunction with the appended drawings. It should be understood that the invention is not limited to the precise embodiments shown in the drawings.

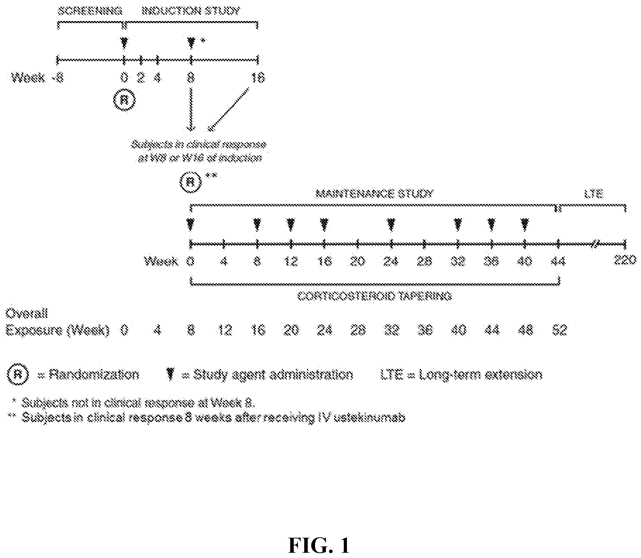

FIG. 1 shows a diagrammatic representation of the study design. Abbreviations: W8= Week 8; W16= Week 16; LTE= Long-term Extension.

### DETAILED DESCRIPTION OF THE INVENTION

Various publications, articles and patents are cited or described in the background and throughout the specification; each of these references is herein incorporated by reference in its entirety. Discussion of documents, acts, materials, devices, articles or the like which has been included in the present specification is for the purpose of providing context for the invention. Such discussion is not an admission that any or all of these matters form part of the prior art with respect to any inventions disclosed or claimed.

Unless defined otherwise, all technical and scientific terms used herein have the same meaning as commonly understood to one of ordinary skill in the art to which this invention pertains. Otherwise, certain terms used herein have the meanings as set forth in the specification. All patents, published patent applications and publications cited herein are incorporated by reference as if set forth fully herein.

It must be noted that as used herein and in the appended claims, the singular forms "a," "an," and "the" include plural reference unless the context clearly dictates otherwise. Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of' excludes any element, step, or ingredient not specified in the claim element. When used herein, "consisting essentially of' does not exclude materials or steps that do not materially affect the basic and novel characteristics of the claim. Any of the aforementioned terms of "comprising", "containing", "including", and "having", whenever used herein in the context of an aspect or embodiment of the invention can be replaced with the term "consisting of" or "consisting essentially of" to vary scopes of the disclosure.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

As used herein, "subject" means any animal, preferably a mammal, most preferably a human, whom will be or has been treated by a method according to an embodiment of the invention. The term "mammal" as used herein, encompasses any mammal. Examples of mammals include, but are not limited to, cows, horses, sheep, pigs, cats, dogs, mice, rats, rabbits, guinea pigs, non-human primates (NHPs) such as monkeys or apes, humans, etc., more preferably a human.

As used herein, the term "in combination", in the context of the administration of two or more therapies to a subject, refers to the use of more than one therapy. The use of the term "in combination" does not restrict the order in which therapies are administered to a subject. For example, a first therapy (e.g., a composition described herein) can be administered prior to (e.g., 5 minutes, 15 minutes, 30 minutes, 45 minutes, 1 hour, 2 hours, 4 hours, 6 hours, 12 hours, 16 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g., 5 minutes, 15 minutes, 30 minutes, 45 minutes, 1 hour, 2 hours, 4 hours, 6 hours, 12 hours, 16 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy to a subject.

As used herein, an "anti-IL-12 antibody," "anti-IL-23 antibody," "anti-IL-12/23p40 antibody," or "IL-12/23p40 antibody," refers to a monoclonal antibody (mAb) or antigen binding fragment thereof, that binds the 40 kDa (p40) subunit shared by the cytokines interleukin-12 and interleukin-23 (IL-12/23p40). The antibody can affect at least one of IL-12/23 activity or function, such as but not limited to, RNA, DNA or protein synthesis, IL-12/23 release, IL-12/23 receptor signaling, membrane IL-12/23 cleavage, IL-12/23 activity, IL-12/23 production and/or synthesis.

The term "antibody" is further intended to encompass antibodies, digestion fragments, specified portions and variants thereof, including antibody mimetics or comprising portions of antibodies that mimic the structure and/or function of an antibody or specified fragment or portion thereof, including single chain antibodies and fragments thereof. Functional fragments include antigen-binding fragments that bind to a mammalian IL-12/23. For example, antibody fragments capable of binding to IL-12/23 or portions thereof, including, but not limited to, Fab (e.g., by papain digestion), Fab' (e.g., by pepsin digestion and partial reduction) and F(ab')₂ (e.g., by pepsin digestion), facb (e.g., by plasmin digestion), pFc' (e.g., by pepsin or plasmin digestion), Fd (e.g., by pepsin digestion, partial reduction and reaggregation), Fv or scFv (e.g., by molecular biology techniques) fragments, are encompassed by the invention (see, e.g., Colligan, Immunology, supra).

Such fragments can be produced by enzymatic cleavage, synthetic or recombinant techniques, as known in the art and/or as described herein. Antibodies can also be produced in a variety of truncated forms using antibody genes in which one or more stop codons have been introduced upstream of the natural stop site. For example, a combination gene encoding a F(ab')₂ heavy chain portion can be designed to include DNA sequences encoding the C_{H}1 domain and/or hinge region of the heavy chain. The various portions of antibodies can be joined together chemically by conventional techniques, or can be prepared as a contiguous protein using genetic engineering techniques.

As used herein, the term "human antibody" refers to an antibody in which substantially every part of the protein (e.g., CDR, framework, C_{L}, C_{H} domains (e.g., C_{H}1, C_{H}2, C_{H}3), hinge, (V_{L}, V_{H})) is substantially non-immunogenic in humans, with only minor sequence changes or variations. A "human antibody" can also be an antibody that is derived from or closely matches human germline immunoglobulin sequences. Human antibodies can include amino acid residues not encoded by germline immunoglobulin sequences (e.g., mutations introduced by random or site-specific mutagenesis in vitro or by somatic mutation in vivo). Often, this means that the human antibody is substantially non-immunogenic in humans. Human antibodies have been classified into groupings based on their amino acid sequence similarities. Accordingly, using a sequence similarity search, an antibody with a similar linear sequence can be chosen as a template to create a human antibody. Similarly, antibodies designated primate (monkey, baboon, chimpanzee, etc.), rodent (mouse, rat, rabbit, guinea pig, hamster, and the like) and other mammals designate such species, sub-genus, genus, sub-family, and family specific antibodies. Further, chimeric antibodies can include any combination of the above. Such changes or variations optionally and preferably retain or reduce the immunogenicity in humans or other species relative to non-modified antibodies. Thus, a human antibody is distinct from a chimeric or humanized antibody.

It is pointed out that a human antibody can be produced by a non-human animal or prokaryotic or eukaryotic cell that is capable of expressing functionally rearranged human immunoglobulin (e.g., heavy chain and/or light chain) genes. Further, when a human antibody is a single chain antibody, it can comprise a linker peptide that is not found in native human antibodies. For example, an Fv can comprise a linker peptide, such as two to about eight glycine or other amino acid residues, which connects the variable region of the heavy chain and the variable region of the light chain. Such linker peptides are considered to be of human origin.

Anti-IL-12/23p40 antibodies (also termed IL-12/23p40 antibodies) (or antibodies to IL-23) useful in the methods and compositions of the present invention can optionally be characterized by high affinity binding to IL-12/23p40, optionally and preferably, having low toxicity. In particular, an antibody, specified fragment or variant of the invention, where the individual components, such as the variable region, constant region and framework, individually and/or collectively, optionally and preferably possess low immunogenicity, is useful in the present invention. The antibodies that can be used in the invention are optionally characterized by their ability to treat subjects for extended periods with measurable alleviation of symptoms and low and/or acceptable toxicity. Low or acceptable immunogenicity and/or high affinity, as well as other suitable properties, can contribute to the therapeutic results achieved. "Low immunogenicity" is defined herein as raising significant HAHA, HACA or HAMA responses in less than about 75%, or preferably less than about 50% of the subjects treated and/or raising low titres in the subject treated (less than about 300, preferably less than about 100 measured with a double antigen enzyme immunoassay) (Elliott et al., Lancet 344:1125-1127 (1994), entirely incorporated herein by reference). "Low immunogenicity" can also be defined as the incidence of titrable levels of antibodies to the anti-IL-12 antibody in subjects treated with anti-IL-12 antibody as occurring in less than 25% of subjects treated, preferably, in less than 10% of subjects treated with the recommended dose for the recommended course of therapy during the treatment period.

The terms "clinically proven efficacy" and "clinically proven effective" as used herein in the context of a dose, dosage regimen, treatment or method refer to the effectiveness of a particular dose, dosage or treatment regimen. Efficacy can be measured based on change in the course of the disease in response to an agent of the present invention. For example, an anti-IL12/23p40 of the present invention (e.g., ustekinumab) is administered to a subject in an amount and for a time sufficient to induce an improvement, preferably a sustained improvement, in at least one indicator that reflects the severity of the disorder that is being treated. Various indicators that reflect the extent of the subject's illness, disease or condition can be assessed for determining whether the amount and time of the treatment is sufficient. Such indicators include, for example, clinically recognized indicators of disease severity, symptoms, or manifestations of the disorder in question. The degree of improvement generally is determined by a physician, who can make this determination based on signs, symptoms, biopsies, or other test results, and who can also employ questionnaires that are administered to the subject, such as quality-of-life questionnaires developed for a given disease. For example, an anti-IL12/23p40 or anti-IL23 antibody of the present invention can be administered to achieve an improvement in a subject's condition related to ulcerative colitis.

Improvement can be indicated by an improvement in an index of disease activity, by amelioration of clinical symptoms or by any other measure of disease activity. Once such index of disease is the ulcerative colitis Mayo score. The Mayo score is an established, validated disease activity index for mild, moderate, and severe ulcerative colitis (UC) that is calculated as the sum of the 4 subscores of stool frequency, rectal bleeding, findings of endoscopy, and physician's global assessment (PGA), and ranges from 0-12. A score of 3 to 5 points indicates mildly active disease, a score of 6 to 10 points indicates moderately active disease, and a score of 11 to 12 points indicates severe disease. The partial Mayo score, which is the Mayo score without the endoscopy subscore, is calculated as the sum of stool frequency, rectal bleeding, and physician's global assessment subscores, and ranges from 0 to 9. The modified Mayo score, which is the Mayo score without the PGA subscore, is calculated as the sum of the stool frequency, rectal bleeding, and endoscopy subscores, and ranges from 0 to 9. Other disease activity indexes for UC include for example, Ulcerative Colitis Endoscopic Index of Severity (UCEIS) score and the Bristol Stool Form Scale (BSFS) score. The UCEIS score provides an overall assessment of endoscopic severity of UC, based on mucosal vascular pattern, bleeding, and ulceration (Travis et al., Gut. 61:535-542 (2012)). The score ranges from 3 to 11 with a higher score indicating more severe disease by endoscopy. The BSFS score is used to classify the form (or consistency) of human feces into 7 categories (Lewis and Heaton, Scand J Gastroenterol. 32(9):920-924 (1997)).

The term "clinical response" as used herein as it relates to a subject's response to drug administration, refers to a decrease from induction baseline in the Mayo score by ≥30% and ≥3 points, with either a decrease from baseline in the rectal bleeding subscore ≥1 or a rectal bleeding subscore of 0 or 1.

The term "clinically proven safe," as it relates to a dose, dosage regimen, treatment or method with anti-IL-12/IL-23p40 antibody of the present invention (e.g., ustekinumab), refers to a favorable risk: benefit ratio with an acceptable frequency and/or acceptable severity of treatment-emergent adverse events (referred to as AEs or TEAEs) compared to the standard of care or to another comparator. As used herein, "adverse event," "treatment-emergent adverse event," and "adverse reaction" mean any harm, unfavorable, unintended or undesired sign or outcome associated with or caused by administration of a pharmaceutical composition or therapeutic. It is an untoward medical occurrence in a subject administered a medicinal product. However, abnormal values or observations are not reported as adverse events unless considered clinically significant by the investigator. As used herein, when referring to an adverse event, "clinically apparent" means clinically significant as determined by a medical doctor or an investigator using standard acceptable to those of ordinary skill in the art. When the harm or undesired outcome of adverse events reaches such a level of severity, a regulatory agency can deem the pharmaceutical composition or therapeutic unacceptable for the proposed use. In particular, "safe" as it relates to a dose, dosage regimen or treatment with an anti-IL12/23p40 or anti-IL23 antibody of the present invention refers to with an acceptable frequency and/or acceptable severity of adverse events associated with administration of the antibody if attribution is considered to be possible, probable, or very likely due to the use of the anti-IL12/23p40 or anti-IL23 antibody.

As used herein, unless otherwise noted, the term "clinically proven" (used independently or to modify the terms "safe" and/or "effective") shall mean that it has been proven by a clinical trial wherein the clinical trial has met the approval standards of U.S. Food and Drug Administration, EMEA or a corresponding national regulatory agency. For example, the clinical study may be an adequately sized, randomized, double-blinded study used to clinically prove the effects of the drug.

As used herein, a dosage amount of an anti-IL-12/IL-23p40 antibody in "mg/kg" refers to the amount of the anti-IL-12/IL-23p40 antibody in milligrams per kilogram of the body weight of a subject to be administered with the antibody.

### Antibodies of the Present Invention - Production and Generation

At least one anti-IL-12/23p40 (or anti-IL-23) used in the method of the present invention can be optionally produced by a cell line, a mixed cell line, an immortalized cell or clonal population of immortalized cells, as well known in the art. See, e.g., Ausubel, et al., ed., Current Protocols in Molecular Biology, John Wiley & Sons, Inc., NY, NY (1987-2001); Sambrook, et al., Molecular Cloning: A Laboratory Manual, 2nd Edition, Cold Spring Harbor, NY (1989); Harlow and Lane, antibodies, a Laboratory Manual, Cold Spring Harbor, NY (1989); Colligan, et al., eds., Current Protocols in Immunology, John Wiley & Sons, Inc., NY (1994-2001); Colligan et al., Current Protocols in Protein Science, John Wiley & Sons, NY, NY, (1997-2001), each entirely incorporated herein by reference.

Human antibodies that are specific for human IL-12/23p40 or IL-23 proteins or fragments thereof can be raised against an appropriate immunogenic antigen, such as an isolated IL-12/23p40 protein, IL-23 protein and/or a portion thereof (including synthetic molecules, such as synthetic peptides). Other specific or general mammalian antibodies can be similarly raised. Preparation of immunogenic antigens, and monoclonal antibody production can be performed using any suitable technique in view of the present disclosure.

In one approach, a hybridoma is produced by fusing a suitable immortal cell line (e.g., a myeloma cell line, such as, but not limited to, Sp2/0, Sp2/0-AG14, NSO, NS1, NS2, AE-1, L.5, L243, P3X63Ag8.653, Sp2 SA3, Sp2 MAI, Sp2 SS1, Sp2 SA5, U937, MLA 144, ACT IV, MOLT4, DA-1, JURKAT, WEHI, K-562, COS, RAJI, NIH 3T3, HL-60, MLA 144, NAMALWA, NEURO 2A, or the like, or heteromylomas, fusion products thereof, or any cell or fusion cell derived therefrom, or any other suitable cell line as known in the art) (see, e.g., www.atcc.org, www.lifetech.com., and the like), with antibody producing cells, such as, but not limited to, isolated or cloned spleen, peripheral blood, lymph, tonsil, or other immune or B cell containing cells, or any other cells expressing heavy or light chain constant or variable or framework or CDR sequences, either as endogenous or heterologous nucleic acid, as recombinant or endogenous, viral, bacterial, algal, prokaryotic, amphibian, insect, reptilian, fish, mammalian, rodent, equine, ovine, goat, sheep, primate, eukaryotic, genomic DNA, cDNA, rDNA, mitochondrial DNA or RNA, chloroplast DNA or RNA, hnRNA, mRNA, tRNA, single, double or triple stranded, hybridized, and the like or any combination thereof. See, e.g., Ausubel, supra, and Colligan, Immunology, supra, chapter 2, entirely incorporated herein by reference.

Antibody producing cells can also be obtained from the peripheral blood or, preferably, the spleen or lymph nodes, of humans or other suitable animals that have been immunized with the antigen of interest. Any other suitable host cell can also be used for expressing heterologous or endogenous nucleic acid encoding an antibody, specified fragment or variant thereof, of the present invention. The fused cells (hybridomas) or recombinant cells can be isolated using selective culture conditions or other suitable known methods, and cloned by limiting dilution or cell sorting, or other known methods. Cells which produce antibodies with the desired specificity can be selected by a suitable assay (e.g., ELISA).

Other suitable methods of producing or isolating antibodies of the requisite specificity can be used, including, but not limited to, methods that select recombinant antibody from a peptide or protein library (e.g., but not limited to, a bacteriophage, ribosome, oligonucleotide, RNA, cDNA, or the like, display library; e.g., as available from Cambridge antibody Technologies, Cambridgeshire, UK; MorphoSys, Martinsreid/Planegg, DE; Biovation, Aberdeen, Scotland, UK; BioInvent, Lund, Sweden; Dyax Corp., Enzon, Affymax/Biosite; Xoma, Berkeley, CA; Ixsys. See, e.g., EP 368,684, PCT/GB91/01134; PCT/GB92/01755; PCT/GB92/002240; PCT/GB92/00883; PCT/GB93/00605; US 08/350260(5/12/94); PCT/GB94/01422; PCT/GB94/02662; PCT/GB97/01835; (CAT/MRC); WO90/14443; WO90/14424; WO90/14430; PCT/US94/1234; WO92/18619; WO96/07754; (Scripps); WO96/13583, WO97/08320 (MorphoSys); WO95/16027 (BioInvent); WO88/06630; WO90/3809 (Dyax); US 4,704,692 (Enzon); PCT/US91/02989 (Affymax); WO89/06283; EP 371 998; EP 550 400; (Xoma); EP 229 046; PCT/US91/07149 (Ixsys); or stochastically generated peptides or proteins - US 5723323, 5763192, 5814476, 5817483, 5824514, 5976862, WO 86/05803, EP 590 689 (Ixsys, predecessor of Applied Molecular Evolution (AME), each entirely incorporated herein by reference)) or that rely upon immunization of transgenic animals (e.g., SCID mice, Nguyen et al., Microbiol. Immunol. 41:901-907 (1997); Sandhu et al., Crit. Rev. Biotechnol. 16:95-118 (1996); Eren et al., Immunol. 93: 154-161 (1998), each entirely incorporated by reference as well as related patents and applications) that are capable of producing a repertoire of human antibodies, as known in the art and/or as described herein. Such techniques, include, but are not limited to, ribosome display (Hanes et al., Proc. Natl. Acad. Sci. USA, 94:4937-4942 (Can 1997); Hanes et al., Proc. Natl. Acad. Sci. USA, 95:14130-14135 (Nov. 1998)); single cell antibody producing technologies (e.g., selected lymphocyte antibody method ("SLAM") (US pat. No. 5,627,052, Wen et al., J. Immunol. 17:887-892 (1987); Babcook et al., Proc. Natl. Acad. Sci. USA 93:7843-7848 (1996)); gel microdroplet and flow cytometry (Powell et al., Biotechnol. 8:333-337 (1990); One Cell Systems, Cambridge, MA; Gray et al., J. Imm. Meth. 182:155-163 (1995); Kenny et al., Bio/Technol. 13:787-790 (1995)); B-cell selection (Steenbakkers etal., Molec. Biol. Reports 19:125-134 (1994); Jonak et al., Progress Biotech, Vol. 5, In Vitro Immunization in Hybridoma Technology, Borrebaeck, ed., Elsevier Science Publishers B.V., Amsterdam, Netherlands (1988)).

Methods for engineering or humanizing non-human or human antibodies can also be used and are well known in the art. Generally, a humanized or engineered antibody has one or more amino acid residues from a source that is non-human, e.g., but not limited to, mouse, rat, rabbit, non-human primate or other mammal. These non-human amino acid residues are replaced by residues often referred to as "import" residues, which are typically taken from an "import" variable, constant or other domain of a known human sequence.

Known human Ig sequences are disclosed, e.g., www.ncbi.nlm.nih.gov/entrez/query.fcgi; www.ncbi.nih.gov/igblast; www.atcc.org/phage/hdb.html; www.mrc-cpe.cam.ac.uk/ALIGNMENTS.php; www.kabatdatabase.com/top.html; ftp.ncbi.nih.gov/repository/kabat; www.sciquest.com; www.abcam.com; www.antibodyresource.com/onlinecomp.html; www.public.iastate.edu/~pedro/research_tools.html; www. whfreeman. com/immunology/CH05/kuby05. htm; www.hhmi.org/grants/lectures/1996/vlab; www.path.cam.ac.uk/~mrc7/mikeimages.html; mcb. harvard. edu/BioLinks/Immunology. html; www.immunologylink.com; pathbox.wustl.edu/-hcenter/index.html; www.appliedbiosystems.com; www.nal.usda.gov/awic/pubs/antibody; www.m.ehime-u.ac.jp/~yasuhito/Elisa.html; www.biodesign.com; www.cancerresearchuk.org; www.biotech.ufl.edu; www.isac-net.org; baserv.uci.kun.nl/~jraats/links1.html; www.recab.uni-hd.de/immuno.bme.nwu.edu; www.mrc-cpe.cam.ac.uk; www.ibt.unam.mx/vir/V_mice.html; http://www.bioinf.org.uk/abs; antibody.bath.ac.uk; www.unizh.ch; www.cryst.bbk.ac.uk/~ubcg07s; www.nimr.mrc.ac.uk/CC/ccaewg/ccaewg.html; www.path.cam.ac.uk/~mrc7/humanisation/TAHHP.html; www.ibt.unam.mx/vir/structure/stat_aim.html; www.biosci.missouri.edu/smithgp/index.html; www.jerini.de; Kabat et al., Sequences of Proteins of Immunological Interest, U.S. Dept. Health (1983), each entirely incorporated herein by reference.

Such imported sequences can be used to reduce immunogenicity or reduce, enhance or modify binding, affinity, on-rate, off-rate, avidity, specificity, half-life, or any other suitable characteristic, as known in the art. In general, the CDR residues are directly and most substantially involved in influencing antigen binding. Accordingly, part or all of the non-human or human CDR sequences are maintained while the non-human sequences of the variable and constant regions can be replaced with human or other amino acids.

Antibodies can also optionally be humanized or human antibodies engineered with retention of high affinity for the antigen and other favorable biological properties. To achieve this goal, humanized (or human) antibodies can be optionally prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, framework (FR) residues can be selected and combined from the consensus and import sequences so that the desired antibody characteristic, such as increased affinity for the target antigen(s), is achieved.

In addition, the human anti-IL-12/23p40 (or anti-IL-23) specific antibody used in the method of the present invention can comprise a human germline light chain framework. In particular embodiments, the light chain germline sequence is selected from human VK sequences including, but not limited to, A1, A10, A11, A14, A17, A18, A19, A2, A20, A23, A26, A27, A3, A30, A5, A7, B2, B3, L1, L10, L11, L12, L14, L15, L16, L18, L19, L2, L20, L22, L23, L24, L25, L4/18a, L5, L6, L8, L9, O1, O11, 012, 014, 018, O2, O4, and O8. In certain embodiments, this light chain human germline framework is selected from V1-11, V1-13, V1-16, V1-17, V1-18, V1-19, V1-2, V1-20, V1-22, V1-3, V1-4, V1-5, V1-7, V1-9, V2-1, V2-11, V2-13, V2-14, V2-15, V2-17, V2-19, V2-6, V2-7, V2-8, V3-2, V3-3, V3-4, V4-1, V4-2, V4-3, V4-4, V4-6, V5-1, V5-2, V5-4, and V5-6.

In other embodiments, the human anti-IL-12/23p40 (or anti-IL-23) specific antibody used in the method of the present invention can comprise a human germline heavy chain framework. In particular embodiments, this heavy chain human germline framework is selected from VH1-18, VH1-2, VH1-24, VH1-3, VH1-45, VH1-46, VH1-58, VH1-69, VH1-8, VH2-26, VH2-5, VH2-70, VH3-11, VH3-13, VH3-15, VH3-16, VH3-20, VH3-21, VH3-23, VH3-30, VH3-33, VH3-35, VH3-38, VH3-43, VH3-48, VH3-49, VH3-53, VH3-64, VH3-66, VH3-7, VH3-72, VH3-73, VH3-74, VH3-9, VH4-28, VH4-31, VH4-34, VH4-39, VH4-4, VH4-59, VH4-61, VH5-51, VH6-1, and VH7-81.

In particular embodiments, the light chain variable region and/or heavy chain variable region comprises a framework region or at least a portion of a framework region (e.g., containing 2 or 3 subregions, such as FR2 and FR3). In certain embodiments, at least FRL1, FRL2, FRL3, or FRL4 is fully human. In other embodiments, at least FRH1, FRH2, FRH3, or FRH4 is fully human. In some embodiments, at least FRL1, FRL2, FRL3, or FRL4 is a germline sequence (e.g., human germline) or comprises human consensus sequences for the particular framework (readily available at the sources of known human Ig sequences described above). In other embodiments, at least FRH1, FRH2, FRH3, or FRH4 is a germline sequence (e.g., human germline) or comprises human consensus sequences for the particular framework. In preferred embodiments, the framework region is a fully human framework region.

Humanization or engineering of antibodies of the present invention can be performed using any known method, such as but not limited to those described in, Winter (Jones et al., Nature 321:522 (1986); Riechmann et al., Nature 332:323 (1988); Verhoeyen et al., Science 239:1534 (1988)), Sims et al., J. Immunol. 151: 2296 (1993); Chothia and Lesk, J. Mol. Biol. 196:901 (1987), Carter et al., Proc. Natl. Acad. Sci. U.S.A. 89:4285 (1992); Presta et al., J. Immunol. 151:2623 (1993), US Patent Nos: 5723323, 5976862, 5824514, 5817483, 5814476, 5763192, 5723323, 5,766886, 5714352, 6204023, 6180370, 5693762, 5530101, 5585089, 5225539; 4816567, PCT/: US98/16280, US96/18978, US91/09630, US91/05939, US94/01234, GB89/01334, GB91/01134, GB92/01755; WO90/14443, WO90/14424, WO90/14430, EP 229246, each entirely incorporated herein by reference, included references cited therein.

In certain embodiments, the antibody comprises an altered (e.g., mutated) Fc region. For example, in some embodiments, the Fc region has been altered to reduce or enhance the effector functions of the antibody. In some embodiments, the Fc region is an isotype selected from IgM, IgA, IgG, IgE, or other isotype. Alternatively, or additionally, it can be useful to combine amino acid modifications with one or more further amino acid modifications that alter C1q binding and/or the complement dependent cytotoxicity function of the Fc region of an IL-23 binding molecule. The starting polypeptide of particular interest can be one that binds to C1q and displays complement dependent cytotoxicity (CDC). Polypeptides with pre-existing C1q binding activity, optionally further having the ability to mediate CDC can be modified such that one or both of these activities are enhanced. Amino acid modifications that alter C1q and/or modify its complement dependent cytotoxicity function are described, for example, in WO0042072, which is hereby incorporated by reference.

As disclosed above, one can design an Fc region of the human anti-IL-12/23p40 (or anti-IL-23) specific antibody of the present invention with altered effector function, e.g., by modifying C1q binding and/or FcyR binding and thereby changing complement dependent cytotoxicity (CDC) activity and/or antibody-dependent cell-mediated cytotoxicity (ADCC) activity. "Effector functions" are responsible for activating or diminishing a biological activity (e.g., in a subject). Examples of effector functions include, but are not limited to: C1q binding; CDC; Fc receptor binding; ADCC; phagocytosis; down regulation of cell surface receptors (e.g., B cell receptor; BCR), etc. Such effector functions can require the Fc region to be combined with a binding domain (e.g., an antibody variable domain) and can be assessed using various assays (e.g., Fc binding assays, ADCC assays, CDC assays, etc.).

For example, one can generate a variant Fc region of the human anti-IL-12/23p40 (or anti-IL-23) antibody with improved C1q binding and improved FcyRIII binding (e.g., having both improved ADCC activity and improved CDC activity). Alternatively, if it is desired that effector function be reduced or ablated, a variant Fc region can be engineered with reduced CDC activity and/or reduced ADCC activity. In other embodiments, only one of these activities can be increased, and, optionally, also the other activity reduced (e.g., to generate an Fc region variant with improved ADCC activity, but reduced CDC activity and vice versa).

Fc mutations can also be introduced in engineer to alter their interaction with the neonatal Fc receptor (FcRn) and improve their pharmacokinetic properties. A collection of human Fc variants with improved binding to the FcRn have been described (Shields et al., (2001). High resolution mapping of the binding site on human IgG1 for FcyRI, FcyRII, FcyRIII, and FcRn and design of IgG1 variants with improved binding to the FcyR, J. Biol. Chem. 276:6591-6604).

Another type of amino acid substitution serves to alter the glycosylation pattern of the Fc region of the human anti-IL-12/23p40 (or anti-IL-23) specific antibody. Glycosylation of an Fc region is typically either N-linked or O-linked. N-linked refers to the attachment of the carbohydrate moiety to the side chain of an asparagine residue. O-linked glycosylation refers to the attachment of one of the sugars N-aceylgalactosamine, galactose, or xylose to a hydroxyamino acid, most commonly serine or threonine, although 5-hydroxyproline or 5-hydroxylysine can also be used. The recognition sequences for enzymatic attachment of the carbohydrate moiety to the asparagine side chain peptide sequences are asparagine-X-serine and asparagine-X-threonine, where X is any amino acid except proline. Thus, the presence of either of these peptide sequences in a polypeptide creates a potential glycosylation site.

The glycosylation pattern can be altered, for example, by deleting one or more glycosylation site(s) found in the polypeptide, and/or adding one or more glycosylation sites that are not present in the polypeptide. Addition of glycosylation sites to the Fc region of a human IL-23 specific antibody is conveniently accomplished by altering the amino acid sequence such that it contains one or more of the above-described tripeptide sequences (for N-linked glycosylation sites). An exemplary glycosylation variant has an amino acid substitution of residue Asn 297 of the heavy chain. The alteration can also be made by the addition of, or substitution by, one or more serine or threonine residues to the sequence of the original polypeptide (for O-linked glycosylation sites). Additionally, a change of Asn 297 to Ala can remove one of the glycosylation sites.

In certain embodiments, the human anti-IL-12/23p40 (or anti-IL-23) specific antibody of the present invention is expressed in cells that express beta (1,4)-N-acetylglucosaminyltransferase III (GnT III), such that GnT III adds GlcNAc to the human anti-IL-12/23p40 (or anti-IL-23) antibody. Methods for producing antibodies in such a fashion are provided in WO/9954342, WO/03011878, patent publication 20030003097A1, and Umana et al., Nature Biotechnology, 17: 176-180, Feb. 1999; all of which are herein specifically incorporated by reference in their entireties.

The human anti-IL-12/23p40 (or anti-IL-23) antibody can also be optionally generated by immunization of a transgenic animal (e.g., mouse, rat, hamster, non-human primate, and the like) capable of producing a repertoire of human antibodies, as described herein and/or as known in the art. Cells that produce a human anti-IL-12/23p40 (or anti-IL-23) antibody can be isolated from such animals and immortalized using suitable methods, such as the methods described herein.

Transgenic mice that can produce a repertoire of human antibodies that bind to human antigens can be produced by known methods (e.g., but not limited to, U.S. Pat. Nos: 5,770,428, 5,569,825, 5,545,806, 5,625,126, 5,625,825, 5,633,425, 5,661,016 and 5,789,650 issued to Lonberg et al.; Jakobovits et al. WO 98/50433, Jakobovits et al. WO 98/24893, Lonberg et al. WO 98/24884, Lonberg et al. WO 97/13852, Lonberg et al. WO 94/25585, Kucherlapate et al. WO 96/34096, Kucherlapate et al. EP 0463 151 B1, Kucherlapate et al. EP 0710 719 A1, Surani et al. US. Pat. No. 5,545,807, Bruggemann et al. WO 90/04036, Bruggemann et al. EP 0438 474 B1, Lonberg et al. EP 0814 259 A2, Lonberg et al. GB 2 272 440 A, Lonberg et al. Nature 368:856-859 (1994), Taylor et al., Int. Immunol. 6(4)579-591 (1994), Green et al, Nature Genetics 7: 13-21 (1994), Mendez et al., Nature Genetics 15:146-156 (1997), Taylor et al., Nucleic Acids Research 20(23):6287-6295 (1992), Tuaillon et al., Proc Natl Acad Sci USA 90(8)3720-3724 (1993), Lonberg et al., Int Rev Immunol 13(1):65-93 (1995) and Fishwald et al., Nat Biotechnol 14(7):845-851 (1996), which are each entirely incorporated herein by reference). Generally, these mice comprise at least one transgene comprising DNA from at least one human immunoglobulin locus that is functionally rearranged, or which can undergo functional rearrangement. The endogenous immunoglobulin loci in such mice can be disrupted or deleted to eliminate the capacity of the animal to produce antibodies encoded by endogenous genes.

Screening antibodies for specific binding to similar proteins or fragments can be conveniently achieved using peptide display libraries. This method involves the screening of large collections of peptides for individual members having the desired function or structure. Antibody screening of peptide display libraries is well known in the art. The displayed peptide sequences can be from 3 to 5000 or more amino acids in length, frequently from 5-100 amino acids long, and often from about 8 to 25 amino acids long. In addition to direct chemical synthetic methods for generating peptide libraries, several recombinant DNA methods have been described. One type involves the display of a peptide sequence on the surface of a bacteriophage or cell. Each bacteriophage or cell contains the nucleotide sequence encoding the particular displayed peptide sequence. Such methods are described in PCT Patent Publication Nos. 91/17271, 91/18980, 91/19818, and 93/08278.

Other systems for generating libraries of peptides have aspects of both in vitro chemical synthesis and recombinant methods. See, PCT Patent Publication Nos. 92/05258, 92/14843, and 96/19256. See also, U.S. Patent Nos. 5,658,754; and 5,643,768. Peptide display libraries, vector, and screening kits are commercially available from such suppliers as Invitrogen (Carlsbad, CA), and Cambridge antibody Technologies (Cambridgeshire, UK). See, e.g., U.S. Pat. Nos. 4704692, 4939666, 4946778, 5260203, 5455030, 5518889, 5534621, 5656730, 5763733, 5767260, 5856456, assigned to Enzon; 5223409, 5403484, 5571698, 5837500, assigned to Dyax, 5427908, 5580717, assigned to Affymax; 5885793, assigned to Cambridge antibody Technologies; 5750373, assigned to Genentech, 5618920, 5595898, 5576195, 5698435, 5693493, 5698417, assigned to Xoma, Colligan, supra; Ausubel, supra; or Sambrook, supra, each of the above patents and publications entirely incorporated herein by reference.

Antibodies used in the method of the present invention can also be prepared using at least one anti-IL-12/23p40 (or anti-IL-23) antibody encoding nucleic acid to provide transgenic animals or mammals, such as goats, cows, horses, sheep, rabbits, and the like, that produce such antibodies in their milk. Such animals can be provided using known methods. See, e.g., but not limited to, US Patent Nos. 5,827,690; 5,849,992; 4,873,316; 5,849,992; 5,994,616; 5,565,362; 5,304,489, and the like, each of which is entirely incorporated herein by reference.

Antibodies used in the method of the present invention can additionally be prepared using at least one anti-IL-12/23p40 (or anti-IL-23) antibody encoding nucleic acid to provide transgenic plants and cultured plant cells (e.g., but not limited to, tobacco and maize) that produce such antibodies, specified portions or variants in the plant parts or in cells cultured therefrom. As a non-limiting example, transgenic tobacco leaves expressing recombinant proteins have been successfully used to provide large amounts of recombinant proteins, e.g., using an inducible promoter. See, e.g., Cramer et al., Curr. Top. Microbol. Immunol. 240:95-118 (1999) and references cited therein. Also, transgenic maize have been used to express mammalian proteins at commercial production levels, with biological activities equivalent to those produced in other recombinant systems or purified from natural sources. See, e.g., Hood et al., Adv. Exp. Med. Biol. 464:127-147 (1999) and references cited therein. Antibodies have also been produced in large amounts from transgenic plant seeds including antibody fragments, such as single chain antibodies (scFv's), including tobacco seeds and potato tubers. See, e.g., Conrad et al., Plant Mol. Biol. 38:101-109 (1998) and references cited therein. Thus, antibodies of the present invention can also be produced using transgenic plants, according to known methods. See also, e.g., Fischer et al., Biotechnol. Appl. Biochem. 30:99-108 (Oct., 1999), Ma et al., Trends Biotechnol. 13:522-7 (1995); Ma et al., Plant Physiol. 109:341-6 (1995); Whitelam et al., Biochem. Soc. Trans. 22:940-944 (1994); and references cited therein. Each of the above references is entirely incorporated herein by reference.

The antibodies used in the method of the invention can bind human IL-12/IL-23p40 or IL-23 with a wide range of affinities (KD). In a preferred embodiment, a human mAb can optionally bind human IL-12/IL-23p40 or IL-23 with high affinity. For example, a human mAb can bind human IL-12/IL-23p40 or IL-23 with a KD equal to or less than about 10-7 M, such as but not limited to, 0.1-9.9 (or any range or value therein) X 10-7, 10-8, 10-9, 10-10, 10-11, 10-12, 10-13 or any range or value therein.

The affinity or avidity of an antibody for an antigen can be determined experimentally using any suitable method. (See, for example, Berzofsky, et al., "Antibody-Antigen Interactions," In Fundamental Immunology, Paul, W. E., Ed., Raven Press: New York, NY (1984); Kuby, Janis Immunology, W. H. Freeman and Company: New York, NY (1992); and methods described herein). The measured affinity of a particular antibody-antigen interaction can vary if measured under different conditions (e.g., salt concentration, pH). Thus, measurements of affinity and other antigen-binding parameters (e.g., KD, Ka, Kd) are preferably made with standardized solutions of antibody and antigen, and a standardized buffer, such as the buffer described herein.

### Vectors and Host Cells

The present invention also relates to vectors that include isolated nucleic acid molecules, host cells that are genetically engineered with the recombinant vectors, and the production of at least one anti-IL-12/IL-23p40 antibody by recombinant techniques, as is well known in the art. See, e.g., Sambrook, et al., supra; Ausubel, et al., supra, each entirely incorporated herein by reference.

The polynucleotides can optionally be joined to a vector containing a selectable marker for propagation in a host. Generally, a plasmid vector is introduced in a precipitate, such as a calcium phosphate precipitate, or in a complex with a charged lipid. If the vector is a virus, it can be packaged in vitro using an appropriate packaging cell line and then transduced into host cells.

The DNA insert should be operatively linked to an appropriate promoter. The expression constructs will further contain sites for transcription initiation, termination and, in the transcribed region, a ribosome binding site for translation. The coding portion of the mature transcripts expressed by the constructs will preferably include a translation initiating at the beginning and a termination codon (e.g., UAA, UGA or UAG) appropriately positioned at the end of the mRNA to be translated, with UAA and UAG preferred for mammalian or eukaryotic cell expression.

Expression vectors will preferably but optionally include at least one selectable marker. Such markers include, e.g., but are not limited to, methotrexate (MTX), dihydrofolate reductase (DHFR, US Pat.Nos. 4,399,216; 4,634,665; 4,656,134; 4,956,288; 5,149,636; 5,179,017, ampicillin, neomycin (G418), mycophenolic acid, or glutamine synthetase (GS, US Pat.Nos. 5,122,464; 5,770,359; 5,827,739) resistance for eukaryotic cell culture, and tetracycline or ampicillin resistance genes for culturing in E. coli and other bacteria or prokaryotics (the above patents are entirely incorporated hereby by reference). Appropriate culture mediums and conditions for the above-described host cells are known in the art. Suitable vectors will be readily apparent to the skilled artisan. Introduction of a vector construct into a host cell can be effected by calcium phosphate transfection, DEAE-dextran mediated transfection, cationic lipid-mediated transfection, electroporation, transduction, infection or other known methods. Such methods are described in the art, such as Sambrook, supra, Chapters 1-4 and 16-18; Ausubel, supra, Chapters 1, 9, 13, 15, 16.

At least one antibody used in the method of the present invention can be expressed in a modified form, such as a fusion protein, and can include not only secretion signals, but also additional heterologous functional regions. For instance, a region of additional amino acids, particularly charged amino acids, can be added to the N-terminus of an antibody to improve stability and persistence in the host cell, during purification, or during subsequent handling and storage. Also, peptide moieties can be added to an antibody of the present invention to facilitate purification. Such regions can be removed prior to final preparation of an antibody or at least one fragment thereof. Such methods are described in many standard laboratory manuals, such as Sambrook, supra, Chapters 17.29-17.42 and 18.1-18.74; Ausubel, supra, Chapters 16, 17 and 18.

Those of ordinary skill in the art are knowledgeable in the numerous expression systems available for expression of a nucleic acid encoding a protein used in the method of the present invention. Alternatively, nucleic acids can be expressed in a host cell by turning on (by manipulation) in a host cell that contains endogenous DNA encoding an antibody. Such methods are well known in the art, e.g., as described in US patent Nos. 5,580,734, 5,641,670, 5,733,746, and 5,733,761, entirely incorporated herein by reference.

Illustrative of cell cultures useful for the production of the antibodies, specified portions or variants thereof, are mammalian cells. Mammalian cell systems often will be in the form of monolayers of cells although mammalian cell suspensions or bioreactors can also be used. A number of suitable host cell lines capable of expressing intact glycosylated proteins have been developed in the art, and include the COS-1 (e.g., ATCC CRL 1650), COS-7 (e.g., ATCC CRL-1651), HEK293, BHK21 (e.g., ATCC CRL-10), CHO (e.g., ATCC CRL 1610) and BSC-1 (e.g., ATCC CRL-26) cell lines, Cos-7 cells, CHO cells, hep G2 cells, P3X63Ag8.653, SP2/0-Ag14, 293 cells, HeLa cells and the like, which are readily available from, for example, American Type Culture Collection, Manassas, Va (www.atcc.org). Preferred host cells include cells of lymphoid origin, such as myeloma and lymphoma cells. Particularly preferred host cells are P3X63Ag8.653 cells (ATCC Accession Number CRL-1580) and SP2/0-Ag14 cells (ATCC Accession Number CRL-1851). In a particularly preferred embodiment, the recombinant cell is a P3X63Ab8.653 or a SP2/0-Ag14 cell.

Expression vectors for these cells can include one or more of the following expression control sequences, such as, but not limited to, an origin of replication; a promoter (e.g., late or early SV40 promoters, the CMV promoter (US Pat.Nos. 5,168,062; 5,385,839), an HSV tk promoter, a pgk (phosphoglycerate kinase) promoter, an EF-1 alpha promoter (US Pat.No. 5,266,491), at least one human immunoglobulin promoter; an enhancer, and/or processing information sites, such as ribosome binding sites, RNA splice sites, polyadenylation sites (e.g., an SV40 large T Ag poly A addition site), and transcriptional terminator sequences. See, e.g., Ausubel et al., supra; Sambrook, et al., supra. Other cells useful for production of nucleic acids or proteins of the present invention are known and/or available, for instance, from the American Type Culture Collection Catalogue of Cell Lines and Hybridomas (www.atcc.org) or other known or commercial sources.

When eukaryotic host cells are employed, polyadenlyation or transcription terminator sequences are typically incorporated into the vector. An example of a terminator sequence is the polyadenlyation sequence from the bovine growth hormone gene. Sequences for accurate splicing of the transcript can also be included. An example of a splicing sequence is the VP1 intron from SV40 (Sprague, et al., J. Virol. 45:773-781 (1983)). Additionally, gene sequences to control replication in the host cell can be incorporated into the vector, as known in the art.

### Purification of an Antibody

An anti-IL-12/IL-23p40 or IL-23 antibody can be recovered and purified from recombinant cell cultures by well-known methods including, but not limited to, protein A purification, ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. High performance liquid chromatography ("HPLC") can also be employed for purification. See, e.g., Colligan, Current Protocols in Immunology, or Current Protocols in Protein Science, John Wiley & Sons, NY, NY, (1997-2001), e.g., Chapters 1, 4, 6, 8, 9, 10, each entirely incorporated herein by reference.

Antibodies used in the method of the present invention include naturally purified products, products of chemical synthetic procedures, and products produced by recombinant techniques from a eukaryotic host, including, for example, yeast, higher plant, insect and mammalian cells. Depending upon the host employed in a recombinant production procedure, the antibody can be glycosylated or can be non-glycosylated, with glycosylated preferred. Such methods are described in many standard laboratory manuals, such as Sambrook, supra, Sections 17.37-17.42; Ausubel, supra, Chapters 10, 12, 13, 16, 18 and 20, Colligan, Protein Science, supra, Chapters 12-14, all entirely incorporated herein by reference.

### Anti-IL-12/IL-23p40 or IL-23 Antibodies

An anti-IL-12/IL-23p40 or IL-23 antibody according to the present invention includes any protein or peptide containing molecule that comprises at least a portion of an immunoglobulin molecule, such as but not limited to, at least one ligand binding portion (LBP), such as but not limited to, a complementarity determining region (CDR) of a heavy or light chain or a ligand binding portion thereof, a heavy chain or light chain variable region, a framework region (e.g., FR1, FR2, FR3, FR4 or fragment thereof, further optionally comprising at least one substitution, insertion or deletion), a heavy chain or light chain constant region, (e.g., comprising at least one CH1, hinge1, hinge2, hinge3, hinge4, CH2, or CH3 or fragment thereof, further optionally comprising at least one substitution, insertion or deletion), or any portion thereof, that can be incorporated into an antibody. An antibody can include or be derived from any mammal, such as but not limited to, a human, a mouse, a rabbit, a rat, a rodent, a primate, or any combination thereof, and the like.

Preferably, the human antibody or antigen-binding fragment binds human IL-12/IL-23p40 or IL-23 and, thereby, partially or substantially neutralizes at least one biological activity of the protein. An antibody, or specified portion or variant thereof, that partially or preferably substantially neutralizes at least one biological activity of at least one IL-12/IL-23p40 or IL-23 protein or fragment can bind the protein or fragment and thereby inhibit activities mediated through the binding of IL-12/IL-23p40 or IL-23 to the IL-12 and/or IL-23 receptor or through other IL-12/IL-23p40 or IL-23-dependent or mediated mechanisms. As used herein, the term "neutralizing antibody" refers to an antibody that can inhibit an IL-12/IL-23p40 or IL-23-dependent activity by about 20-120%, preferably by at least about 10, 20, 30, 40, 50, 55, 60, 65, 70, 75, 80, 85, 90, 91, 92, 93, 94, 95, 96, 97, 98, 99, 100% or more depending on the assay. The capacity of an anti-IL-12/IL-23p40 or IL-23 antibody to inhibit an IL-12/IL-23p40 or IL-23-dependent activity is preferably assessed by at least one suitable IL-12/IL-23p40 or IL-23 protein or receptor assay, as described herein and/or as known in the art. A human antibody can be of any class (IgG, IgA, IgM, IgE, IgD, etc.) or isotype and can comprise a kappa or lambda light chain. In one embodiment, the human antibody comprises an IgG heavy chain or defined fragment, for example, at least one of isotypes, IgG1, IgG2, IgG3 or IgG4 (e.g., γ1, y2, y3, y4). Antibodies of this type can be prepared by employing a transgenic mouse or other trangenic non-human mammal comprising at least one human light chain (e.g., IgG, IgA, and IgM) transgenes as described herein and/or as known in the art. In another embodiment, the anti-IL-23 human antibody comprises an IgG1 heavy chain and an IgG1 light chain.

An antibody binds at least one specified epitope specific to at least one IL-12/IL-23p40 or IL-23 protein, subunit, fragment, portion or any combination thereof. The at least one epitope can comprise at least one antibody binding region that comprises at least one portion of the protein, which epitope is preferably comprised of at least one extracellular, soluble, hydrophillic, external or cytoplasmic portion of the protein.

Generally, the human antibody or antigen-binding fragment will comprise an antigen-binding region that comprises at least one human complementarity determining region (CDR1, CDR2 and CDR3) or variant of at least one heavy chain variable region and at least one human complementarity determining region (CDR1, CDR2 and CDR3) or variant of at least one light chain variable region. The CDR sequences can be derived from human germline sequences or closely match the germline sequences. For example, the CDRs from a synthetic library derived from the original non-human CDRs can be used. These CDRs can be formed by incorporation of conservative substitutions from the original non-human sequence. In another particular embodiment, the antibody or antigen-binding portion or variant can have an antigen-binding region that comprises at least a portion of at least one light chain CDR (i.e., CDR1, CDR2 and/or CDR3) having the amino acid sequence of the corresponding CDRs 1, 2 and/or 3.

Such antibodies can be prepared by chemically joining together the various portions (e.g., CDRs, framework) of the antibody using conventional techniques, by preparing and expressing a (i.e., one or more) nucleic acid molecule that encodes the antibody using conventional techniques of recombinant DNA technology or by using any other suitable method.

In one embodiment, an anti-IL-12/23p40 antibody useful for the invention is a monoclonal antibody, preferably a human mAb, comprising heavy chain complementarity determining regions (CDRs) HCDR1, HCDR2, and HCDR3 of SEQ ID NOs: 1, 2, and 3, respectively; and light chain CDRs LCDR1, LCDR2, and LCDR3, of SEQ ID NOs: 4, 5, and 6, respectively.

The anti-IL-12/IL-23p40 or IL-23 specific antibody can comprise at least one of a heavy or light chain variable region having a defined amino acid sequence. For example, in a preferred embodiment, the anti-IL-12/IL-23p40 or IL-23 antibody comprises an anti-IL-12/IL-23p40 antibody with a heavy chain variable region comprising an amino acid sequence at least 85%, preferably at least 90%, more preferably at least 95%, and most preferably 100% identical to SEQ ID NO:7, and a light chain variable region comprising an amino acid sequence at least 85%, preferably at least 90%, more preferably at least 95%, and most preferably 100% identical to SEQ ID NO:8.

The anti-IL-12/IL-23p40 or IL-23 specific antibody can also comprise at least one of a heavy or light chain having a defined amino acid sequence. In another preferred embodiment, the anti-IL-12/IL-23p40 or IL-23 antibody comprises an anti-IL-12/IL-23p40 antibody with a heavy chain comprising an amino acid sequence at least 85%, preferably at least 90%, more preferably at least 95%, and most preferably 100% identical to SEQ ID NO: 10, and a light chain variable region comprising an amino acid sequence at least 85%, preferably at least 90%, more preferably at least 95%, and most preferably 100% identical to SEQ ID NO: 11.

Preferably, the anti-IL-12/23p40 antibody is ustekinumab (Stelara^{®}), comprising a heavy chain having the amino acid sequence of SEQ ID NO: 10 and a light chain comprising the amino acid sequence of SEQ ID NO: 11. Other examples of anti-IL12/23p40 antibodies useful for the invention include, but are not limited to, Briakinumab (ABT-874, Abbott) and other antibodies described in U.S. Patent Nos. 6,914,128, 7,247,711, 7700739, the entire contents of which are incorporated herein by reference).

The invention also relates to antibodies, antigen-binding fragments, immunoglobulin chains and CDRs comprising amino acids in a sequence that is substantially the same as an amino acid sequence described herein. Preferably, such antibodies or antigen-binding fragments and antibodies comprising such chains or CDRs can bind human IL-12/IL-23p40 or IL-23 with high affinity (e.g., KD less than or equal to about 10⁻⁹ M). Amino acid sequences that are substantially the same as the sequences described herein include sequences comprising conservative amino acid substitutions, as well as amino acid deletions and/or insertions. A conservative amino acid substitution refers to the replacement of a first amino acid by a second amino acid that has chemical and/or physical properties (e.g., charge, structure, polarity, hydrophobicity/hydrophilicity) that are similar to those of the first amino acid. Conservative substitutions include, without limitation, replacement of one amino acid by another within the following groups: lysine (K), arginine (R) and histidine (H); aspartate (D) and glutamate (E); asparagine (N), glutamine (Q), serine (S), threonine (T), tyrosine (Y), K, R, H, D and E; alanine (A), valine (V), leucine (L), isoleucine (I), proline (P), phenylalanine (F), tryptophan (W), methionine (M), cysteine (C) and glycine (G); F, W and Y; C, S and T.

Antibodies that bind to human IL-12/IL-23p40 or IL-23 and that comprise a defined heavy or light chain variable region can be prepared using suitable methods, such as phage display (Katsube, Y., et al., Int J Mol. Med, 1(5):863-868 (1998)) or methods that employ transgenic animals, as known in the art and/or as described herein. For example, a transgenic mouse, comprising a functionally rearranged human immunoglobulin heavy chain transgene and a transgene comprising DNA from a human immunoglobulin light chain locus that can undergo functional rearrangement, can be immunized with human IL-12/IL-23p40 or IL-23 or a fragment thereof to elicit the production of antibodies. If desired, the antibody producing cells can be isolated and hybridomas or other immortalized antibody-producing cells can be prepared as described herein and/or as known in the art. Alternatively, the antibody, specified portion or variant can be expressed using the encoding nucleic acid or portion thereof in a suitable host cell.

An anti-IL-12/IL-23p40 or IL-23 antibody used in the method of the present invention can include one or more amino acid substitutions, deletions or additions, either from natural mutations or human manipulation, as specified herein.

The number of amino acid substitutions a skilled artisan would make depends on many factors, including those described above. Generally speaking, the number of amino acid substitutions, insertions or deletions for any given anti-IL-12/IL-23p40 or IL-23 antibody, fragment or variant will not be more than 40, 30, 20, 19, 18, 17, 16, 15, 14, 13, 12, 11, 10, 9, 8, 7, 6, 5, 4, 3, 2, 1, such as 1-30 or any range or value therein, as specified herein.

Amino acids in an anti-IL-12/IL-23p40 or IL-23 specific antibody that are essential for function can be identified by methods known in the art, such as site-directed mutagenesis or alanine-scanning mutagenesis (e.g., Ausubel, supra, Chapters 8, 15; Cunningham and Wells, Science 244:1081-1085 (1989)). The latter procedure introduces single alanine mutations at every residue in the molecule. The resulting mutant molecules are then tested for biological activity, such as, but not limited to, at least one IL-12/IL-23p40 or IL-23 neutralizing activity. Sites that are critical for antibody binding can also be identified by structural analysis, such as crystallization, nuclear magnetic resonance or photoaffinity labeling (Smith, et al., J. Mol. Biol. 224:899-904 (1992) and de Vos, et al., Science 255:306-312 (1992)).

Anti-IL-12/IL-23p40 or IL-23 antibodies can include, but are not limited to, at least one portion, sequence or combination selected from 5 to all of the contiguous amino acids of at least one of SEQ ID NOs 1, 2, 3, 4, 5, 6, 7, 8, 10, or 11.

IL-12/IL-23p40 or IL-23 antibodies or specified portions or variants can include, but are not limited to, at least one portion, sequence or combination selected from at least 3-5 contiguous amino acids of the SEQ ID NOs above; 5-17 contiguous amino acids of the SEQ ID NOs above, 5-10 contiguous amino acids of the SEQ ID NOs above, 5-11 contiguous amino acids of the SEQ ID NOs above, 5-7 contiguous amino acids of the SEQ ID NOs above; 5-9 contiguous amino acids of the SEQ ID NOs above.

An anti-IL-12/IL-23p40 or IL-23 antibody can further optionally comprise a polypeptide of at least one of 70-100% of 5, 17, 10, 11, 7, 9, 119, 108, 449, or 214 contiguous amino acids of the SEQ ID NOs above. In one embodiment, the amino acid sequence of an immunoglobulin chain, or portion thereof (e.g., variable region, CDR) has about 70-100% identity (e.g., 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 or any range or value therein) to the amino acid sequence of the corresponding chain of at least one of the SEQ ID NOs above. For example, the amino acid sequence of a light chain variable region can be compared with the sequence of the SEQ ID NOs above, or the amino acid sequence of a heavy chain CDR3 can be compared with the SEQ ID NOs above. Preferably, 70-100% amino acid identity (i.e., 90, 91, 92, 93, 94, 95, 96, 97, 98, 99, 100 or any range or value therein) is determined using a suitable computer algorithm, as known in the art.

"Identity," as known in the art, is a relationship between two or more polypeptide sequences or two or more polynucleotide sequences, as determined by comparing the sequences. In the art, "identity" also means the degree of sequence relatedness between polypeptide or polynucleotide sequences, as determined by the match between strings of such sequences. "Identity" and "similarity" can be readily calculated by known methods, including, but not limited to, those described in Computational Molecular Biology, Lesk, A. M., ed., Oxford University Press, New York, 1988; Biocomputing:Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; and Carillo, H., and Lipman, D., Siam J. Applied Math., 48:1073 (1988). In addition, values for percentage identity can be obtained from amino acid and nucleotide sequence alignments generated using the default settings for the AlignX component of Vector NTI Suite 8.0 (Informax, Frederick, MD).

Preferred methods to determine identity are designed to give the largest match between the sequences tested. Methods to determine identity and similarity are codified in publicly available computer programs. Preferred computer program methods to determine identity and similarity between two sequences include, but are not limited to, the GCG program package (Devereux, J., et al., Nucleic Acids Research 12(1): 387 (1984)), BLASTP, BLASTN, and FASTA (Atschul, S. F. et al., J. Molec. Biol. 215:403-410 (1990)). The BLAST X program is publicly available from NCBI and other sources (BLAST Manual, Altschul, S., et al., NCBINLMNIH Bethesda, Md. 20894: Altschul, S., et al., J. Mol. Biol. 215:403-410 (1990). The well-known Smith Waterman algorithm can also be used to determine identity.

Exemplary heavy chain and light chain variable regions sequences and portions thereof are provided in the SEQ ID NOs above. The antibodies of the present invention, or specified variants thereof, can comprise any number of contiguous amino acid residues from an antibody of the present invention, wherein that number is selected from the group of integers consisting of from 10-100% of the number of contiguous residues in an anti-IL-12/IL-23p40 or IL-23 antibody. Optionally, this subsequence of contiguous amino acids is at least about 10, 20, 30, 40, 50, 60, 70, 80, 90, 100, 110, 120, 130, 140, 150, 160, 170, 180, 190, 200, 210, 220, 230, 240, 250 or more amino acids in length, or any range or value therein. Further, the number of such subsequences can be any integer selected from the group consisting of from 1 to 20, such as at least 2, 3, 4, or 5.

As those of skill will appreciate, the present invention includes at least one biologically active antibody of the present invention. Biologically active antibodies have a specific activity at least 20%, 30%, or 40%, and, preferably, at least 50%, 60%, or 70%, and, most preferably, at least 80%, 90%, or 95%-100% or more (including, without limitation, up to 10 times the specific activity) of that of the native (non-synthetic), endogenous or related and known antibody. Methods of assaying and quantifying measures of enzymatic activity and substrate specificity are well known to those of skill in the art.

In another aspect, the invention relates to human antibodies and antigen-binding fragments, as described herein, which are modified by the covalent attachment of an organic moiety. Such modification can produce an antibody or antigen-binding fragment with improved pharmacokinetic properties (e.g., increased in vivo serum half-life). The organic moiety can be a linear or branched hydrophilic polymeric group, fatty acid group, or fatty acid ester group. In particular embodiments, the hydrophilic polymeric group can have a molecular weight of about 800 to about 120,000 Daltons and can be a polyalkane glycol (e.g., polyethylene glycol (PEG), polypropylene glycol (PPG)), carbohydrate polymer, amino acid polymer or polyvinyl pyrolidone, and the fatty acid or fatty acid ester group can comprise from about eight to about forty carbon atoms.

The modified antibodies and antigen-binding fragments can comprise one or more organic moieties that are covalently bonded, directly or indirectly, to the antibody. Each organic moiety that is bonded to an antibody or antigen-binding fragment of the invention can independently be a hydrophilic polymeric group, a fatty acid group or a fatty acid ester group. As used herein, the term "fatty acid" encompasses mono-carboxylic acids and di-carboxylic acids. A "hydrophilic polymeric group," as the term is used herein, refers to an organic polymer that is more soluble in water than in octane. For example, polylysine is more soluble in water than in octane. Thus, an antibody modified by the covalent attachment of polylysine is encompassed by the invention. Hydrophilic polymers suitable for modifying antibodies of the invention can be linear or branched and include, for example, polyalkane glycols (e.g., PEG, monomethoxy-polyethylene glycol (mPEG), PPG and the like), carbohydrates (e.g., dextran, cellulose, oligosaccharides, polysaccharides and the like), polymers of hydrophilic amino acids (e.g., polylysine, polyarginine, polyaspartate and the like), polyalkane oxides (e.g., polyethylene oxide, polypropylene oxide and the like) and polyvinyl pyrolidone. Preferably, the hydrophilic polymer that modifies the antibody of the invention has a molecular weight of about 800 to about 150,000 Daltons as a separate molecular entity. For example, PEG5000 and PEG20,000, wherein the subscript is the average molecular weight of the polymer in Daltons, can be used. The hydrophilic polymeric group can be substituted with one to about six alkyl, fatty acid or fatty acid ester groups. Hydrophilic polymers that are substituted with a fatty acid or fatty acid ester group can be prepared by employing suitable methods. For example, a polymer comprising an amine group can be coupled to a carboxylate of the fatty acid or fatty acid ester, and an activated carboxylate (e.g., activated with N, N-carbonyl diimidazole) on a fatty acid or fatty acid ester can be coupled to a hydroxyl group on a polymer.

Fatty acids and fatty acid esters suitable for modifying antibodies of the invention can be saturated or can contain one or more units of unsaturation. Fatty acids that are suitable for modifying antibodies of the invention include, for example, n-dodecanoate (C12, laurate), n-tetradecanoate (C14, myristate), n-octadecanoate (C18, stearate), n-eicosanoate (C20, arachidate), n-docosanoate (C22, behenate), n-triacontanoate (C30), n-tetracontanoate (C40), cis-Δ9-octadecanoate (C18, oleate), all cis-Δ5,8,11,14-eicosatetraenoate (C20, arachidonate), octanedioic acid, tetradecanedioic acid, octadecanedioic acid, docosanedioic acid, and the like. Suitable fatty acid esters include mono-esters of dicarboxylic acids that comprise a linear or branched lower alkyl group. The lower alkyl group can comprise from one to about twelve, preferably, one to about six, carbon atoms.

The modified human antibodies and antigen-binding fragments can be prepared using suitable methods, such as by reaction with one or more modifying agents. A "modifying agent" as the term is used herein, refers to a suitable organic group (e.g., hydrophilic polymer, a fatty acid, a fatty acid ester) that comprises an activating group. An "activating group" is a chemical moiety or functional group that can, under appropriate conditions, react with a second chemical group thereby forming a covalent bond between the modifying agent and the second chemical group. For example, amine-reactive activating groups include electrophilic groups, such as tosylate, mesylate, halo (chloro, bromo, fluoro, iodo), N-hydroxysuccinimidyl esters (NHS), and the like. Activating groups that can react with thiols include, for example, maleimide, iodoacetyl, acrylolyl, pyridyl disulfides, 5-thiol-2-nitrobenzoic acid thiol (TNB-thiol), and the like. An aldehyde functional group can be coupled to amine- or hydrazide-containing molecules, and an azide group can react with a trivalent phosphorous group to form phosphoramidate or phosphorimide linkages. Suitable methods to introduce activating groups into molecules are known in the art (see for example, Hermanson, G. T., Bioconjugate Techniques, Academic Press: San Diego, CA (1996)). An activating group can be bonded directly to the organic group (e.g., hydrophilic polymer, fatty acid, fatty acid ester), or through a linker moiety, for example, a divalent C1-C12 group wherein one or more carbon atoms can be replaced by a heteroatom, such as oxygen, nitrogen or sulfur. Suitable linker moieties include, for example, tetraethylene glycol, -(CH2)3-, -NH-(CH2)6-NH-, -(CH2)2-NH- and -CH2-O-CH2-CH2-O-CH2-CH2-O-CH-NH-. Modifying agents that comprise a linker moiety can be produced, for example, by reacting a mono-Boc-alkyldiamine (e.g., mono-Boc-ethylenediamine, mono-Boc-diaminohexane) with a fatty acid in the presence of 1-ethyl-3-(3-dimethylaminopropyl) carbodiimide (EDC) to form an amide bond between the free amine and the fatty acid carboxylate. The Boc protecting group can be removed from the product by treatment with trifluoroacetic acid (TFA) to expose a primary amine that can be coupled to another carboxylate, as described, or can be reacted with maleic anhydride and the resulting product cyclized to produce an activated maleimido derivative of the fatty acid. (See, for example, Thompson, et al., WO 92/16221, the entire teachings of which are incorporated herein by reference.)

The modified antibodies can be produced by reacting a human antibody or antigen-binding fragment with a modifying agent. For example, the organic moieties can be bonded to the antibody in a non-site specific manner by employing an amine-reactive modifying agent, for example, an NHS ester of PEG. Modified human antibodies or antigen-binding fragments can also be prepared by reducing disulfide bonds (e.g., intra-chain disulfide bonds) of an antibody or antigen-binding fragment. The reduced antibody or antigen-binding fragment can then be reacted with a thiol-reactive modifying agent to produce the modified antibody of the invention. Modified human antibodies and antigen-binding fragments comprising an organic moiety that is bonded to specific sites of an antibody of the present invention can be prepared using suitable methods, such as reverse proteolysis (Fisch et al., Bioconjugate Chem., 3:147-153 (1992); Werlen et al., Bioconjugate Chem., 5:411-417 (1994); Kumaran et al., Protein Sci. 6(10):2233-2241 (1997); Itoh et al., Bioorg. Chem., 24(1): 59-68 (1996); Capellas et al., Biotechnol. Bioeng., 56(4):456-463 (1997)), and the methods described in Hermanson, G. T., Bioconjugate Techniques, Academic Press: San Diego, CA (1996).

The method of the present invention also uses an anti-IL-12/IL-23p40 or IL-23 antibody composition comprising at least one, at least two, at least three, at least four, at least five, at least six or more anti-IL-12/IL-23p40 or IL-23 antibodies thereof, as described herein and/or as known in the art that are provided in a non-naturally occurring composition, mixture or form. Such compositions comprise non-naturally occurring compositions comprising at least one or two full length, C- and/or N-terminally deleted variants, domains, fragments, or specified variants, of the anti-IL-12/IL-23p40 or IL-23 antibody amino acid sequence selected from the group consisting of 70-100% of the contiguous amino acids of the SEQ ID NOs above, or specified fragments, domains or variants thereof. Preferred anti-IL-12/IL-23p40 or IL-23 antibody compositions include at least one or two full length, fragments, domains or variants as at least one CDR or LBP containing portions of the anti-IL-12/IL-23p40 or IL-23 antibody sequence described herein, for example, 70-100% of the SEQ ID NOs above, or specified fragments, domains or variants thereof. Further preferred compositions comprise, for example, 40-99% of at least one of 70-100% of the SEQ ID NOs above, etc., or specified fragments, domains or variants thereof. Such composition percentages are by weight, volume, concentration, molarity, or molality as liquid or dry solutions, mixtures, suspension, emulsions, particles, powder, or colloids, as known in the art or as described herein.

### Antibody Compositions Comprising Further Therapeutically Active Ingredients

The antibody compositions used in the method of the invention can optionally further comprise an effective amount of at least one compound or protein selected from at least one of an anti-infective drug, a cardiovascular (CV) system drug, a central nervous system (CNS) drug, an autonomic nervous system (ANS) drug, a respiratory tract drug, a gastrointestinal (GI) tract drug, a hormonal drug, a drug for fluid or electrolyte balance, a hematologic drug, an antineoplastic, an immunomodulation drug, an ophthalmic, otic or nasal drug, a topical drug, a nutritional drug or the like. Such drugs are well known in the art, including formulations, indications, dosing and administration for each presented herein (see, e.g., Nursing 2001 Handbook of Drugs, 21st edition, Springhouse Corp., Springhouse, PA, 2001; Health Professional's Drug Guide 2001, ed., Shannon, Wilson, Stang, Prentice-Hall, Inc, Upper Saddle River, NJ; Pharmcotherapy Handbook, Wells et al., ed., Appleton & Lange, Stamford, CT, each entirely incorporated herein by reference).

By way of example of the drugs that can be combined with the antibodies for the method of the present invention, the anti-infective drug can be at least one selected from amebicides or at least one antiprotozoals, anthelmintics, antifungals, antimalarials, antituberculotics or at least one antileprotics, aminoglycosides, penicillins, cephalosporins, tetracyclines, sulfonamides, fluoroquinolones, antivirals, macrolide anti-infectives, and miscellaneous anti-infectives. The hormonal drug can be at least one selected from corticosteroids, androgens or at least one anabolic steroid, estrogen or at least one progestin, gonadotropin, antidiabetic drug or at least one glucagon, thyroid hormone, thyroid hormone antagonist, pituitary hormone, and parathyroid-like drug. The at least one cephalosporin can be at least one selected from cefaclor, cefadroxil, cefazolin sodium, cefdinir, cefepime hydrochloride, cefixime, cefmetazole sodium, cefonicid sodium, cefoperazone sodium, cefotaxime sodium, cefotetan disodium, cefoxitin sodium, cefpodoxime proxetil, cefprozil, ceftazidime, ceftibuten, ceftizoxime sodium, ceftriaxone sodium, cefuroxime axetil, cefuroxime sodium, cephalexin hydrochloride, cephalexin monohydrate, cephradine, and loracarbef.

The at least one coricosteroid can be at least one selected from betamethasone, betamethasone acetate or betamethasone sodium phosphate, betamethasone sodium phosphate, cortisone acetate, dexamethasone, dexamethasone acetate, dexamethasone sodium phosphate, fludrocortisone acetate, hydrocortisone, hydrocortisone acetate, hydrocortisone cypionate, hydrocortisone sodium phosphate, hydrocortisone sodium succinate, methylprednisolone, methylprednisolone acetate, methylprednisolone sodium succinate, prednisolone, prednisolone acetate, prednisolone sodium phosphate, prednisolone tebutate, prednisone, triamcinolone, triamcinolone acetonide, and triamcinolone diacetate. The at least one androgen or anabolic steroid can be at least one selected from danazol, fluoxymesterone, methyltestosterone, nandrolone decanoate, nandrolone phenpropionate, testosterone, testosterone cypionate, testosterone enanthate, testosterone propionate, and testosterone transdermal system.

The at least one immunosuppressant can be at least one selected from azathioprine, basiliximab, cyclosporine, daclizumab, lymphocyte immune globulin, muromonab-CD3, mycophenolate mofetil, mycophenolate mofetil hydrochloride, sirolimus, 6-mercaptopurine, methotrexate, mizoribine, and tacrolimus.

The at least one local anti-infective can be at least one selected from acyclovir, amphotericin B, azelaic acid cream, bacitracin, butoconazole nitrate, clindamycin phosphate, clotrimazole, econazole nitrate, erythromycin, gentamicin sulfate, ketoconazole, mafenide acetate, metronidazole (topical), miconazole nitrate, mupirocin, naftifine hydrochloride, neomycin sulfate, nitrofurazone, nystatin, silver sulfadiazine, terbinafine hydrochloride, terconazole, tetracycline hydrochloride, tioconazole, and tolnaftate. The at least one scabicide or pediculicide can be at least one selected from crotamiton, lindane, permethrin, and pyrethrins. The at least one topical corticosteroid can be at least one selected from betamethasone dipropionate, betamethasone valerate, clobetasol propionate, desonide, desoximetasone, dexamethasone, dexamethasone sodium phosphate, diflorasone diacetate, fluocinolone acetonide, fluocinonide, flurandrenolide, fluticasone propionate, halcionide, hydrocortisone, hydrocortisone acetate, hydrocortisone butyrate, hydrocorisone valerate, mometasone furoate, and triamcinolone acetonide. (See, e.g., pp. 1098-1136 of Nursing 2001 Drug Handbook.)

Anti-IL-12/IL-23p40 or IL-23 antibody compositions can further comprise at least one of any suitable and effective amount of a composition or pharmaceutical composition comprising at least one anti-IL-12/IL-23p40 or IL-23 antibody contacted or administered to a cell, tissue, organ, animal or subject in need of such modulation, treatment or therapy, optionally further comprising at least one selected from at least one TNF antagonist (e.g., but not limited to a TNF chemical or protein antagonist, TNF monoclonal or polyclonal antibody or fragment, a soluble TNF receptor (e.g., p55, p70 or p85) or fragment, fusion polypeptides thereof, or a small molecule TNF antagonist, e.g., TNF binding protein I or II (TBP-1 or TBP-II), nerelimonmab, infliximab, eternacept, CDP-571, CDP-870, afelimomab, lenercept, and the like), an antirheumatic (e.g., methotrexate, auranofin, aurothioglucose, azathioprine, etanercept, gold sodium thiomalate, hydroxychloroquine sulfate, leflunomide, sulfasalzine), an immunization, an immunoglobulin, an immunosuppressive (e.g., azathioprine, basiliximab, cyclosporine, daclizumab), a cytokine or a cytokine antagonist. Non-limiting examples of such cytokines include, but are not limited to, any of IL-1 to IL-23 et al. (e.g., IL-1, IL-2, etc.). Suitable dosages are well known in the art. See, e.g., Wells et al., eds., Pharmacotherapy Handbook, 2nd Edition, Appleton and Lange, Stamford, CT (2000); PDR Pharmacopoeia, Tarascon Pocket Pharmacopoeia 2000, Deluxe Edition, Tarascon Publishing, Loma Linda, CA (2000), each of which references are entirely incorporated herein by reference.

Anti-IL-12/IL-23p40 or IL-23 antibody compounds, compositions or combinations used in the method of the present invention can further comprise at least one of any suitable auxiliary, such as, but not limited to, diluent, binder, stabilizer, buffers, salts, lipophilic solvents, preservative, adjuvant or the like. Pharmaceutically acceptable auxiliaries are preferred. Non-limiting examples of, and methods of preparing such sterile solutions are well known in the art, such as, but limited to, Gennaro, Ed., Remington's Pharmaceutical Sciences, 18th Edition, Mack Publishing Co. (Easton, PA) 1990. Pharmaceutically acceptable carriers can be routinely selected that are suitable for the mode of administration, solubility and/or stability of the anti-IL-12/IL-23p40, fragment or variant composition as well known in the art or as described herein.

Pharmaceutical excipients and additives useful in the present composition include, but are not limited to, proteins, peptides, amino acids, lipids, and carbohydrates (e.g., sugars, including monosaccharides, di-, tri-, tetra-, and oligosaccharides; derivatized sugars, such as alditols, aldonic acids, esterified sugars and the like; and polysaccharides or sugar polymers), which can be present singly or in combination, comprising alone or in combination 1-99.99% by weight or volume. Exemplary protein excipients include serum albumin, such as human serum albumin (HSA), recombinant human albumin (rHA), gelatin, casein, and the like. Representative amino acid/antibody components, which can also function in a buffering capacity, include alanine, glycine, arginine, betaine, histidine, glutamic acid, aspartic acid, cysteine, lysine, leucine, isoleucine, valine, methionine, phenylalanine, aspartame, and the like. One preferred amino acid is glycine.

Carbohydrate excipients suitable for use in the invention include, for example, monosaccharides, such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol sorbitol (glucitol), myoinositol and the like. Preferred carbohydrate excipients for use in the present invention are mannitol, trehalose, and raffinose.

Anti-IL-12/IL-23p40 or IL-23 antibody compositions can also include a buffer or a pH adjusting agent; typically, the buffer is a salt prepared from an organic acid or base. Representative buffers include organic acid salts, such as salts of citric acid, ascorbic acid, gluconic acid, carbonic acid, tartaric acid, succinic acid, acetic acid, or phthalic acid; Tris, tromethamine hydrochloride, or phosphate buffers. Preferred buffers for use in the present compositions are organic acid salts, such as citrate.

Additionally, anti-IL-12/IL-23p40 or IL-23 antibody compositions can include polymeric excipients/additives, such as polyvinylpyrrolidones, ficolls (a polymeric sugar), dextrates (e.g., cyclodextrins, such as 2-hydroxypropyl-β-cyclodextrin), polyethylene glycols, flavoring agents, antimicrobial agents, sweeteners, antioxidants, antistatic agents, surfactants (e.g., polysorbates, such as "TWEEN 20" and "TWEEN 80"), lipids (e.g., phospholipids, fatty acids), steroids (e.g., cholesterol), and chelating agents (e.g., EDTA).

These and additional known pharmaceutical excipients and/or additives suitable for use in the anti-IL-12/IL-23p40 or IL-23 antibody, portion or variant compositions according to the invention are known in the art, e.g., as listed in "Remington: The Science & Practice of Pharmacy," 19th ed., Williams & Williams, (1995), and in the "Physician's Desk Reference," 52nd ed., Medical Economics, Montvale, NJ (1998), the disclosures of which are entirely incorporated herein by reference. Preferred carrier or excipient materials are carbohydrates (e.g., saccharides and alditols) and buffers (e.g., citrate) or polymeric agents. An exemplary carrier molecule is the mucopolysaccharide, hyaluronic acid, which can be useful for intraarticular delivery.

### Formulations

As noted above, the invention provides for stable formulations, which preferably comprise a phosphate buffer with saline or a chosen salt, as well as preserved solutions and formulations containing a preservative as well as multi-use preserved formulations suitable for pharmaceutical or veterinary use, comprising at least one anti-IL-12/IL-23p40 or IL-23 antibody in a pharmaceutically acceptable formulation. Preserved formulations contain at least one known preservative or optionally selected from the group consisting of at least one phenol, m-cresol, p-cresol, o-cresol, chlorocresol, benzyl alcohol, phenylmercuric nitrite, phenoxyethanol, formaldehyde, chlorobutanol, magnesium chloride (e.g., hexahydrate), alkylparaben (methyl, ethyl, propyl, butyl and the like), benzalkonium chloride, benzethonium chloride, sodium dehydroacetate and thimerosal, or mixtures thereof in an aqueous diluent. Any suitable concentration or mixture can be used as known in the art, such as 0.001-5%, or any range or value therein, such as, but not limited to 0.001, 0.003, 0.005, 0.009, 0.01, 0.02, 0.03, 0.05, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.3, 4.5, 4.6, 4.7, 4.8, 4.9, or any range or value therein. Non-limiting examples include, no preservative, 0.1-2% m-cresol (e.g., 0.2, 0.3. 0.4, 0.5, 0.9, 1.0%), 0.1-3% benzyl alcohol (e.g., 0.5, 0.9, 1.1, 1.5, 1.9, 2.0, 2.5%), 0.001-0.5% thimerosal (e.g., 0.005, 0.01), 0.001-2.0% phenol (e.g., 0.05, 0.25, 0.28, 0.5, 0.9, 1.0%), 0.0005-1.0% alkylparaben(s) (e.g., 0.00075, 0.0009, 0.001, 0.002, 0.005, 0.0075, 0.009, 0.01, 0.02, 0.05, 0.075, 0.09, 0.1, 0.2, 0.3, 0.5, 0.75, 0.9, 1.0%), and the like.

As noted above, the method of the invention uses an article of manufacture, comprising packaging material and at least one vial comprising a solution of at least one anti-IL-12/IL-23p40 or IL-23 antibody with the prescribed buffers and/or preservatives, optionally in an aqueous diluent, wherein said packaging material comprises a label that indicates that such solution can be held over a period of 1, 2, 3, 4, 5, 6, 9, 12, 18, 20, 24, 30, 36, 40, 48, 54, 60, 66, 72 hours or greater. The invention further uses an article of manufacture, comprising packaging material, a first vial comprising lyophilized anti-IL-12/IL-23p40 or IL-23 antibody, and a second vial comprising an aqueous diluent of prescribed buffer or preservative, wherein said packaging material comprises a label that instructs a subject to reconstitute the anti-IL-12/IL-23p40 or IL-23 antibody in the aqueous diluent to form a solution that can be held over a period of twenty-four hours or greater.

The anti-IL-12/IL-23p40 or IL-23 antibody used in accordance with the present invention can be produced by recombinant means, including from mammalian cell or transgenic preparations, or can be purified from other biological sources, as described herein or as known in the art.

The range of the anti-IL-12/IL-23p40 or IL-23 antibody includes amounts yielding upon reconstitution, if in a wet/dry system, concentrations from about 1.0 µg/ml to about 1000 mg/ml, although lower and higher concentrations are operable and are dependent on the intended delivery vehicle, e.g., solution formulations will differ from transdermal patch, pulmonary, transmucosal, or osmotic or micro pump methods.

Preferably, the aqueous diluent optionally further comprises a pharmaceutically acceptable preservative. Preferred preservatives include those selected from the group consisting of phenol, m-cresol, p-cresol, o-cresol, chlorocresol, benzyl alcohol, alkylparaben (methyl, ethyl, propyl, butyl and the like), benzalkonium chloride, benzethonium chloride, sodium dehydroacetate and thimerosal, or mixtures thereof. The concentration of preservative used in the formulation is a concentration sufficient to yield an anti-microbial effect. Such concentrations are dependent on the preservative selected and are readily determined by the skilled artisan.

Other excipients, e.g., isotonicity agents, buffers, antioxidants, and preservative enhancers, can be optionally and preferably added to the diluent. An isotonicity agent, such as glycerin, is commonly used at known concentrations. A physiologically tolerated buffer is preferably added to provide improved pH control. The formulations can cover a wide range of pHs, such as from about pH 4 to about pH 10, and preferred ranges from about pH 5 to about pH 9, and a most preferred range of about 6.0 to about 8.0. Preferably, the formulations of the present invention have a pH between about 6.8 and about 7.8. Preferred buffers include phosphate buffers, most preferably, sodium phosphate, particularly, phosphate buffered saline (PBS).

Other additives, such as a pharmaceutically acceptable solubilizers like Tween 20 (polyoxyethylene (20) sorbitan monolaurate), Tween 40 (polyoxyethylene (20) sorbitan monopalmitate), Tween 80 (polyoxyethylene (20) sorbitan monooleate), Pluronic F68 (polyoxyethylene polyoxypropylene block copolymers), and PEG (polyethylene glycol) or nonionic surfactants, such as polysorbate 20 or 80 or poloxamer 184 or 188, Pluronic^{®} polyls, other block co-polymers, and chelators, such as EDTA and EGTA, can optionally be added to the formulations or compositions to reduce aggregation. These additives are particularly useful if a pump or plastic container is used to administer the formulation. The presence of pharmaceutically acceptable surfactant mitigates the propensity for the protein to aggregate.

The formulations can be prepared by a process which comprises mixing at least one anti-IL-12/IL-23p40 or IL-23 antibody and a preservative selected from the group consisting of phenol, m-cresol, p-cresol, o-cresol, chlorocresol, benzyl alcohol, alkylparaben, (methyl, ethyl, propyl, butyl and the like), benzalkonium chloride, benzethonium chloride, sodium dehydroacetate and thimerosal or mixtures thereof in an aqueous diluent. Mixing the at least one anti-IL-12/IL-23p40 or IL-23 specific antibody and preservative in an aqueous diluent is carried out using conventional dissolution and mixing procedures. To prepare a suitable formulation, for example, a measured amount of at least one anti-IL-12/IL-23p40 or IL-23 antibody in buffered solution is combined with the desired preservative in a buffered solution in quantities sufficient to provide the protein and preservative at the desired concentrations. Variations of this process would be recognized by one of ordinary skill in the art. For example, the order the components are added, whether additional additives are used, the temperature and pH at which the formulation is prepared, are all factors that can be optimized for the concentration and means of administration used.

The formulations can be provided to subjects as clear solutions or as dual vials comprising a vial of lyophilized anti-IL-12/IL-23p40 or IL-23 specific antibody that is reconstituted with a second vial containing water, a preservative and/or excipients, preferably, a phosphate buffer and/or saline and a chosen salt, in an aqueous diluent. Either a single solution vial or dual vial requiring reconstitution can be reused multiple times and can suffice for a single or multiple cycles of subject treatment and thus can provide a more convenient treatment regimen than currently available.

The present articles of manufacture are useful for administration over a period ranging from immediate to twenty-four hours or greater. Accordingly, the presently claimed articles of manufacture offer significant advantages to the subject. Formulations of the invention can optionally be safely stored at temperatures of from about 2°C to about 40°C and retain the biologically activity of the protein for extended periods of time, thus allowing a package label indicating that the solution can be held and/or used over a period of 6, 12, 18, 24, 36, 48, 72, or 96 hours or greater. If preserved diluent is used, such label can include use up to 1-12 months, one-half, one and a half, and/or two years.

The solutions of anti-IL-12/IL-23p40 or IL-23 specific antibody can be prepared by a process that comprises mixing at least one antibody in an aqueous diluent. Mixing is carried out using conventional dissolution and mixing procedures. To prepare a suitable diluent, for example, a measured amount of at least one antibody in water or buffer is combined in quantities sufficient to provide the protein and, optionally, a preservative or buffer at the desired concentrations. Variations of this process would be recognized by one of ordinary skill in the art. For example, the order the components are added, whether additional additives are used, the temperature and pH at which the formulation is prepared, are all factors that can be optimized for the concentration and means of administration used.

The claimed products can be provided to subjects as clear solutions or as dual vials comprising a vial of lyophilized at least one anti-IL-12/IL-23p40 or IL-23 specific antibody that is reconstituted with a second vial containing the aqueous diluent. Either a single solution vial or dual vial requiring reconstitution can be reused multiple times and can suffice for a single or multiple cycles of subject treatment and thus provides a more convenient treatment regimen than currently available.

The claimed products can be provided indirectly to subjects by providing to pharmacies, clinics, or other such institutions and facilities, clear solutions or dual vials comprising a vial of lyophilized at least one anti-IL-12/IL-23p40 or IL-23 specific antibody that is reconstituted with a second vial containing the aqueous diluent. The clear solution in this case can be up to one liter or even larger in size, providing a large reservoir from which smaller portions of the at least one antibody solution can be retrieved one or multiple times for transfer into smaller vials and provided by the pharmacy or clinic to their customers and/or subjects.

Recognized devices comprising single vial systems include pen-injector devices for delivery of a solution, such as BD Pens, BD Autojector^{®}, Humaject^{®}, NovoPen^{®}, B-D^{®}Pen, AutoPen^{®}, and OptiPen^{®}, GenotropinPen^{®}, Genotronorm Pen^{®}, Humatro Pen^{®}, Reco-Pen^{®}, Roferon Pen^{®}, Biojector^{®}, Iject^{®}, J-tip Needle-Free Injector^{®}, Intraject^{®}, Medi-Ject^{®}, Smartject^{®} e.g., as made or developed by Becton Dickensen (Franklin Lakes, NJ, www.bectondickenson.com), Disetronic (Burgdorf, Switzerland, www.disetronic.com; Bioject, Portland, Oregon (www.bioject.com); National Medical Products, Weston Medical (Peterborough, UK, www.weston-medical.com), Medi-Ject Corp (Minneapolis, MN, www.mediject.com), and similarly suitable devices. Recognized devices comprising a dual vial system include those pen-injector systems for reconstituting a lyophilized drug in a cartridge for delivery of the reconstituted solution, such as the HumatroPen^{®}. Examples of other devices suitable include pre-filled syringes, auto-injectors, needle free injectors, and needle free IV infusion sets.

The products can include packaging material. The packaging material provides, in addition to the information required by the regulatory agencies, the conditions under which the product can be used. The packaging material of the present invention provides instructions to the subject, as applicable, to reconstitute the at least one anti-IL-12/IL-23p40 or IL-23 antibody in the aqueous diluent to form a solution and to use the solution over a period of 2-24 hours or greater for the two vial, wet/dry, product. For the single vial, solution product, pre-filled syringe or auto-injector, the label indicates that such solution can be used over a period of 2-24 hours or greater. The products are useful for human pharmaceutical product use.

The formulations used in the method of the present invention can be prepared by a process that comprises mixing an anti-IL-12/IL-23p40 and a selected buffer, preferably, a phosphate buffer containing saline or a chosen salt. Mixing the anti-IL-12/IL-23p40 antibody and buffer in an aqueous diluent is carried out using conventional dissolution and mixing procedures. To prepare a suitable formulation, for example, a measured amount of at least one antibody in water or buffer is combined with the desired buffering agent in water in quantities sufficient to provide the protein and buffer at the desired concentrations. Variations of this process would be recognized by one of ordinary skill in the art. For example, the order the components are added, whether additional additives are used, the temperature and pH at which the formulation is prepared, are all factors that can be optimized for the concentration and means of administration used.

The method of the invention provides pharmaceutical compositions comprising various formulations useful and acceptable for administration to a human or animal subject. Such pharmaceutical compositions are prepared using water at "standard state" as the diluent and routine methods well known to those of ordinary skill in the art. For example, buffering components such as histidine and histidine monohydrochloride hydrate, can be provided first followed by the addition of an appropriate, non-final volume of water diluent, sucrose and polysorbate 80 at "standard state." Isolated antibody can then be added. Last, the volume of the pharmaceutical composition is adjusted to the desired final volume under "standard state" conditions using water as the diluent. Those skilled in the art will recognize a number of other methods suitable for the preparation of the pharmaceutical compositions.

The pharmaceutical compositions can be aqueous solutions or suspensions comprising the indicated mass of each constituent per unit of water volume or having an indicated pH at "standard state." As used herein, the term "standard state" means a temperature of 25°C +/- 2°C and a pressure of 1 atmosphere. The term "standard state" is not used in the art to refer to a single art recognized set of temperatures or pressure, but is instead a reference state that specifies temperatures and pressure to be used to describe a solution or suspension with a particular composition under the reference "standard state" conditions. This is because the volume of a solution is, in part, a function of temperature and pressure. Those skilled in the art will recognize that pharmaceutical compositions equivalent to those disclosed here can be produced at other temperatures and pressures. Whether such pharmaceutical compositions are equivalent to those disclosed here should be determined under the "standard state" conditions defined above (e.g. 25°C +/- 2°C and a pressure of 1 atmosphere).

Importantly, such pharmaceutical compositions can contain component masses "about" a certain value (e.g. "about 0.53 mg L-histidine") per unit volume of the pharmaceutical composition or have pH values about a certain value. A component mass present in a pharmaceutical composition or pH value is "about" a given numerical value if the isolated antibody present in the pharmaceutical composition is able to bind a peptide chain while the isolated antibody is present in the pharmaceutical composition or after the isolated antibody has been removed from the pharmaceutical composition (e.g., by dilution). Stated differently, a value, such as a component mass value or pH value, is "about" a given numerical value when the binding activity of the isolated antibody is maintained and detectable after placing the isolated antibody in the pharmaceutical composition.

Competition binding analysis is performed to determine if the IL-12/IL-23p40 or IL-23 specific mAbs bind to similar or different epitopes and/or compete with each other. Abs are individually coated on ELISA plates. Competing mAbs are added, followed by the addition of biotinylated hrIL-12 or IL-23. For positive control, the same mAb for coating can be used as the competing mAb ("self-competition"). IL-12/IL-23p40 or IL-23 binding is detected using streptavidin. These results demonstrate whether the mAbs recognize similar or partially overlapping epitopes on IL-12/IL-23p40 or IL-23.

In one embodiment of the pharmaceutical compositions, the isolated antibody concentration is from about 77 to about 104 mg per ml of the pharmaceutical composition. In another embodiment of the pharmaceutical compositions the pH is from about 5.5 to about 6.5.

The stable or preserved formulations can be provided to subjects as clear solutions or as dual vials comprising a vial of lyophilized at least one anti-IL-12/IL-23p40 that is reconstituted with a second vial containing a preservative or buffer and excipients in an aqueous diluent. Either a single solution vial or dual vial requiring reconstitution can be reused multiple times and can suffice for a single or multiple cycles of subject treatment and thus provides a more convenient treatment regimen than currently available.

Other formulations or methods of stabilizing the anti-IL-12/IL-23p40 can result in other than a clear solution of lyophilized powder comprising the antibody. Among non-clear solutions are formulations comprising particulate suspensions, said particulates being a composition containing the anti-IL-12/IL-23p40 in a structure of variable dimension and known variously as a microsphere, microparticle, nanoparticle, nanosphere, or liposome. Such relatively homogenous, essentially spherical, particulate formulations containing an active agent can be formed by contacting an aqueous phase containing the active agent and a polymer and a nonaqueous phase followed by evaporation of the nonaqueous phase to cause the coalescence of particles from the aqueous phase as taught in U.S. 4,589,330. Porous microparticles can be prepared using a first phase containing active agent and a polymer dispersed in a continuous solvent and removing said solvent from the suspension by freeze-drying or dilution-extraction-precipitation as taught in U.S. 4,818,542. Preferred polymers for such preparations are natural or synthetic copolymers or polymers selected from the group consisting of glelatin agar, starch, arabinogalactan, albumin, collagen, polyglycolic acid, polylactic aced, glycolide-L(-) lactide poly(episilon-caprolactone, poly(epsilon-caprolactone-CO-lactic acid), poly(epsilon-caprolactone-CO-glycolic acid), poly(β-hydroxy butyric acid), polyethylene oxide, polyethylene, poly(alkyl-2-cyanoacrylate), poly(hydroxyethyl methacrylate), polyamides, poly(amino acids), poly(2-hydroxyethyl DL-aspartamide), poly(ester urea), poly(L-phenylalanine/ethylene glycol/1,6-diisocyanatohexane) and poly(methyl methacrylate). Particularly preferred polymers are polyesters, such as polyglycolic acid, polylactic aced, glycolide-L(-) lactide poly(episilon-caprolactone, poly(epsilon-caprolactone-CO-lactic acid), and poly(epsilon-caprolactone-CO-glycolic acid. Solvents useful for dissolving the polymer and/or the active include: water, hexafluoroisopropanol, methylenechloride, tetrahydrofuran, hexane, benzene, or hexafluoroacetone sesquihydrate. The process of dispersing the active containing phase with a second phase can include pressure forcing said first phase through an orifice in a nozzle to affect droplet formation.

Dry powder formulations can result from processes other than lyophilization, such as by spray drying or solvent extraction by evaporation or by precipitation of a crystalline composition followed by one or more steps to remove aqueous or non-aqueous solvent. Preparation of a spray-dried antibody preparation is taught in U.S. 6,019,968. The antibody-based dry powder compositions can be produced by spray drying solutions or slurries of the antibody and, optionally, excipients, in a solvent under conditions to provide a respirable dry powder. Solvents can include polar compounds, such as water and ethanol, which can be readily dried. Antibody stability can be enhanced by performing the spray drying procedures in the absence of oxygen, such as under a nitrogen blanket or by using nitrogen as the drying gas. Another relatively dry formulation is a dispersion of a plurality of perforated microstructures dispersed in a suspension medium that typically comprises a hydrofluoroalkane propellant as taught in WO 9916419. The stabilized dispersions can be administered to the lung of a subject using a metered dose inhaler. Equipment useful in the commercial manufacture of spray dried medicaments are manufactured by Buchi Ltd. or Niro Corp.

An anti-IL-12/IL-23p40 in either the stable or preserved formulations or solutions described herein, can be administered to a subject in accordance with the present invention via a variety of delivery methods including SC or IM injection; transdermal, pulmonary, transmucosal, implant, osmotic pump, cartridge, micro pump, or other means appreciated by the skilled artisan, as well-known in the art.

### Therapeutic Applications

The present invention also provides a method for modulating or treating ulcerative colitis, in a cell, tissue, organ, animal, or subject, as known in the art or as described herein, using at least one IL-23 antibody of the present invention, e.g., administering or contacting the cell, tissue, organ, animal, or subject with a therapeutic effective amount of IL-12/IL-23p40 or IL-23 specific antibody.

Any method of the present invention can comprise administering an effective amount of a composition or pharmaceutical composition comprising an IL-12/IL-23p40 to a cell, tissue, organ, animal or subject in need of such modulation, treatment or therapy. Such a method can optionally further comprise co-administration or combination therapy for treating such diseases or disorders, wherein the administering of said at least one IL-12/IL-23p40, specified portion or variant thereof, further comprises administering, before concurrently, and/or after, at least one selected from at least one TNF antagonist (e.g., but not limited to, a TNF chemical or protein antagonist, TNF monoclonal or polyclonal antibody or fragment, a soluble TNF receptor (e.g., p55, p70 or p85) or fragment, fusion polypeptides thereof, or a small molecule TNF antagonist, e.g., TNF binding protein I or II (TBP-1 or TBP-II), nerelimonmab, infliximab, eternacept (Enbrel^{™}), adalimulab (Humira^{™}), CDP-571, CDP-870, afelimomab, lenercept, and the like), an antirheumatic (e.g., methotrexate, auranofin, aurothioglucose, azathioprine, gold sodium thiomalate, hydroxychloroquine sulfate, leflunomide, sulfasalzine), a muscle relaxant, a narcotic, a non-steroid anti-inflammatory drug (NSAID) (e.g., 5-aminosalicylate), an analgesic, an anesthetic, a sedative, a local anesthetic, a neuromuscular blocker, an antimicrobial (e.g., aminoglycoside, an antifungal, an antiparasitic, an antiviral, a carbapenem, cephalosporin, a flurorquinolone, a macrolide, a penicillin, a sulfonamide, a tetracycline, another antimicrobial), an antipsoriatic, a corticosteriod, an anabolic steroid, a diabetes related agent, a mineral, a nutritional, a thyroid agent, a vitamin, a calcium related hormone, an antidiarrheal, an antitussive, an antiemetic, an antiulcer, a laxative, an anticoagulant, an erythropoietin (e.g., epoetin alpha), a filgrastim (e.g., G-CSF, Neupogen), a sargramostim (GM-CSF, Leukine), an immunization, an immunoglobulin, an immunosuppressive (e.g., basiliximab, cyclosporine, daclizumab), a growth hormone, a hormone replacement drug, an estrogen receptor modulator, a mydriatic, a cycloplegic, an alkylating agent, an antimetabolite, a mitotic inhibitor, a radiopharmaceutical, an antidepressant, antimanic agent, an antipsychotic, an anxiolytic, a hypnotic, a sympathomimetic, a stimulant, donepezil, tacrine, an asthma medication, a beta agonist, an inhaled steroid, a leukotriene inhibitor, a methylxanthine, a cromolyn, an epinephrine or analog, dornase alpha (Pulmozyme), a cytokine or a cytokine antagonist. Suitable dosages are well known in the art. See, e.g., Wells et al., eds., Pharmacotherapy Handbook, 2nd Edition, Appleton and Lange, Stamford, CT (2000); PDR Pharmacopoeia, Tarascon Pocket Pharmacopoeia 2000, Deluxe Edition, Tarascon Publishing, Loma Linda, CA (2000); Nursing 2001 Handbook of Drugs, 21st edition, Springhouse Corp., Springhouse, PA, 2001; Health Professional's Drug Guide 2001, ed., Shannon, Wilson, Stang, Prentice-Hall, Inc, Upper Saddle River, NJ, each of which references are entirely incorporated herein by reference.

### Therapeutic Treatments

Treatment of ulcerative colitis is affected by administering an effective amount or dosage of an anti-IL-12/23p40 composition in a subject in need thereof. The dosage administered can vary depending upon known factors, such as the pharmacodynamic characteristics of the particular agent, and its mode and route of administration; age, health, and weight of the recipient; nature and extent of symptoms, kind of concurrent treatment, frequency of treatment, and the effect desired. In some instances, to achieve the desired therapeutic amount, it can be necessary to provide for repeated administration, i.e., repeated individual administrations of a particular monitored or metered dose, where the individual administrations are repeated until the desired daily dose or effect is achieved.

In one exemplary regimen of providing safe and effective treatment of severely active UC in a subject in need thereof, a total dosage of about 130 mg of an anti-IL-12/IL-23p40 antibody is administered intravenously to the subject per administration. For example, the total volume of the composition administered is appropriately adjusted to provide to the subject the target dosage of the antibody at 80 mg, 90 mg, 100 mg, 110 mg, 120 mg, 130 mg, 140 mg, 150 mg, 160 mg, 170 mg or 180 mg per administration.

In another exemplary regimen of providing safe and effective treatment of severely active UC in a subject in need thereof, a total dosage of about 6.0 mg/kg ± 1.5 mg/kg of an anti-IL-12/IL-23p40 antibody is administered intravenously to the subject per administration. For example, the total volume of the composition administered is appropriately adjusted to provide to the subject the target dosage of the antibody at 3.0 mg/kg, 3.5 mg/kg, 4.0 mg/kg, 4.5 mg/kg, 5.0 mg/kg, 5.5 mg/kg, 6.0 mg/kg, 6.5 mg/kg, 7.0 mg/kg, 7.5 mg/kg, 8.0 mg/kg, 8.5 mg/kg, or 9.0 mg/kg body weight of the subject per administration.

The total dosage of an anti-IL-12/IL-23p40 antibody to be administered to the subject per administration can be administered by intravenous infusion over a period of about 30 minutes to 180 minutes, preferably 60 minutes to 120 minutes, such as 30 minutes, 60 minutes, 90 minutes, 120 minutes, 150 minutes, or 180 minutes.

In yet another exemplary regimen of providing safe and effective treatment of severely active UC in a subject in need thereof, a total dosage of about 90 mg of an anti-IL-12/IL-23p40 antibody is administered subcutaneously to the subject per administration. For example, the total volume of the composition administered is appropriately adjusted to provide to the subject the target dosage of the antibody at 40 mg, 50 mg, 60 mg, 70 mg, 80 mg, 90 mg, 100 mg, 110 mg, 120 mg, 130 mg or 140 mg per administration. The target dosage per administration can be administered in a single subcutaneous injection or in multiple subcutaneous injections, such as 1, 2, 3, 4, 5, or more subcutaneous injections.

The total dosage of the anti-IL-12/IL-23p40 antibody can be administered once per day, once per week, once per month, once every six months, etc. for a period of one day, one week, one month, six months, 1 year, 2 years or longer. Multiple administrations of the anti-IL-12/IL-23p40 antibody, each at a total dosage of described herein, can be administered to a subject in need thereof.

Dosage forms (composition) suitable for internal administration generally contain from about 0.001 milligram to about 500 milligrams of active ingredient per unit or container.

For parenteral administration, the antibody can be formulated as a solution, suspension, emulsion, particle, powder, or lyophilized powder in association, or separately provided, with a pharmaceutically acceptable parenteral vehicle. Examples of such vehicles are water, saline, Ringer's solution, dextrose solution, and 1-10% human serum albumin. Liposomes and nonaqueous vehicles, such as fixed oils, can also be used. The vehicle or lyophilized powder can contain additives that maintain isotonicity (e.g., sodium chloride, mannitol) and chemical stability (e.g., buffers and preservatives). The formulation is sterilized by known or suitable techniques.

Suitable pharmaceutical carriers are described in the most recent edition of Remington's Pharmaceutical Sciences, A. Osol, a standard reference text in this field.

Many known and developed modes can be used according to the present invention for administering pharmaceutically effective amounts of an IL-12/IL-23p40 antibody. IL-12/IL-23p40 or IL-23 antibodies of the present invention can be delivered in a carrier, as a solution, emulsion, colloid, or suspension, or as a dry powder, using any of a variety of devices and methods suitable for administration by inhalation or other modes described here within or known in the art.

Formulations for parenteral administration can contain as common excipients sterile water or saline, polyalkylene glycols, such as polyethylene glycol, oils of vegetable origin, hydrogenated naphthalenes and the like. Aqueous or oily suspensions for injection can be prepared by using an appropriate emulsifier or humidifier and a suspending agent, according to known methods. Agents for injection can be a non-toxic, non-orally administrable diluting agent, such as aqueous solution, a sterile injectable solution or suspension in a solvent. As the usable vehicle or solvent, water, Ringer's solution, isotonic saline, etc. are allowed; as an ordinary solvent or suspending solvent, sterile involatile oil can be used. For these purposes, any kind of involatile oil and fatty acid can be used, including natural or synthetic or semisynthetic fatty oils or fatty acids; natural or synthetic or semisynthtetic mono- or di- or tri-glycerides. Parental administration is known in the art and includes, but is not limited to, conventional means of injections, a gas pressured needle-less injection device as described in U.S. Pat. No. 5,851,198, and a laser perforator device as described in U.S. Pat. No. 5,839,446 entirely incorporated herein by reference.

### Alternative Delivery

The invention further relates to the administration of an anti-IL-12/IL-23p40 or IL-23 antibody by parenteral, subcutaneous, intramuscular, intravenous, intrarticular, intrabronchial, intraabdominal, intracapsular, intracartilaginous, intracavitary, intracelial, intracerebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, intralesional, bolus, vaginal, rectal, buccal, sublingual, intranasal, or transdermal means. An anti-IL-12/IL-23p40 or IL-23 antibody composition can be prepared for use for parenteral (subcutaneous, intramuscular or intravenous) or any other administration particularly in the form of liquid solutions or suspensions; for use in vaginal or rectal administration particularly in semisolid forms, such as, but not limited to, creams and suppositories; for buccal, or sublingual administration, such as, but not limited to, in the form of tablets or capsules; or intranasally, such as, but not limited to, the form of powders, nasal drops or aerosols or certain agents; or transdermally, such as not limited to a gel, ointment, lotion, suspension or patch delivery system with chemical enhancers such as dimethyl sulfoxide to either modify the skin structure or to increase the drug concentration in the transdermal patch (Junginger, et al. In "Drug Permeation Enhancement;" Hsieh, D. S., Eds., pp. 59-90 (Marcel Dekker, Inc. New York 1994, entirely incorporated herein by reference), or with oxidizing agents that enable the application of formulations containing proteins and peptides onto the skin (WO 98/53847), or applications of electric fields to create transient transport pathways, such as electroporation, or to increase the mobility of charged drugs through the skin, such as iontophoresis, or application of ultrasound, such as sonophoresis (U.S. Pat. Nos. 4,309,989 and 4,767,402) (the above publications and patents being entirely incorporated herein by reference).

### EMBODIMENTS

The invention provides also the following non-limiting embodiments.
1. A method of treating moderately to severely active ulcerative colitis (UC) in a subject in need thereof, comprising administering to the subject a pharmaceutical composition comprising a clinically proven safe and clinically proven effective amount of an anti-IL-12/IL-23p40 antibody, wherein the antibody comprises a heavy chain variable region and a light chain variable region, the heavy chain variable region comprising: a complementarity determining region heavy chain 1 (CDRH1) amino acid sequence of SEQ ID NO: 1; a CDRH2 amino acid sequence of SEQ ID NO:2; and a CDRH3 amino acid sequence of SEQ ID NO:3; and the light chain variable region comprising: a complementarity determining region light chain 1 (CDRL1) amino acid sequence of SEQ ID NO:4; a CDRL2 amino acid sequence of SEQ ID NO:5; and a CDRL3 amino acid sequence of SEQ ID NO:6.
2. The method of embodiment 1, wherein the antibody comprises the heavy chain variable region of the amino acid sequence of SEQ ID NO:7 and the light chain variable region of the amino acid sequence of SEQ ID NO:8.
3. The method of embodiment 1, wherein the antibody comprises a heavy chain of the amino acid sequence of SEQ ID NO: 10 and a light chain of the amino acid sequence of SEQ ID NO: 11.
4. The method of any one of embodiments 1 to 3, wherein the antibody is administered intravenously to the subject, preferably at week 0 of the treatment, at a dosage of about 6.0 mg/kg body weight of the subject or 130 mg per administration.
5. The method of any one of embodiments 1 to 4, wherein the antibody is further administered subcutaneously to the subject, preferably at week 8 of the treatment, at a dosage of about 90 mg per administration.
6. The method of any one of embodiments 1 to 5, wherein the subject had previously failed or were intolerant of at least one therapy selected from the group consisting of an anti-TNF, vedolizumab, corticosteroids, azathioprine (AZA), and 6 mercaptopurine (6 MP), or the subject had demonstrated corticosteroid dependence.
7. The method of embodiment 5, wherein the antibody is administered in a maintenance dose every 8 weeks after the treatment at week 8 or every 12 weeks after the treatment at week 8.
8. The method of embodiment 7, wherein the subject is a responder to the treatment with the antibody and is identified as having a clinical remission based on at least one of the global definition and the US definition by week 16, preferably by week 8, more preferably by week 2, of the treatment and the clinical remission continues at least 44 weeks after week 0.
9. The method of embodiment 8, wherein the subject is in corticosteroid-free clinical remission at least 44 weeks after week 0.
10. The method of embodiment 7, wherein the subject is a responder to the treatment with the antibody and is identified as having an endoscopic healing continuing at least 44 weeks after week 0.
11. The method of embodiment 7, wherein the subject is a responder to the treatment with the antibody and is identified as achieving a clinical response based on the Mayo endoscopy subscore continuing at least 44 weeks after week 0.
12. The method of embodiment 7, wherein the subject is a responder to the treatment with the antibody and is identified as having a change from baseline in Inflammatory Bowel Disease Questionnaire (IBDQ) score continuing at least 44 weeks after week 0.
13. The method of embodiment 7, wherein the subject is a responder to the treatment with the antibody and is identified as having a mucosal healing continuing at least 44 weeks after week 0.
14. The method of embodiment 7, wherein the subject is a responder to the treatment with the antibody and is identified as having a decrease from baseline in Mayo score continuing at least 44 weeks after week 0.
15. The method of embodiment 7, wherein the subject is a responder to the treatment with the antibody and is identified as having a normalization of one or more biomarkers selected from the group consisting of C-reactive protein, fecal lactoferrin and fecal calprotectin continuing at least 44 weeks after week 0.
16. The method of embodiment 7, wherein the subject is in clinical response as determined by a decrease from baseline in the Mayo score by ≥30% and ≥3 points and a decrease from baseline in the rectal bleeding subscore ≥1 points or a rectal bleeding subscore of 0 or 1 continuing at least 44 weeks after week 0.
17. A method of treating moderately to severely active ulcerative colitis (UC) in a subject in need thereof, comprising:
   a. intravenously administering to the subject an anti-IL-12/IL-23p40 antibody in a first pharmaceutical composition at a dosage of about 6.0 mg/kg body weight of the subject or 130 mg per administration at week 0 of the treatment, and
   b. subcutaneously administering to the subject the anti-IL-12/IL-23p40 antibody in a second pharmaceutical composition at a dosage of 90 mg per administration, preferably at week 8 of the treatment,
      wherein the antibody comprises a heavy chain variable region and a light chain variable region, the heavy chain variable region comprising: a complementarity determining region heavy chain 1 (CDRH1) amino acid sequence of SEQ ID NO: 1; a CDRH2 amino acid sequence of SEQ ID NO:2; and a CDRH3 amino acid sequence of SEQ ID NO:3; and the light chain variable region comprising: a complementarity determining region light chain 1 (CDRL1) amino acid sequence of SEQ ID NO:4; a CDRL2 amino acid sequence of SEQ ID NO:5; and a CDRL3 amino acid sequence of SEQ ID NO:6; and
      wherein the subject had previously failed or were intolerant of at least one therapy selected from the group consisting of: an anti-TNF, vedolizumab, corticosteroids, azathioprine (AZA), and 6 mercaptopurine (6 MP), or the subject had demonstrated corticosteroid dependence.
18. The method of embodiment 17, wherein the antibody comprises the heavy chain variable region of the amino acid sequence of SEQ ID NO:7 and the light chain variable region of the amino acid sequence of SEQ ID NO:8.
19. The method of embodiment 17, wherein the antibody comprises a heavy chain of the amino acid sequence of SEQ ID NO: 10 and a light chain of the amino acid sequence of SEQ ID NO: 11.
20. The method of any one of embodiments 1-19, wherein the pharmaceutical composition for intravenous administration further comprises a solution comprising 10 mM L-histidine, 8.5% (w/v) sucrose, 0.04% (w/v) polysorbate 80, 0.4 mg/mL L-methionine, and 20 µg/mL EDTA disodium salt, dehydrate, at pH 6.0.
21. The method of any one of embodiments 1-20, wherein the pharmaceutical composition for subcutaneous administration further comprises a solution comprising 6.7 mM L-histidine, 7.6% (w/v) sucrose, 0.004% (w/v) polysorbate 80, at pH 6.0.
22. The method of any one of embodiments 1-21, wherein the subject is a responder to the treatment with the antibody and is identified as having a clinical remission based on at least one of the global definition and the US definition by week 16, preferably by week 8, more preferably by week 2, of the treatment.
23. The method of any one of embodiments 1-22, wherein the subject is a responder to the treatment with the antibody and is identified as having an endoscopic healing by week 16, preferably by week 8, more preferably by week 2, of the treatment.
24. The method of any one of embodiments 1-23, wherein the subject is a responder to the treatment with the antibody and is identified as achieving a clinical response based on the Mayo endoscopy subscore by week 16, preferably by week 8, more preferably by week 2, of the treatment.
25. The method of any one of embodiments 1-24, wherein the subject is a responder to the treatment with the antibody and is identified as having a change from baseline in Inflammatory Bowel Disease Questionnaire (IBDQ) score by week 16, preferably by week 8, more preferably by week 2, of the treatment.
26. The method of any one of embodiments 1-25, wherein the subject is a responder to the treatment with the antibody and is identified as having a mucosal healing by week 16, preferably by week 8, more preferably by week 2, of the treatment.
27. The method of any one of embodiments 1-26, wherein the subject is a responder to the treatment with the antibody and is identified as having a decrease from baseline in Mayo score by week 16, preferably by week 8, more preferably by week 2, of the treatment.
28. The method of any one of embodiments 1-27, wherein the subject is a responder to the treatment with the antibody and is identified as having a normalization of one or more biomarkers selected from the group consisting of C-reactive protein, fecal lactoferrin and fecal calprotectin by week 16, preferably by week 8, more preferably by week 2, of the treatment.
29. The method of any one of embodiments 1-28, wherein the subject is in clinical response as determined by a decrease from baseline in the Mayo score by ≥30% and ≥3 points and a decrease from baseline in the rectal bleeding subscore ≥1 points or a rectal bleeding subscore of 0 or 1 by week 16, preferably by week 8, more preferably by week 2, of the treatment.
30. The method of any one of embodiments 17-21, wherein the subject is not a responder to the treatment with the antibody by week 8 and is a responder to the treatment by week 16 of the treatment.
31. A method of treating moderately to severely active ulcerative colitis (UC) in a subject in need thereof, comprising:
   a. intravenously administering to the subject an anti-IL-12/IL-23p40 antibody in a first pharmaceutical composition at a dosage of about 6.0 mg/kg body weight of the subject or 130 mg per administration at week 0 of the treatment, and
   b. subcutaneously administering to the subject the anti-IL-12/IL-23p40 antibody in a second pharmaceutical composition at a dosage of 90 mg per administration, preferably at week 8 of the treatment,
      wherein the antibody comprises a heavy chain variable region and a light chain variable region, the heavy chain variable region comprising: a complementarity determining region heavy chain 1 (CDRH1) amino acid sequence of SEQ ID NO: 1; a CDRH2 amino acid sequence of SEQ ID NO:2; and a CDRH3 amino acid sequence of SEQ ID NO:3; and the light chain variable region comprising: a complementarity determining region light chain 1 (CDRL1) amino acid sequence of SEQ ID NO:4; a CDRL2 amino acid sequence of SEQ ID NO:5; and a CDRL3 amino acid sequence of SEQ ID NO:6 followed by a maintenance therapy
      wherein the maintenance therapy comprises subcutaneously administering to the subject the anti-IL-12/IL-23p40 antibody at a dosage of 90 mg per administration, once every 8 weeks or once every 12 weeks, and wherein the maintenance therapy is provided for 44 weeks.
32. A pharmaceutical composition of an anti-IL-12/IL-23p40 antibody, comprising an antibody and packaging comprising one or more drug product label elements disclosed in Annex I including data from a randomized, double-blind, placebo-controlled, clinical study in adult men and women with moderately to severely active ulcerative colitis (UC), wherein the antibody comprises: (i) a heavy chain variable region and a light chain variable region, the heavy chain variable region comprising: a complementarity determining region heavy chain 1 (CDRH1) amino acid sequence of SEQ ID NO:1; a CDRH2 amino acid sequence of SEQ ID NO:2; and a CDRH3 amino acid sequence of SEQ ID NO:3; and the light chain variable region comprising: a complementarity determining region light chain 1 (CDRL1) amino acid sequence of SEQ ID NO:4; a CDRL2 amino acid sequence of SEQ ID NO:5; and a CDRL3 amino acid sequence of SEQ ID NO:6; (ii) a heavy chain variable region of the amino acid sequence of SEQ ID NO:7 and a light chain variable region of the amino acid sequence of SEQ ID NO:8; or (iii) a heavy chain of the amino acid sequence of SEQ ID NO:10 and a light chain of the amino acid sequence of SEQ ID NO:11.
33. A method of selling a drug product comprising ustekinumab, comprising: manufacturing ustekinumab; promoting that a therapy comprising ustekinumab is safe and effective for treatment of a subject with ulcerative colitis, wherein performing the steps a) and b) results in a health care professional (HCP) to purchase the drug product; thereby selling the drug product.

Having generally described the invention, the same will be more readily understood by reference to the following Examples, which are provided by way of illustration and are not intended as limiting. Further details of the invention are illustrated by the following non-limiting Examples. The disclosures of all citations in the specification are expressly incorporated herein by reference.

### EXAMPLES

### Example 1: Induction Study of ustekinumab in the treatment of ulcerative colitis in humans

The following multicenter, randomized, double-blind, placebo-controlled, clinical study in adult men and women with moderately to severely active ulcerative colitis (UC) was performed: A Phase 3, Randomized, Double-blind, Placebo-controlled, Parallel-group, Multicenter Study to Evaluate the Safety and Efficacy of ustekinumab Induction and Maintenance Therapy in Subjects with Moderately to Severely Active Ulcerative Colitis

### Overall rationale

A study was performed to assess the efficacy of intravenous (IV) administration of ustekinumab in subjects with moderately to severely active ulcerative colitis who demonstrated inadequate response or failure to tolerate conventional (corticosteroids or 6-mercaptopurine/azathioprine [6-MP/AZA]) or biologic therapy (TNF antagonist and/or the integrin antagonist, vedolizumab). Subjects received a single 130 mg, a single 6 mg/kg IV dose, or placebo at Week 0. Subjects who demonstrated no clinical response at Week 8 received an additional IV or subcutaneous (SC) dose at Week 8.

### Objectives

The primary objectives of the study included (1) evaluating the efficacy of ustekinumab in inducing clinical remission in subjects with moderately to severely active UC; and (2) evaluating the safety of the IV ustekinumab in subjects with moderately to severely active UC.

The secondary objectives of the study included (1) evaluating the efficacy of IV ustekinumab in inducing endoscopic healing (i.e. improvement in the endoscopic appearance of mucosa) in subjects with moderately to severely active UC; (2) evaluating the efficacy of IV ustekinumab in inducing clinical response in subjects with moderately to severely active UC; (3) evaluating the impact of IV ustekinumab on disease-specific health-related quality of life; (4) evaluating the efficacy of ustekinumab treatment on mucosal healing (i.e, endoscopic healing and histologic healing); (5) evaluating the efficacy of induction therapy with IV ustekinumab by biologic failure status; and (6) evaluating the pharmacokinetics (PK), immunogenicity, and pharmacodynamics (PD) of ustekinumab induction therapy in subjects with moderately to severely active UC, including changes in C-reactive protein (CRP), fecal calprotectin, fecal lactoferrin, and other PD biomarkers.

The exploratory objectives of the study included (1) evaluating response using the Mayo score without the physician's global assessment (PGA) subscore and (2) evaluating the performance of the Bristol Stool Form Scale (BSFS) score.

### Experimental Design

The Phase 3 development program for ustekinumab comprised 2 separate studies, an induction study and a maintenance study. In the induction study, subjects were randomized at Week 0 into one of three treatment groups: placebo, low-dose ustekinumab, and high-dose ustekinumab. At Week 8, all subjects were evaluated for the primary endpoint of clinical remission and clinical response. Subjects who achieved a clinical response at Week 8 were eligible to enter the maintenance study. Subjects who did not achieve clinical response at Week 8 received a second dose of ustekinumab at Week 8 of treatment.

At Week 16, subjects who did not achieve clinical response at Week 8 were re-evaluated for clinical response. Subjects who achieved clinical response at Week 16 were eligible to enter the maintenance study. Subjects who did not achieve clinical response at Week 16 were not eligible to enter the maintenance study and had a safety follow-up visit approximately 20 weeks after their last dose of study agent (Week 8).

Subjects who were in clinical response to IV ustekinumab during induction comprised the primary population in the maintenance study. The maintenance study is a randomized withdrawal study designed to evaluate maintenance therapy using SC ustekinumab and is currently ongoing.

### Dosage and administration

Subjects received a single IV dose of ustekinumab or placebo at Week 0 of the study. The induction study antibodies with the administered doses are as follows:
- Ustekinumab at a low, fixed does of 130 mg
- Ustekinumab at a high, weight-range based dose of ~6 mg/kg:
   ∘ Ustekinumab 260 mg (body-weight ≤55 kg)
   ∘ Ustekinumab 390 mg (body-weight >55 kg but ≤85 kg)
   ∘ Ustekinumab 520 mg (body-weight >85 kg)

Subjects who did not present a clinical response received a second dose of ustekinumab at Week 8. The study antibodies with the second administered doses are as follows:
- Subjects who were randomized to placebo at Week 0 received 1 dose of ustekinumab ~6 mg/kg IV + placebo SC (to maintain the blind) at Week 8.
- Subjects who were randomized to ustekinumab at Week 0 received 1 dose of ustekinumab 90 mg SC + placebo IV (to maintain the blind) at Week 8.

### Safety Evaluations

Safety was evaluated based on AEs and clinical laboratory test results (i.e., hematology and serum chemistry). Adverse events were either voluntarily reported by the subject or were obtained by means of interviewing subjects in a non-directed manner at study visits. Safety evaluations included the following clinical laboratory tests:
- Hematology: Hemoglobin (Hb), hematocrit, red blood cell count, white blood cell (WBC) count, and platelets.
- Serum Chemistry: Sodium, potassium, chloride, blood urea nitrogen (BUN), creatinine, aspartate aminotransferase (AST), alanine aminotransferase (ALT), total and direct bilirubin, alkaline phosphatase, calcium, phosphate, albumin, total protein.
- Screening: Serology for human immunodeficiency virus antibody, serology for hepatitis C virus (HCV) antibody, serology for hepatitis B virus (HBV) antibody, hepatitis B surface antigen, HBV surface antibody (anti-HBs), and HBV core (anti-HBc) antibody total, QuantiFERON-TB Gold test, pregnancy (β human chorionic gonadotropin [β-HCG]).

### Pharmacokinetics

Blood samples for the measurement of serum ustekinumab concentrations were collected at Week 0 (pre- and postinfusion) and Weeks 2, 4, and 8. Analyses of serum ustekinumab concentrations were performed using a validated electrochemiluminescent immunoassay (ECLIA) method on the Meso Scale Discovery (MSD^{®}) platform (Gaithersburg, MD, USA). The lowest quantifiable concentration in a sample for the ECLIA method using the MSD platform was 0.1688 µg/mL.

### Immunogenicity

Antibodies to ustekinumab were evaluated using serum samples collected from all subjects. Analyses of antibodies to ustekinumab were performed using a validated, drug-tolerant, electrochemiluminescence immunoassay (ECLIA), in which ustekinumab was used to capture and detect induced immune responses to ustekinumab. Antibody titers were determined for all subjects who had antibodies to ustekinumab and the neutralizing antibody (Nab) status of anti-drug antibody positive samples were determined.

### Efficacy Evaluation

Efficacy evaluations were collected throughout the study. Mayo score and partial Mayo score, Uceraltive Colitis Endoscopic Index of Severity (UCEIS), Bristol Stool Form Scale (BSFS), C-reactive protein (CRP), fecal lactoferrin, fecal calprotectin, Inflammatory Bowel Disease Questionnaire (IBDQ), 36-item Short Form Health Survey (SF-36), and EuroQoL-5D Health Questionnaire were all evaluated to determine efficacy. The efficacy criteria were defined as follows:
- Clinical remission (global submissions): Mayo score ≤2 points, with no individual subscore >1.
- Clinical remission (US submissions): absolute stool number ≤3, rectal bleeding subscore of 0, and Mayo endoscopy subscore of 0 or 1.
- Clinical response: a decrease from induction baseline in the Mayo score by ≥30% and ≥3 points, with either a decrease from baseline in the rectal bleeding subscore ≥1 or a rectal bleeding subscore of 0 or 1.
- Endoscopic healing (i.e., improvement in the endoscopic appearance of the mucosa): Mayo endoscopy subscore of 0 or 1.
- Histologic healing: based on the Geboes score and is defined as 0 to <5% neutrophils in epithelium and no crypt destruction, erosions, ulcerations, or granulations.
- Mucosal healing: both endoscopic healing and histologic healing.
- Normal or inactive mucosal disease: Mayo endoscopy subscore of 0.
- Symptomatic remission: Mayo stool frequency subscore of 0 or 1 and a rectal bleeding subscore of 0.
- Normalization of CRP concentration: CRP concentration ≤3 mg/L.
- Normalization of fecal lactoferrin concentration: fecal lactoferrin concentration ≤7.24 µg/g.
- Normalization of fecal calprotectin concentration: fecal calprotectin concentration ≤250 mg/kg.
- Modified Mayo score response:
   ∘ Definition 1: a decrease in the modified Mayo score of ≥2 points and ≥35% and either a decrease in the rectal bleeding subscore of ≥1 or a rectal bleeding subscore of 0 or 1.
   ∘ Definition 2: a decrease in the modified Mayo score of ≥2 points and ≥30% and either a decrease in rectal bleeding of ≥1 or a rectal bleeding score of 0 or 1.

### Safety Results

Intravenous ustekinumab doses of both ~6 mg/kg and 130 mg were generally well-tolerated with a safety profile that was generally comparable with placebo through Week 8.Of the 960 subjects in the safety analysis set, 1 or more treatment-emergent AEs was reported through Week 8 for 50.0%, 41.4%, and 48.0% of subjects in the ~6 mg/kg, 130 mg, and placebo groups, respectively. Through Week 8, serious adverse effects (SAEs) were reported for 3.1%, 3.7%, and 6.6% of subjects in the ~6 mg/kg, 130 mg, and placebo groups, respectively.

AEs within 1 hour of infusion were 0.9%, 2.2%, and 1.9% in the ~6 mg/kg, 130 mg, and placebo groups, respectively.

The proportions of subjects with 1 or more infections were 15.3%, 15.9%, and 15.0% in the ~6 mg/kg, 130 mg, and placebo groups, respectively. Serious infections were reported for 0.3%, 0.6%, and 1.3% of subjects in the ~6 mg/kg, 130 mg, and placebo groups, respectively.

### Pharmacokinetics Results

Serum samples were collected at Week 0 (preadministration), Week 0 (1 hr post-administration, Week 2, Week 4, and Week 8. For subjects randomized to ustekinumab treatment, a single IV infusion of ustekinumab was given either as a weight-based tiered dose of ~6 mg/kg (ie, 260 mg for subjects with body-weight ≤55 kg, 390 mg for subjects with body-weight >55 kg and ≤85 kg, or 520 mg for subjects with body-weight >85 kg), or as a fixed dose of 130 mg. Considering that the median body-weight of subjects in the 130 mg group was 72 kg, the ustekinumab 130 mg dose corresponded to ~2 mg/kg on a per-kg basis. Thus, on average, ustekinumab exposure in the ~6 mg/kg group was approximately 3 times that of the 130 mg group. In line with this expectation, after a single IV administration of ustekinumab ~6 mg/kg or 130 mg, median serum ustekinumab concentrations were approximately dose proportional at all sampling timepoints through Week 8. Median peak serum ustekinumab concentrations, which were observed 1 hour after the end of the infusion at Week 0, were 127.0 µg/mL and 43.16 µg/mL for the ~6 mg/kg and 130 mg groups, respectively. At Week 8, the time of the primary efficacy endpoint, the median serum ustekinumab concentrations were 8.59 µg/mL and 2.51 µg/mL for the ~6 mg/kg and 130 mg groups, respectively.

Subjects who were not in clinical response at Week 8 following administration of placebo IV at Week 0 received ustekinumab ~6 mg/kg IV at Week 8, while subjects who were not in clinical response at Week 8 following administration of ustekinumab IV at Week 0 received ustekinumab 90 mg SC at Week 8. Among subjects who received placebo IV at Week 0 and who subsequently received ustekinumab ~6 mg/kg IV at Week 8, median serum ustekinumab concentration at Week 16 (8 weeks after the ustekinumab IV dose) was slightly higher than that observed at Week 8 (among subjects who received ustekinumab ~6 mg/kg IV at Week 0 [10.51 µg/mL versus 8.59 µg/mL, respectively]). Among subjects who received ustekinumab 90 mg SC at Week 8 (following their initial IV ustekinumab dose at Week 0), the median serum ustekinumab concentration at Week 16 was slightly higher in subjects who received ustekinumab ~6 mg/kg IV at Week 0 compared to those who received ustekinumab 130 mg at Week 0 (1.92 µg/mL versus 1.59 µg/mL, respectively)

### Immunogenicity Results

Of the 635 subjects in the ustekinumab groups with appropriate samples for the assessment of antibodies to ustekinumab, 4 (0.6%) subjects were positive for antibodies to ustekinumab through Week 8. Of these 4 subjects, 2 (50%) were positive for NAbs.

Of 822 subjects who received ustekinumab at any time through Week 16, and had appropriate samples for the assessment of anti-drug antibodies (ADAs), 18 subjects (2.2%) were positive for antibodies to ustekinumab through the final safety visit. Of these, 4 of 15 subjects (26.7%) were positive for NAbs among those evaluable for NAbs through the final safety visit. Among subjects who received ustekinumab 90 mg SC at Week 8, the incidence of antibodies to ustekinumab through Week 16 was numerically higher in the 130 mg IV→90 mg SC group compared to the ~6 mg/kg IV->90 mg SC group (4.5% [6 of 132 subjects] vs 1.0% [1 of 101 subjects]).

### Efficacy Results

### Clinical Remission at Week 8- Global Definition

At Week 8, significantly greater proportions of subjects in the ~6 mg/kg and 130 mg groups achieved clinical remission (15.5% and 15.6%, respectively) compared with subjects in the placebo group (5.3%; p<0.001 for both comparisons; Table 1).

**Table 1. Number of Subjects in Clinical Remission (Global Definition) at Week 8**

| | | Ustekinumab IV | | |
|---|---|---|---|---|
| | Placebo IV | 130 mg | 6 mg/kg | Combined |
| Primary Efficacy Analysis Set | 319 | 320 | 322 | 642 |
| Week 8 (N) | 319 | 320 | 322 | 642 |
| Subjects in clinical remission | 17 (5.3%) | 50 (15.6%) | 50 (15.5%) | 100 (15.6%) |
| Adjusted Treatment difference | | 10.3 | 10.2 | 10.2 |
| (97.5% CI) | | (5.7, 14.9) | (5.6, 14.8) | (6.6, 13.9) |
| p-value | | <0.001 | <0.001 | <0.001 |
| N= number of subjects; CI= confidence interval | | | | |

### Clinical Remission at Week 8- US Definition

At Week 8, significantly greater proportions of subjects in the ~6 mg/kg and 130 mg groups achieved clinical remission (18.9% and 16.6%, respectively) compared with subjects in the placebo group (6.3%; p<0.001 for both comparisons; Table 2).

**Table 2. Number of Subjects in Clinical Remission (US Definition) at Week 8**

| | | Ustekinumab IV | | |
|---|---|---|---|---|
| | Placebo IV | 130 mg | 6 mg/kg | Combined |
| Primary Efficacy Analysis Set | 319 | 320 | 322 | 642 |
| Week 8 (N) | 319 | 320 | 322 | 642 |
| Subjects in clinical remission | 20 (6.3%) | 53 (16.6%) | 61 (18.9%) | 114 (17.8%) |
| Adjusted Treatment difference | | 10.3 | 12.7 | 11.5 |
| (97.5% CI) | | (4.8, 15.8) | (7.0, 18.4) | (7.0, 16) |
| p-value | | <0.001 | <0.001 | <0.001 |
| N= number of subjects; CI= confidence interval | | | | |

### Endoscopic Healing at Week 8

At Week 8, significantly greater proportions of subjects in the ~6 mg/kg and 130 mg groups achieved endoscopic healing (27.0% and 26.3%, respectively) compared with subjects in the placebo group (13.8%; p<0.001 for both comparisons; Table 3).

**Table 3. Number of Subjects with Endoscopic Healing at Week 8**

| | | Ustekinumab IV | | |
|---|---|---|---|---|
| | Placebo IV | 130 mg | 6 mg/kg | Combined |
| Primary Efficacy Analysis Set | 319 | 320 | 322 | 642 |
| Week 8 (N) | 319 | 320 | 322 | 642 |
| Subjects with endoscopic healing | 44 (13.8%) | 84 (26.3%) | 87 (27.0%) | 171 (26.6%) |
| Adjusted Treatment difference | | 12.4 | 13.3 | 12.8 |
| (95% CI) | | (6.5, 18.4) | (7.3, 19.3) | (7.9, 17.8) |
| (97.5% CI) | | (5.2, 19.2) | (6.4, 20.1) | (7.2, 18.5) |
| p-value | | <0.001 | <0.001 | <0.001 |
| N= number of subjects; CI= confidence interval | | | | |

### Clinical Response at Week 8

At Week 8, significantly greater proportions of subjects in the ~6 mg/kg and 130 mg groups achieved clinical response (61.8% and 51.3%, respectively) compared with subjects in the placebo group (31.3%; p<0.001 for both comparisons; Table 4).

**Table 4. Number of Subjects in Clinical Response**

| | | Ustekinumab IV | | |
|---|---|---|---|---|
| | Placebo IV | 130 mg | 6 mg/kg | Combined |
| Primary Efficacy Analysis Set | 319 | 320 | 322 | 642 |
| Week 8 (N) | 319 | 320 | 322 | 642 |
| Subjects in clinical response | 100 (31.3%) | 164 (51.3%) | 199 (61.8%) | 363 (56.5%) |
| Adjusted Treatment difference | | 19.9 | 30.5 | 25.2 |
| (95% CI) | | (12.8, 27.3) | (23.2, 37.8) | (18.9, 31.5) |
| (97.5% CI) | | (11.4, 28.3) | (22.2, 38.8) | (18.0, 32.4) |
| p-value | | <0.001 | <0.001 | <0.001 |
| N= number of subjects; CI= confidence interval | | | | |

### Change in Baseline in Total IBDQ Score at Week 8

At baseline, median IBDQ scores were similar across all treatment groups. At Week 8, the median improvements from baseline in the IBDQ scores were significantly greater in the ~6 mg/kg and 130 mg groups (31.0 and 31.5, respectively) compared with the placebo group (10.0; p<0.001 for both comparisons).

### Clinical Remission at Week 8

When remission was assessed as clinical remission (global definition) with a rectal bleeding subscore of 0 at Week 8, the proportions of subjects who achieved this endpoint were almost identical to that observed based on the primary efficacy analysis (global definition). Significantly greater proportions of subjects in the ~6 mg/kg and 130 mg groups achieved this endpoint (15.2% and 15.3%, respectively) compared with subjects in the placebo group (5.3%; p<0.001 for both comparisons).

### Symptomatic Remission at Week 8

At Week 8, significantly greater proportions of subjects in the ~6 mg/kg and 130 mg groups achieved symptomatic remission (44.7% and 41.3%, respectively) compared with subjects in the placebo group (22.6%; p<0.001 for both comparisons).

### Histologic Healing at Week 8

Histologic healing was defined as 0 to <5% neutrophils in epithelium and no crypt destruction, erosions, ulcerations, or granulations. At Week 8, significantly greater proportions of subjects in the ~6 mg/kg and 130 mg groups achieved histologic healing (35.6% and 37.9%, respectively) compared with subjects in the placebo group (21.9%; p<0.001 for both comparisons).

### Change from Baseline in Mayo Score at Week 8

At baseline, the mean Mayo scores were the same across all treatment groups (8.9 for all groups). At Week 8, the mean decreases from baseline in Mayo scores were significantly greater in the ~6 mg/kg and 130 mg groups (3.5 and 3.2, respectively) compared with the placebo group (1.8; p<0.001 for both comparisons).

### Change from Baseline in partial Mayo Score Through Week 8

At baseline, the mean partial Mayo scores were the same across all treatment groups (6.2 for all groups). As early as Week 2 and continuing for visits through Week 8, the mean decreases in the partial Mayo score were significantly greater in the ~6 mg/kg and 130 mg groups compared with the placebo group. At Week 2, the mean decreases from baseline in the partial Mayo scores were 1.6 and 1.5, in the ~6 mg/kg and 130 mg, respectively, compared with 1.0 in the placebo group (p<0.001 for both comparisons). At Week 8, the mean decreases from baseline in the partial Mayo scores were 2.9 and 2.6, in the ~6 mg/kg and 130 mg, respectively, compared with 1.5 in the placebo group (p<0.001 for both comparisons).

### UCEIS Score at Week 8

The UCEIS score provides an overall assessment of endoscopic severity of UC, based on mucosal vascular pattern, bleeding, and ulceration. The score ranges from 3 to 11 with a higher score indicating more severe disease by endoscopy. The UCEIS score was assessed only during the central read of the video of the endoscopy.

At baseline, the mean UCEIS scores were similar across all treatment groups (7.6, 7.5, 7.5 in the ~6 mg/kg, 130 mg and placebo groups, respectively). At Week 8, the mean decreases from baseline in UCEIS scores were significantly greater in the ~6 mg/kg and 130 mg groups (1.3 and 1.1, respectively) compared with the placebo group (0.5; p<0.001 for both comparisons).

At Week 8, significantly greater proportions of subjects in the ~6 mg/kg and 130 mg groups had a UCEIS score of ≤4 (20.2% and 19.1%, respectively) compared with subjects in the placebo group (11.0%; p<0.001 and p=0.004, respectively). It is hypothesized that a UCEIS score of ≤4 is associated with Mayo endoscopic subscores of 0 or 1 that have defined endoscopic healing in this study.

### Bristol Stool Form Scale Score

The BSFS score at a visit was the average of the 3-day daily average of the BSFS score prior to the visit. The same 3 days used to calculate the stool frequency and rectal bleeding subscores of the Mayo score were used to calculate the average BSFS score for the visit.

Approximately 40% (370/961) of randomized subjects had BSFS score collected at baseline. At baseline, 99.2% (367/370) of the subjects had average BSFS scores of ≥3 and the majority of subjects (54.3%) had average BSFS scores of ≥6, indicating diarrhea. As early as Week 2 and continuing for visits through Week 8, the proportions of subjects with diarrhea (average BSFS scores of ≥6) were smaller in the ~6 mg/kg and 130 mg groups compared with the placebo group. At Week 8, 22.8%, 21.1%, and 32.0% of subjects had diarrhea (average BSFS scores of ≥6) in the ~6 mg/kg, 130 mg and placebo groups, respectively. Furthermore, at Week 8 the proportion of subjects with normal stool (≥3 and <5) was greater in the ~ 6mg/kg and 130 mg groups compared with placebo (48.3%, 48.9%, and 29.3%, respectively).

### Normalization of C-reactive Protein

C-reactive protein (CRP) is used as a marker of inflammation in subjects with IBD. In UC, elevated CRP has been associated with severe clinical activity, an elevated sedimentation rate, and active disease as detected by colonoscopy. C-reactive protein was assayed using a validated, high-sensitivity CRP assay.

At baseline, the proportion of subjects who had abnormal CRP (>3 mg/L) was similar across all treatment groups; overall, 59.2% of randomized subjects had abnormal CRP concentrations at baseline. As early as Week 2 and continuing for visits through Week 8, among subjects who had abnormal values at baseline, significantly greater proportions of subjects in the ~6 mg/kg and 130 mg groups achieved normalization of CRP (≤3 mg/L) compared with the placebo group. At Week 8, 38.7% and 34.1% of subjects achieved normalization of CRP in the ~6 mg/kg and 130 mg groups, respectively, compared with 21.1% of subjects in the placebo group (p<0.001 for both comparisons).

### Normalization of Fecal Lactoferrin

At baseline, the proportions of subjects with abnormal fecal lactoferrin (>7.24 µg/g) were similar across all treatment groups; overall 90.0% of randomized subjects had abnormal fecal lactoferrin concentrations at baseline. At Week 4 and Week 8, among subjects who had abnormal values at baseline, significantly greater proportions of subjects in the ~6 mg/kg and 130 mg groups achieved normalization of fecal lactoferrin (≤7.24 µg/g) compared with the placebo group. At Week 8, 14.6% and 17.2% of subjects in the ~6 mg/kg and 130 mg groups, respectively, achieved normalization of fecal lactoferrin compared with 9.3% of subjects in the placebo group (p=0.042, p=0.006, respectively, for the ustekinumab groups).

### Normalization of Fecal Calprotectin

At baseline, the proportions of subjects with abnormal fecal calprotectin (>250 mg/kg) were slightly greater in the ~6 mg/kg group (85.1%) compared with the placebo group (78.4%); 82.5% of subjects in the 130 mg group had abnormal fecal calprotectin at baseline. At Week 2 and Week 4, among subjects who had abnormal values at baseline, significantly greater proportions of subjects in the ~6 mg/kg and 130 mg groups achieved normalization of fecal calprotectin (≤250 mg/kg). At Week 8, among subjects with abnormal fecal calprotectin at baseline, the proportions of subjects with normalized fecal calprotectin, though not significant, were numerically greater in the ustekinumab ~6 mg/kg and 130 mg groups (25.5% and 24.2%, respectively), compared with subjects in the placebo group (20.4%; p=0.148, p=0.301 for both comparisons, respectively).

### Example 2: Maintenance Study of ustekinumab in the treatment of ulcerative colitis in humans

### Methodology

In this randomized-withdrawal maintenance study, all subjects enrolled were to be responders to study agent administered in the induction study. Primary (randomized) population: Subjects who were in clinical response to IV ustekinumab following induction comprised the primary population in the maintenance study. This population included the following: subjects who were randomized to receive ustekinumab (ie, 130 mg IV or ~6 mg/kg IV) at Week 0 of the induction study and were in clinical response at induction Week 8; and subjects who were randomized to receive placebo at Week 0 of the induction study and were not in clinical response at induction Week 8 but were in clinical response at induction Week 16 after receiving a dose of IV ustekinumab (~6 mg/kg) at induction Week 8 (placebo -> ustekinumab ~6 mg/kg IV). These subjects were randomized in a 1:1:1 ratio at maintenance Week 0 to receive ustekinumab 90 mg SC every 8 weeks (q8w), ustekinumab 90 mg SC every 12 weeks (q12w), or placebo SC. Nonrandomized population: Additional subjects entering the maintenance study were not randomized in the primary population and received maintenance treatment in this study as follows: ustekinumab induction delayed responders (ie, subjects who were not in clinical response to IV ustekinumab at induction Week 8 but were in clinical response at induction Week 16 after receiving ustekinumab 90 mg SC at induction Week 8) received ustekinumab 90 mg SC q8w; and placebo induction responders (ie, subjects who were in clinical response to placebo IV induction) received placebo SC. Nonrandomized subjects were followed for both efficacy and safety but were not included in the key efficacy analyses.

All subjects received their assigned dose of SC study agent at the maintenance Week 0 visit. Thereafter, to maintain the blind, all subjects received study agent at all scheduled study agent administration visits. Subjects were assessed for clinical flare at every visit and, if loss of clinical response was confirmed, were eligible for rescue medication. The main portion of the maintenance study was through Week 44 and a long-term study extension will continue through Week 220.

### Number of Subjects (planned and analyzed):

783 subjects who completed the induction study and were in clinical response to induction study agent were enrolled in this maintenance study. The numbers of subjects in each treatment group at maintenance Week 0 were as follows:
- Randomized (primary) population (523 subjects [327 subjects were planned]):
   - 176 subjects were randomized to ustekinumab 90 mg SC q8w.
   - 172 subjects were randomized to ustekinumab 90 mg SC q12w.
   - 175 subjects were randomized to placebo SC.
- Nonrandomized population (260 subjects):
   - 157 subjects who were ustekinumab induction delayed responders (ie, were not in clinical response to ustekinumab at induction Week 8 but were in clinical response at induction Week 16) received ustekinumab 90 mg SC q8w.
   - 103 subjects who were in clinical response to placebo IV induction (placebo induction responders) received placebo SC.

### Diagnosis and Main Criteria for Inclusion:

All subjects enrolled into this randomized-withdrawal maintenance study were those with moderately to severely active UC who had an inadequate response or had failed to tolerate conventional therapy (ie, corticosteroids or immunomodulators) or biologic therapy (ie, a TNF antagonist and/or vedolizumab), and demonstrated a clinical response to study agent during the induction study. This included subjects who were in clinical response to IV ustekinumab, in clinical response to IV placebo, or in delayed clinical response to ustekinumab, and had not received a protocol-prohibited medication change during the induction study.

### Criteria for Evaluation:

- Pharmacokinetics (PK): Serum ustekinumab concentration
- Immunogenicity: Antibodies to ustekinumab
- Pharmacodynamics (PD)/biomarkers: Serum biomarkers; fecal microbiome; RNA expression and histologic assessment of disease activity and healing in mucosal biopsies
- Genetics and epigenetics: Whole blood deoxyribonucleic acid (DNA)
- Efficacy: Mayo score and partial Mayo score, UC Endoscopic Index of Severity (UCEIS), CRP, fecal lactoferrin, and fecal calprotectin
- Health-related Quality of Life: Inflammatory Bowel Disease Questionnaire (IBDQ), 36-item Short Form Health Survey (SF-36), EuroQoL-5D Health Questionnaire (EQ-5D)
- Health economics: UC disease-related hospitalizations and surgeries; productivity Visual Analog Scale (VAS), and Work Productivity and Activity Impairment Questionnaire-General Health (WPAI-GH)
- Safety: Adverse events (AEs), serious adverse events (SAEs), infections, injection site reactions, allergic reactions, hematology and chemistry parameters, vital signs, physical examinations, and early detection of tuberculosis *ENDPOINTS*
- The **primary endpoint** was clinical remission at Week 44. The definition of clinical remission (as well as the testing procedure) is different for submissions in the US and outside the US to accommodate the global and US preferred definitions of clinical remission. Each definition of clinical remission was applied to all subjects in the primary efficacy analysis set.
   - The global definition of the primary endpoint of clinical remission was defined as a Mayo score ≤2 points, with no individual subscore >1.
   - The US definition of clinical remission was defined as an absolute stool number ≤3, a Mayo rectal bleeding subscore of 0, and a Mayo endoscopy subscore of 0 or 1.
- The **major secondary endpoints**, listed in the order in which they were tested, were:
   - Maintenance of clinical response through Week 44
   - Endoscopic healing at Week 44
   - Clinical remission and not receiving concomitant corticosteroids (corticosteroid-free clinical remission) at Week 44
   - Maintenance of clinical remission through Week 44 among the subjects who had achieved clinical remission at maintenance baseline

For the 3rd and 4th major secondary endpoints, the global definition of clinical remission was used to support submissions for countries outside the US and the US definition of clinical remission was used to support the submission in the United States.

Demographic and baseline disease characteristics were summarized based on the 961 subjects in the primary efficacy analysis set.

Analyses of multiplicity-controlled endpoints, except for the fourth major secondary endpoint related to maintenance of clinical remission, were conducted using a Cochran-Mantel-Haenszel (CMH) chi square test stratified by clinical remission (global definition) status at maintenance baseline (yes/no as determined by the IWRS) and induction treatment (placebo IV [I-0] → ustekinumab ~6 mg/kg IV [I-8], ustekinumab 130 mg IV [I-0], or ustekinumab ~6 mg/kg IV [I-0]). For the fourth major secondary endpoint (maintenance of clinical remission), a CMH chi-square test stratified by induction treatment was used.

Global and US-specific multiple testing procedures were prespecified to control the overall Type 1 error rate at the 0.05 level over the multiplicity-controlled endpoints in this study (Section 3.11.2.7.3). All statistical testing was performed at the 2-sided 0.05 significance level. Nominal p-values are presented.

Safety was assessed by summarizing the frequency and type of treatment-emergent adverse events (AEs), laboratory parameters (hematology and chemistry), and vital signs parameters. Safety summaries are provided separately for randomized subjects, nonrandomized subjects, and all treated subjects. Presentation of the safety data focuses on the randomized population.

### RESULTS:

### STUDY POPULATION

A total of 783 subjects who completed the induction study and were in clinical response to induction study agent were enrolled in this maintenance study. Of these, 523 subjects were in the targeted primary population for the maintenance study and were randomized to receive a SC administration of ustekinumab or placebo at maintenance Week 0 (176, 172, and 175 subjects in the ustekinumab 90 mg SC q8w, ustekinumab 90 mg SC q12w, and placebo groups, respectively). The remaining 250 subjects were in the nonrandomized population, including 157 ustekinumab induction delayed responders (who received ustekinumab 90 mg SC q8w) and 103 placebo induction responders (who received placebo). All enrolled subjects who were assigned treatment at maintenance baseline received their study agent at that time.

Prior to Week 40 (last dosing visit of the maintenance study), 85 subjects (16.3%) in the primary population discontinued study agent. The proportion of subjects who discontinued study agent was greater in the placebo group (24.6%) than those in the ustekinumab q8w and q12w groups (10.2% and 14.0%, respectively). The most common reasons for discontinuation were lack of efficacy and an adverse event due to worsening of UC. Prior to Week 44, 29 subjects (5.5%) in the primary population terminated study participation; the most common reason for termination of study participation was withdrawal of consent.

Baseline clinical disease characteristics were representative of a population of subjects with moderately to severely active UC that was refractory to available therapies and were generally well-balanced across the 3 treatment groups. The median duration of disease was 6.05 years and the median baseline Mayo score was 9.0, with 86.9% and 13.1% presenting with moderate and severe UC, respectively. At induction baseline, 52.2% of subjects in the primary population of the maintenance study were taking corticosteroids, 26.6% were taking immunomodulatory drugs, and 70.7% were taking aminosalicylates. The majority of subjects (93.5%) had an inadequate response to, or were intolerant of, corticosteroids and/or 6-MP/AZA, or demonstrated corticosteroid dependence at induction baseline. Overall in the primary population, 47.6% of subjects had a history of documented biologic failure and 52.4% of subjects did not. Also, 47.2% had failed at least 1 anti-TNF whereas 13.4% had failed both an anti-TNF and vedolizumab, and 49.3% were naive to biologic therapy; 2 subjects were biologic failures to only vedolizumab.

### EFFICACY RESULTS

Ustekinumab maintenance therapy demonstrated efficacy in a population of subjects with moderately to severely active UC who had previously failed or were intolerant of conventional or biologic therapies, including TNF antagonists and/or vedolizumab, and were in clinical response 8 weeks after receiving a single dose of ustekinumab IV induction therapy.

Based on the pre-specified global and US-specific multiple testing procedures, statistical significance can be claimed for both ustekinumab dose regimens (90 mg q8w and 90 mg q12w) for the primary endpoint of clinical remission at Week 44 and the three major secondary endpoints of maintenance of clinical response through Week 44, endoscopic healing at Week 44, and corticosteroid-free clinical remission at Week 44. Additionally, statistical significance can be claimed for maintenance of clinical remission through Week 44 (among the subjects who had achieved clinical remission at maintenance baseline) for both ustekinumab doses based on the US-specific testing procedure, and for the ustekinumab q12w regimen based on the global testing procedure.
- Clinical Efficacy in the Primary Population (ie, Subjects in Clinical Response 8 Weeks After Receiving Ustekinumab IV Induction Therapy)
   - Primary Endpoint: Clinical Remission
      o The proportions of subjects in clinical remission (based on the global definition) at Week 44 were significantly greater in the ustekinumab q8w group and ustekinumab q12w group (43.8% and 38.4%, respectively) compared with subjects in the placebo group (24.0%; p<0.001 and p=0.002, respectively).
      ∘ The proportions of subjects in clinical remission (based on the US-specific definition) at Week 44 were significantly greater in the ustekinumab q8w group and ustekinumab q12w group (42.6% and 39.5%, respectively) compared with subjects in the placebo group (24.6%; p<0.001 and p=0.002, respectively).
      ∘ The effect of ustekinumab on achieving clinical remission (based on both the global and US specific definitions) was generally consistent across subgroups (including subjects who were biologic failures and those who were not biologic failures as well as subjects who were receiving concomitant immunomodulators or corticosteroids at induction baseline and those who were not) and was robust to prespecified changes in data-handling rules.
   - Major Secondary Endpoints: Maintenance of Clinical Response, Endoscopic Healing, Corticosteroid-Free Clinical Remission, and Maintenance of Clinical Remission
      ∘ The proportions of subjects who maintained clinical response through Week 44, achieved endoscopic healing, achieved corticosteroid-free remission (applying both global and US specific definitions of clinical remission) were significantly greater (p<0.01) in the ustekinumab q8w and q12w groups compared with that in the placebo group.
      ∘ The proportions of subjects who maintained clinical remission among the subjects who had achieved clinical remission at maintenance baseline was numerically greater for both the ustekinumab q8w and q12w groups compared with that in the placebo group (applying both the global and US specific definition of clinical remission). Statistical significance (p<0.01) was achieved for both comparisons of the q8w and q12w groups versus placebo using the US-specific definition of clinical remission; however, statistical significance was only achieved for the q12w group (p<0.01) compared to placebo using the global definition of clinical remission.
   - Other Histologic, Mucosal, Clinical, and Endoscopic Endpoints
      The analyses summarized below were not adjusted for multiplicity. Statements of statistical significance are based on nominal p-values.
      ∘ The proportions of subjects who achieved histologic healing (ie, neutrophil infiltration in <5% of crypts, no crypt destruction, and no erosions, ulcerations, or granulation tissue) at Week 44 were significantly (p<0.001) greater in the ustekinumab q8w and q12w groups compared with the placebo group.
      ∘ The proportions of subjects who achieved mucosal healing (a combination of endoscopic healing and histologic healing) at Week 44 were significantly (p<0.01) greater in the ustekinumab q8w and q12w groups compared with the placebo group.
      ∘ Applying both global and US-specific definitions of clinical remission, the proportions of subjects achieving corticosteroid-free remission for at least 90 days prior to Week 44 was significantly greater (p<0.01) in the ustekinumab q8w and q12w groups compared with that in the placebo group. Furthermore, among subjects receiving corticosteroids at maintenance baseline, significantly greater proportions of subjects (p<0.05) were in clinical remission and not receiving concomitant corticosteroids for at least 90 days prior to Week 44 in the ustekinumab q8w and q 12w groups compared with those in the placebo group.
      ∘ The efficacy of ustekinumab maintenance treatment was also demonstrated in clinical outcomes as measured by maintained improvement in the partial Mayo score, maintenance of symptomatic remission as well as maintenance of endoscopic healing. Further evidence of the efficacy of ustekinumab maintenance treatment was observed in partial Mayo remission and symptomatic remission over time as well as symptom control (stool frequency and rectal bleeding).
   - Inflammatory Biomarkers
      ∘ Over time through Week 44, the ustekinumab treatment groups maintained their CRP, fecal lactoferrin, and fecal calprotectin concentration levels observed at maintenance baseline, whereas median CRP, fecal lactoferrin, and fecal calprotectin concentrations worsened (increased) in the placebo group.
      ∘ At Week 44, the proportion of subjects with normalized CRP, fecal calprotectin and fecal lactoferrin were generally significantly greater in the ustekinumab q8w and q 12w groups compared with the placebo group.
   - Clinical Endpoints by Biologic Failure Status
      ∘ For subjects with and subjects without a history of biologic failure, the proportions of subjects who achieved each of the primary and major secondary endpoints and mucosal healing were generally greater in the ustekinumab q8w and q12w groups compared with subjects in the placebo group.
      ∘ In some cases, where treatment effects were similar in the biologic non-failure and failure populations, there was a consistent trend in the biologic-failure subjects across endpoints that the treatment effect for the ustekinumab q8w group was greater than that for the ustekinumab q12w group. This trend was not observed in the biologic non-failure population.
   - Efficacy Based on Inflammatory Biomarker Subgroups
      ∘ Among subjects with a higher inflammatory burden (elevated CRP and/or elevated fecal inflammatory markers) at either induction or maintenance baseline, while both dosages generally demonstrated efficacy compared to placebo, the efficacy of ustekinumab q8w seemed to be better across the range of clinical endpoints than the ustekinumab q12w group. However, in subjects with low inflammatory burden at baseline, the ustekinumab q8w and q12w groups demonstrated similar efficacy over the endpoints
   - Health-Related Quality of Life
      ∘ Through Week 44, subjects in the ustekinumab q8w and q12w groups were generally able to maintain improvement in health-related quality of life as assessed using the IBDQ, SF 36 and EQ 5D instruments compared to subjects in the placebo group.
      - Outcomes for the Ustekinumab 90 mg q8w Dose and Ustekinumab 90 mg q12w Dose
         ∘ While both the ustekinumab q8w and q12w groups demonstrated generally similar efficacy for the primary and major secondary endpoints, q8w was modestly better than q12w based on the following more objective and stringent measures of efficacy, including:
            ◆ Endoscopic and mucosal healing at Week 44
            ◆ Durable partial Mayo remission at Week 44
            ◆ Corticosteroid-free clinical remission as well as the elimination of corticosteroids for at least 90 days prior to Week 44 among subjects receiving corticosteroids at maintenance baseline
         ∘ Furthermore, when efficacy was examined over time (for the following endpoints), the q8w group showed greater efficacy than the q12w group:
            ◆ Mayo stool frequency and rectal bleeding subscores indicating inactive or mild disease (ie, subscores of 0 or 1), as well as an absolute stool number ≤3 over time through Week 44.
            ◆ Partial Mayo remission and symptomatic remission over time through Week 44
            ◆ Median changes from baseline in fecal lactoferrin and calprotectin concentrations over time through Week 44.
- Efficacy in Ustekinumab Induction Delayed Responders
   Subjects who were delayed responders to ustekinumab induction therapy were able to maintain clinical response and achieve clinical remission, endoscopic, histologic, and mucosal healing (a combination of endoscopic healing and histologic healing) while receiving ustekinumab 90 mg q8w.
- Efficacy and Pharmacokinetics/Immunogenicity
   - In general, during maintenance, a positive association was observed between serum ustekinumab concentration and the clinical efficacy outcomes of clinical remission and endoscopic healing. In addition, lower levels of inflammation, as measured by CRP, were observed in subjects with higher serum ustekinumab concentrations.
   - Among subjects receiving maintenance ustekinumab, the development of antibodies to ustekinumab did not appear to have an impact on clinical efficacy as measured by multiple endpoints such as clinical remission, endoscopic healing, clinical response, and change from maintenance baseline in Mayo score; however, the interpretation of the data is limited by the small sample size.

### PHARMACOKINETIC AND IMMUNOGENICITY RESULTS

- Following maintenance treatment with ustekinumab 90 mg SC q8w or q 12w, steady-state was reached at approximately 8 or 12 weeks after subjects began receiving ustekinumab 90 mg SC q8w, or ustekinumab 90 mg SC q12w maintenance dose regimens, respectively. Median steady state trough serum ustekinumab concentrations over time were approximately 3-fold greater the concentrations in the ustekinumab q8w group (2.69 µg/mL to 3.09 µg/mL) than in the q12w group (0.92 µg/mL to 1.19 µg/mL).
- Following maintenance dose regimens of ustekinumab 90 mg SC q8w or q12w, serum ustekinumab concentrations were sustained through Week 44 in almost all subjects, with a smaller proportion of subjects with undetectable trough concentrations over time in the 90 mg q8w group (0.7% to 2.4%) compared to those in the 90 mg q12w group (4.9% to 7.1%). The median ustekinumab concentration in subjects in the placebo group was below detectable levels by Week 16.
- The impact of the different ustekinumab IV induction doses on serum ustekinumab concentrations during maintenance continued to diminish over time, as expected.
- Median trough serum ustekinumab concentrations tended to be lower in subjects with higher body weight.
- Nonrandomized subjects in the ustekinumab induction delayed responders group tended to have lower serum ustekinumab concentrations over time compared to randomized subjects in the ustekinumab q8w group following SC administration of the same ustekinumab dose regimen of 90 mg q8w.
- Among 680 treated subjects with appropriate samples for the assessment of antibodies to ustekinumab, 39 (5.7%) were positive for antibodies to ustekinumab through 52 weeks of treatment, the majority with antibody titers ≤1:800. Of the 39 treated subjects who were positive for antibodies to ustekinumab in this maintenance study, 11 (28.2%) were positive for neutralizing antibodies.
- In all randomized treatment groups, median serum ustekinumab concentrations were lower over time in subjects who were positive for antibodies to ustekinumab compared with levels in subjects who were negative for antibodies to ustekinumab.

### SAFETY RESULTS

Subcutaneous maintenance regimens of ustekinumab 90 mg administered q12w or q8w through Week 44 were generally well tolerated and consistent with the known safety profile of ustekinumab.
- AEs were reported in 77.3%, 69.2%, and 78.9% of subjects in the ustekinumab q8w, ustekinumab q12w, and placebo groups, respectively.
   - Reasonably related AEs were reported in 26.1%, 17.4%, and 28.6% of subjects in the ustekinumab q8w, ustekinumab q12w, and placebo groups, respectively.
- Infections (as identified by the investigator) were reported in 48.9%, 33.7%, and 46.3% of subjects in the ustekinumab q8w, ustekinumab q12w, and placebo groups, respectively.
   - Infections requiring oral or parenteral antibiotic treatment were reported in 22.7%, 15.7%, and 19.4% of subjects in the ustekinumab q8w, ustekinumab q12w, and placebo groups, respectively.
- Serious infections were infrequent among randomized subjects and were reported in 1.7%, 3.5%, and 2.3% in the ustekinumab q8w, ustekinumab q12w, and placebo groups, respectively. Opportunistic infections were identified in 3 subjects (all in the randomized population); cytomegalovirus colitis was diagnosed for 2 subjects in the ustekinumab q12w group and 1 subject was diagnosed with concurrent moderate AEs of ophthalmic and labial herpes. No cases of active TB were reported among ustekinumab-treated subjects through Week 44.
- The proportion of randomized subjects with AEs leading to discontinuation of study agent was higher in the placebo group than in the q12w and q8w groups and the most frequent AEs leading to discontinuation in the placebo group was worsening UC.
- Among all treated subjects, including delayed ustekinumab induction responders, the overall safety profile was consistent with that observed in the randomized population.
- There was 1 death reported for a subject who was a delayed ustekinumab induction responder and was receiving ustekinumab q8w. The cause of death was attributed to acute respiratory failure that occurred during thyroid surgery for a multinodular goiter.
- Among all treated subjects, 2 subjects (1 subject in the ustekinumab induction delayed-responders group [receiving ustekinumab q8w] and 1 subject randomized to the placebo group who had received ustekinumab IV during induction) reported serious major adverse cardiovascular events; both events were associated with perioperative complications.
- Among all treated subjects, there were 6 subjects for whom malignancies were reported (5 ustekinumab-treated subjects and 1 placebo-only subject).
   - Three ustekinumab-treated subjects reported non-melanoma skin cancers (NMSCs); all had either a prior history of azathioprine or 6-MP treatment and 2 were on concomitant immunomodulator therapy at the time of the diagnosis.
   - Two ustekinumab-treated subjects were reported to have solid tumors; one subject with a papillary renal cell carcinoma (q12w) and one subject with colon cancer (q8w); both tumors were detected early during the subject's participation in this maintenance study.
- There were no cases of anaphylaxis or delayed hypersensitivity reactions identified among ustekinumab treated subjects.
- There were no notable differences in the proportions of subjects with post-baseline maximum toxicity Grade ≥1 chemistry and hematology laboratory between the placebo and respective ustekinumab groups. Grade 3 and Grade 4 chemistry and hematology laboratory values were infrequent.

### HEALTH ECONOMICS AND MEDICAL RESOURCE UTILIZATION RESULTS

- Through Week 44, fewer subjects in the combined ustekinumab group had a UC disease-related hospitalization or surgery compared with the placebo group.
- At Week 44, change from maintenance baseline in productivity visual analog scores (VAS) demonstrated improvement in subjects in the ustekinumab treatment groups and worsening in subjects in the placebo group.
- At Week 44, percentages within each of the 4 WPAI-GH domains were maintained from maintenance baseline for the ustekinumab treatment groups, with additional improvement observed in subjects in the ustekinumab q8w group for percent impairment while working due to health, percent overall work impairment due to health, and percent activity impairment due to health. For subjects in the placebo group, percentages for all 4 WPAI-GH domains worsened (ie, increased).

### CONCLUSIONS

- The ustekinumab maintenance study provided consistent and definitive evidence that the ustekinumab 90 mg SC q12w and q8w dose regimens were both effective in adult subjects with moderately to severely active UC who had responded to a single IV ustekinumab induction dose.
   - The efficacy of ustekinumab was observed in subjects who were biologic failures as well as those who failed conventional but not biologic therapy (ie, biology-naive).
   - Of note, while both doses of ustekinumab were effective, the q8w dose regimen demonstrated modestly better efficacy across several objective and/or more stringent endpoints (eg, endoscopic healing and durable partial Mayo remission) as well as in overtime analyses of symptomatic and partial Mayo remission.
- Maintenance dosing with ustekinumab SC dose regimens of 90 mg q12w and 90 mg q8w was generally well-tolerated over 44 weeks in this population of adult subjects with moderate to severe ulcerative colitis.
- The safety and efficacy data from this study support a positive benefit/risk profile for ustekinumab SC maintenance therapy.

The U.S. Food and Drug Administration has approved STELARA^{®} (ustekinumab) for the treatment of ulcerative colitis (UC) in the U.S. as of October 18, 2019. The approved label is shown in Annex I below.

The present invention further comprises a pharmaceutical composition of an anti-IL-12/IL-23p40 antibody and packaging comprising one or more label elements disclosed in Annexes I, II and III, wherein the antibody comprises: (i) a heavy chain variable region and a light chain variable region, the heavy chain variable region comprising: a complementarity determining region heavy chain 1 (CDRH1) amino acid sequence of SEQ ID NO:1; a CDRH2 amino acid sequence of SEQ ID NO:2; and a CDRH3 amino acid sequence of SEQ ID NO:3; and the light chain variable region comprising: a complementarity determining region light chain 1 (CDRL1) amino acid sequence of SEQ ID NO:4; a CDRL2 amino acid sequence of SEQ ID NO:5; and a CDRL3 amino acid sequence of SEQ ID NO:6; (ii) a heavy chain variable region of the amino acid sequence of SEQ ID NO: 7 and a light chain variable region of the amino acid sequence of SEQ ID NO:8; or (iii) a heavy chain of the amino acid sequence of SEQ ID NO:10 and a light chain of the amino acid sequence of SEQ ID NO:11.

### ANNEX I

### HIGHLIGHTS OF PRESCRIBING INFORMATION

**These highlights do not include all the information needed to use STELARA^{®} safety and effectively. See full prescribing information** for **STELARA^{®}.**

**STELARA^{®} (ustekinumab) injection, for subcutaneous or intravenous use**

**Initial U.S. Approval: 2009**

### -------------------------- RECENT MAJOR CHANGES ---------------------------

| | |
|---|---|
| Indications and Usage, Ulcerative Colitis (1.4) | 10/2019 |
| Dosage and Administration (2.3) | 10/2019 |
| Warnings and Precautions (5.1) | 10/2019 |

### --------------------------- INDICATIONS AND USAGE---------------------------

STELARA^{®} is a human interleukin-12 and -23 antagonist indicated for the treatment of

Adult patients with
- *moderate to severe plaque psoriasis (Ps)* who are candidates for phototherapy or systemic therapy (1.1)
- *active psoriatic arthritis (PsA),* alone or in combination with methotrexate. (12)
- *moderately to severely active Crohn's disease (CD).* (1.3)
- *moderately to severely active ulcerative colitis.* (1.4)

Adolescent patients (12 years or older) with
- *moderate to severe plaque psoriasis,* who are candidates for phototherapy or systemic therapy (1.1)

### --------------------------- DOSAGE AND ADMINISTRATION-----------------------

Psonasis Adult Subcutaneous Recommended Dosage (2.1):

| Weight Range (kilogram) | Dosage Regimen |
|---|---|
| less than or equal to 100 kg | 45 mg administered subcutaneously initially and 4 weeks later, followed by 45 mg administered subcutaneously every 12 weeks |
| greater than 100 kg | 90 mg administered subcutaneously initially and 4 weeks later, followed by 90 mg administered subcutaneously every 12 weeks |

Psonasis Adolescent (12 years and older) Subcutaneous Recommended Dosage (2.1): Weight based dosing is recommended at the initial dose, 4 weeks later, then every 12 weeks thereafter.

| Weight Range (kilogram) | Dosage Regimen |
|---|---|
| less than 60 kg | 0.75 mg/kg |
| 60 kg to 100 kg | 45 mg |
| greater than 100 kg | 90 mg |

*Psoriatic Arthritis Adult Subcutaneous Recommended Dosage (2.2)*:
- The recommended dosage is 45 mg administered subcutaneously initially and 4 weeks later, followed by 45 mg administered subcutaneously every 12 weeks.
- For patients with co-existent moderate-to-severe plaque psoriasis weighing greater than 100 kg, the recommended dosage is 90 mg administered subcutaneously initially and 4 weeks later, followed by 90 mg administered subcutaneously every 12 weeks.

Crohn's Disease and Ulcerative Colitis Initial Adult Intravenous Recommended Dosage (2.3): A single intravenous infusion using weight-based dosing

| Weight Range (kilogram) | Recommended Dosage |
|---|---|
| up to 55 kg | 260 mg (2 vials) |
| greater than 55 kg to 85 kg | 390 mg (3 vials) |
| greater than 85 kg | 520 mg (4 vials) |

Crohn's Disease and Ulcerative Colitis Maintenance Adult Subcutaneous Recommended Dosage (2.3): A subcutaneous 90 mg dose 8 weeks after the initial intravenous dose, then every 8 weeks thereafter.

### --------------------------- DOSAGE FORMS AND STRENGTHS---------------------------

### Subcutaneous Injection (3)

- Injection 45 mg/0.5 mL or 90 mg/mL in a single-dose prefilled syringe
- Injection 45 mg/0.5 mL in a single-dose vial

### Intravenous Infusion (3)

- Injection 130 mg/26 mL (5 mg/mL) solution in a single-dose vial (3)

### ----------------------------- CONTRAINDICATIONS---------------------------------

Clinically significant hypersensitivity to ustekinumab or to any of the excipients. (4)

### -------------------------WARNINGS AND PRECAUTIONS----------------------

- Infections: Serious infections have occurred. Do not start STELARA^{®} during any clinically important active infection. If a serious infection or clinically significant infection develops, consider discontinuing STELARA^{®} until the infection resolves. (5.1)
- Theoretical Risk for Particular Infections: Serious infections from mycobacteria, salmonella and Bacillus Calmette-Guerin (BCG) vaccinations have been reported in patients genetically deficient in IL-12/IL-23. Diagnostic tests for these infections should be considered as dictated by clinical circumstances. (52)
- Tuberculosis (TB): Evaluate patients for TB prior to initiating treatment with STELARA^{®}. Initiate treatment of latent TB before administering STELARA^{®}. (5.3)
- Malignancies: STELARA^{®} may increase nsk of malignancy. The safety of STELARA^{®} in patients with a history of or a known malignancy has not been evaluated. (5.4)
- Hypersensitivity Reactions: Anaphylaxis or other clinically significant hypersensitivity reactions may occur (5.5)
- Reversible Posterior Leukoencephalopathy Syndrome (RPLS): One case was reported. If suspected, treat promptly and discontinue STELARA^{®}. (5.6)
- Noninfectious Pneumonia: Cases of interstitial pneumonia, eosinophilic pneumonia and cryptogenic organizing pneumonia have been reported during post-approval use of STELARA^{®}. If diagnosis is confirmed, discontinue STELARA^{®} and institute appropriate treatment. (5.9)

### ------------------------------ ADVERSE REACTIONS -------------------------------

Most common adverse reactions are • Psonasis (≥3%): nasopharyngitis, upper respiratory tract infection, headache, and fatigue. (6.1)
- Crohn's Disease, induction (≥3%) vomiting. (6.1)
- Crohn's Disease, maintenance (≥3%): nasopharyngitis, injection site erythema, vulvovaginal candidiasis/mycotic infection, bronchitis, pruntus, unnary tract infection, and sinusitis. (6.1)
- Ulcerative colitis, induction (≥3%): nasopharyngitis (6.1)
- Ulcerative colitis, maintenance (≥3%): nasopharyngitis, headache, abdominal pain, influenza, fever, diarrhea, sinusitis, fatigue, and nausea (6.1)

**To report SUSPECTED ADVERSE REACTIONS, contact Janssen Biotech, Inc. at 1-800-JANSSEN (1-800-526-7736) or FDA at 1-800-FDA-1088 or *www.fda.gov*/*medwatch.***

**See 17 for PATIENT COUNSELING INFORMATION and Medication Guide.**

### FULL PRESCRIBING INFORMATION: CONTENTS*

**1 INDICATIONS AND USAGE**
   1.1 Psoriasis (Ps)
   1.2 Psoriatic Arthritis (PsA)
   1.3 Crohn's Disease (CD)
   *1.4 Ulcerative Colitis*
**2 DOSAGE AND ADMINISTRATION**
   2.1 Psoriasis
   2.2 Psoriatic Arthritis
   2.3 Crohn's Disease *and Ulcerative Colitis*
   2.4 General Considerations for Administration
   2.5 Instructions for Administration of STELARA^{®} Prefilled Syringes Equipped with Needle Safety Guard
   2.6 Preparation and Administration of STELARA^{®} 130 mg/26 mL (5 mg/mL) Vial for Intravenous Infusion (Crohn's Disease *and Ulcerative Colitis*)
**3 DOSAGE FORMS AND STRENGTHS**
**4 CONTRAINDICATIONS**
**5 WARNINGS AND PRECAUTIONS**
   5.1 Infections
   5.2 Theoretical Risk for Vulnerability to Particular Infections
   5.3 Pre-treatment Evaluation for Tuberculosis
   5.4 Malignancies
   5.5 Hypersensitivity Reactions
   5.6 Reversible Posterior Leukoencephalopathy Syndrome
   5.7 Immunizations
   5.8 Concomitant Therapies
   5.9 Noninfectious Pneumonia
**6 ADVERSE REACTIONS**
   6.1 Clinical Trials Experience
   6.2 Immunogenicity
   6.3 Postmarketing Experience
**7 DRUG INTERACTIONS**
   7.1 Live Vaccines
   7.2 Concomitant Therapies
   7.3 CYP450 Substrates
   7.4 Allergen Immunotherapy
**8 USE IN SPECIFIC POPULATIONS**
   8.1 Pregnancy
   8.2 Lactation
   8.4 Pediatric Use
   8.5 Geriatric Use
**10 OVERDOSAGE**
**11 DESCRIPTION**
**12 CLINICAL PHARMACOLOGY**
   12.1 Mechanism of Action
   12.2 Pharmacodynamics
   12.3 Pharmacokinetics
**13 NONCLINICAL TOXICOLOGY**
   13.1 Carcinogenesis, Mutagenesis, Impairment of Fertility
   13.2 Animal Toxicology and/or Pharmacology
**14 CLINICAL STUDIES**
   14.1 Psoriasis
   14.2 Adolescent Subjects with Plaque Psoriasis
   14.3 Psoriatic Arthritis
   14.4 Crohn's Disease
   *14.5 Ulcerative Colitis*
**15 REFERENCES**
**16 HOW SUPPLIED/STORAGE AND HANDLING**
**17 PATIENT COUNSELING INFORMATION**

*Sections or subsections omitted from the full presenting information are not listed.

### FULL PRESCRIBING INFORMATION

**1** INDICATIONS AND USAGE
   **1.1** Psoriasis (Ps)
      STELARA^{®} is indicated for the treatment of patients 12 years or older with moderate to severe plaque psoriasis who are candidates for phototherapy or systemic therapy.
   **1.2** Psoriatic Arthritis (PsA)
      STELARA^{®} is indicated for the treatment of adult patients with active psoriatic arthritis. STELARA^{®} can be used alone or in combination with methotrexate (MTX).
   **1.3** Crohn's Disease (CD)
      STELARA^{®} is indicated for the treatment of adult patients with moderately to severely active Crohn's disease.
   **1.4 Ulcerative Colitis**
      STELARA^{®} is indicated for the treatment of adult patients with moderately to severely active ulcerative colitis.
**2** DOSAGE AND ADMINISTRATION
   **2.1** Psoriasis
      Subcutaneous Adult Dosage Regimen
      - For patients weighing 100 kg or less, the recommended dose is 45 mg initially and 4 weeks later, followed by 45 mg every 12 weeks.
      - For patients weighing more than 100 kg, the recommended dose is 90 mg initially and 4 weeks later, followed by 90 mg every 12 weeks.

In subjects weighing more than 100 kg, 45 mg was also shown to be efficacious. However, 90 mg resulted in greater efficacy in these subjects *[see Clinical Studies (14)].*

### Subcutaneous Adolescent Dosage Regimen

Administer STELARA^{®} subcutaneously at Weeks 0 and 4, then every 12 weeks thereafter.

The recommended dose of STELARA^{®} for adolescents (12-17 years old) based on body weight is shown below (Label Table 1).

**Label Table 1: Recommended Dose of STELARA^{®} for Subcutaneous Injection in Adolescent Patients with Psoriasis**

| **Body Weight of Patient at the Time of Dosing** | **Recommended Dose** |
|---|---|
| less than 60 kg | 0.75 mg/kg |
| 60 kg to 100 kg | 45 mg |
| more than 100 kg | 90 mg |

For adolescent patients weighing less than 60 kg, the administration volume for the recommended dose (0.75 mg/kg) is shown in Label Table 2; withdraw the appropriate volume from the single-dose vial.

**Label Table 2: Injection volumes of STELARA^{®} 45 mg/0.5mL single-dose vials for adolescent psoriasis patients less than 60 kg**

| **Body Weight (kg) at the time of dosing** | **Dose (mg)** | **Volume of injection (mL)** |
|---|---|---|
| 30 | 22.5 | 0.25 |
| 31 | 23.3 | 0.26 |
| 32 | 24 | 0.27 |
| 33 | 24.8 | 0.27 |
| 34 | 25.5 | 0.28 |
| 35 | 26.3 | 0.29 |
| 36 | 27 | 0.3 |
| 37 | 27.8 | 0.31 |
| 38 | 28.5 | 0.32 |
| 39 | 29.3 | 0.32 |
| 40 | 30 | 0.33 |
| 41 | 30.8 | 0.34 |
| 42 | 31.5 | 0.35 |
| 43 | 32.3 | 0.36 |
| 44 | 33 | 0.37 |
| 45 | 33.8 | 0.37 |
| 46 | 34.5 | 0.38 |
| 47 | 35.3 | 0.39 |
| 48 | 36 | 0.4 |
| 49 | 36.8 | 0.41 |
| 50 | 37.5 | 0.42 |
| 51 | 38.3 | 0.42 |
| 52 | 39 | 0.43 |
| 53 | 39.8 | 0.44 |
| 54 | 40.5 | 0.45 |
| 55 | 41.3 | 0.46 |
| 56 | 42 | 0.46 |
| 57 | 42.8 | 0.47 |
| 58 | 43.5 | 0.48 |
| 59 | 44.3 | 0.49 |

### 2.2 Psoriatic Arthritis

### Subcutaneous Adult Dosage Regimen

- The recommended dose is 45 mg initially and 4 weeks later, followed by 45 mg every 12 weeks.
- For patients with co-existent moderate-to-severe plaque psoriasis weighing more than 100 kg, the recommended dose is 90 mg initially and 4 weeks later, followed by 90 mg every 12 weeks.

### 2.3 Crohn's Disease and Ulcerative Colitis

### Intravenous Induction Adult Dosage Regimen

A single intravenous infusion dose of STELARA^{®} using the weight-based dosage regimen specified in Label Table 3 *[see Instructions for dilution of STELARA*^{®} *130 mg vial for intravenous infusion (2.6)].*

**Label Table 3: Initial Intravenous Dosage of STELARA^{®}**

| **Body Weight of Patient at the time of dosing** | **Dose** | **Number of 130 mg/26 mL (5 mg/mL) STELARA^{®} vials** |
|---|---|---|
| 55 kg or less | 260 mg | 2 |
| more than 55 kg to 85 kg | 390 mg | 3 |
| more than 85 kg | 520 mg | 4 |

### Subcutaneous Maintenance Adult Dosage Regimen

The recommended maintenance dosage is a subcutaneous 90 mg dose administered 8 weeks after the initial intravenous dose, then every 8 weeks thereafter.

### 2.4 General Considerations for Administration

- STELARA^{®} is intended for use under the guidance and supervision of a physician. STELARA^{®} should only be administered to patients who will be closely monitored and have regular follow-up visits with a physician. The appropriate dose should be determined by a healthcare provider using the patient's current weight at the time of dosing. In adolescent patients, it is recommended that STELARA^{®} be administered by a health care provider. If a physician determines that it is appropriate, a patient may self-inject or a caregiver may inject STELARA^{®} after proper training in subcutaneous injection technique. Patients should be instructed to follow the directions provided in the Medication Guide *[see Medication Guide].*
- The needle cover on the prefilled syringe contains dry natural rubber (a derivative of latex). The needle cover should not be handled by persons sensitive to latex.
- It is recommended that each injection be administered at a different anatomic location (such as upper arms, gluteal regions, thighs, or any quadrant of abdomen) than the previous injection, and not into areas where the skin is tender, bruised, erythematous, or indurated. When using the single-dose vial, a 1 mL syringe with a 27 gauge, ½ inch needle is recommended.
- Prior to administration, visually inspect STELARA^{®} for particulate matter and discoloration. STELARA^{®} is a colorless to light yellow solution and may contain a few small translucent or white particles. Do not use STELARA^{®} if it is discolored or cloudy, or if other particulate matter is present. STELARA^{®} does not contain preservatives; therefore, discard any unused product remaining in the vial and/or syringe.

### 2.5 Instructions for Administration of STELARA^{®} Prefilled Syringes Equipped with Needle Safety Guard

Refer to the diagram below for the provided instructions.

**To prevent premature activation of the needle safety guard, do not touch the NEEDLE GUARD ACTIVATION CLIPS at any time during use.** 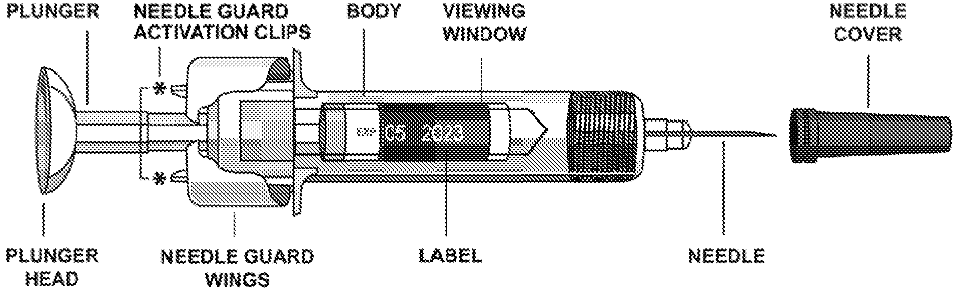
PLUNGER
NEEDLE GUARD
ACTIVATION CLIPS
BODY
VIEWING
WINDOW
NEEDLE
COVER
EXP 05 2023
PLUNGER
HEAD
NEEDLE GUARD
WINGS
LABEL
NEEDLE
- Hold the BODY and remove the NEEDLE COVER. **Do not hold the PLUNGER or PLUNGER HEAD while removing the NEEDLE COVER or the PLUNGER may move. Do not use the prefilled syringe if it is dropped without the NEEDLE COVER in place.**
- Inject STELARA^{®} subcutaneously as recommended *[see Dosage and Administration (2.1, 2.2, 2.3)].*
- Inject all of the medication by pushing in the PLUNGER until the PLUNGER HEAD is completely between the needle guard wings. **Injection of the entire prefilled syringe contents is necessary to activate the needle guard**. 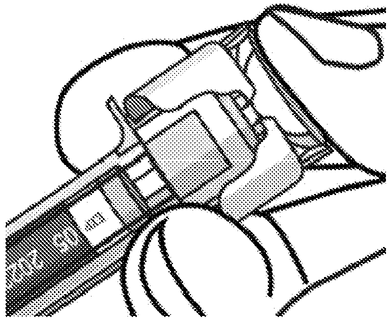
- After injection, maintain the pressure on the PLUNGER HEAD and remove the needle from the skin. Slowly take your thumb off the PLUNGER HEAD to allow the empty syringe to move up until the entire needle is covered by the needle guard, as shown by the illustration below: 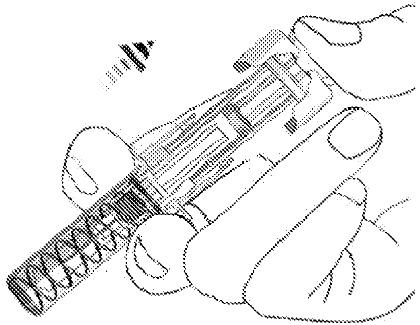
- Used syringes should be placed in a puncture-resistant container.

### 2.6 Preparation and Administration of STELARA^{®} 130 mg/26 mL (5 mg/mL) Vial for Intravenous Infusion (Crohn's Disease and Ulcerative Colitis)

STELARA^{®} solution for intravenous infusion must be diluted, prepared and infused by a healthcare professional using aseptic technique.
1. Calculate the dose and the number of STELARA^{®} vials needed based on patient weight (Label Table 3). Each 26 mL vial of STELARA^{®} contains 130 mg of ustekinumab.
2. Withdraw, and then discard a volume of the 0.9% Sodium Chloride Injection, USP from the 250 mL infusion bag equal to the volume of STELARA^{®} to be added (discard 26 mL sodium chloride for each vial of STELARA^{®} needed, for 2 vials- discard 52 mL, for 3 vials- discard 78 mL, 4 vials- discard 104 mL).
3. Withdraw 26 mL of STELARA^{®} from each vial needed and add it to the 250 mL infusion bag. The final volume in the infusion bag should be 250 mL. Gently mix.
4. Visually inspect the diluted solution before infusion. Do not use if visibly opaque particles, discoloration or foreign particles are observed.
5. Infuse the diluted solution over a period of at least one hour. Once diluted, the infusion solution may be stored for up to four hours prior to infusion.
6. Use only an infusion set with an in-line, sterile, non-pyrogenic, low protein-binding filter (pore size 0.2 micrometer).
7. Do not infuse STELARA^{®} concomitantly in the same intravenous line with other agents.
8. STELARA^{®} does not contain preservatives. Each vial is for single use only. Discard any remaining solution. Dispose any unused medicinal product in accordance with local requirements.

### Storage

If necessary, the diluted infusion solution may be stored for up to 4 hours at room temperature up to 25°C (77°F). Do not freeze. Discard any unused portion of the infusion solution.

### 3 DOSAGE FORMS AND STRENGTHS

STELARA^{®} (ustekinumab) is a colorless to light yellow solution and may contain a few small translucent or white particles.

### Subcutaneous Injection

- Injection: 45 mg/0.5 mL or 90 mg/mL solution in a single-dose prefilled syringe
- Injection: 45 mg/0.5 mL solution in a single-dose vial

### Intravenous Infusion

- Injection: 130 mg/26 mL (5 mg/mL) solution in a single-dose vial

### 4 CONTRAINDICATIONS

STELARA^{®} is contraindicated in patients with clinically significant hypersensitivity to ustekinumab or to any of the excipients *[see Warnings and Precautions (5.5)].*

### 5 WARNINGS AND PRECAUTIONS

### 5.1 Infections

STELARA^{®} may increase the risk of infections and reactivation of latent infections. Serious bacterial, fungal, and viral infections were observed in subjects receiving STELARA^{®} *[see Adverse Reactions (6.1)].*

Serious infections requiring hospitalization, or otherwise clinically significant infections, reported in clinical studies included the following:
- *Psoriasis*: diverticulitis, cellulitis, pneumonia, appendicitis, cholecystitis, sepsis, osteomyelitis, viral infections, gastroenteritis and urinary tract infections.
- *Psoriatic arthritis*: cholecystitis.
- *Crohn's disease*: anal abscess, gastroenteritis, ophthalmic herpes zoster, pneumonia, and listeria meningitis.
- *Ulcerative colitis*: gastroenteritis, ophthalmic herpes zoster, pneumonia, and listeriosis.

Treatment with STELARA^{®} should not be initiated in patients with any clinically important active infection until the infection resolves or is adequately treated. Consider the risks and benefits of treatment prior to initiating use of STELARA^{®} in patients with a chronic infection or a history of recurrent infection.

Instruct patients to seek medical advice if signs or symptoms suggestive of an infection occur while on treatment with STELARA^{®} and consider discontinuing STELARA^{®} for serious or clinically significant infections until the infection resolves or is adequately treated.

### 5.2 Theoretical Risk for Vulnerability to Particular Infections

Individuals genetically deficient in IL-12/IL-23 are particularly vulnerable to disseminated infections from mycobacteria (including nontuberculous, environmental mycobacteria), salmonella (including nontyphi strains), and Bacillus Calmette-Guerin (BCG) vaccinations. Serious infections and fatal outcomes have been reported in such patients.

It is not known whether patients with pharmacologic blockade of IL-12/IL-23 from treatment with STELARA^{®} may be susceptible to these types of infections. Appropriate diagnostic testing should be considered, e.g., tissue culture, stool culture, as dictated by clinical circumstances.

### 5.3 Pre-treatment Evaluation for Tuberculosis

Evaluate patients for tuberculosis infection prior to initiating treatment with STELARA^{®}.

Do not administer STELARA^{®} to patients with active tuberculosis infection. Initiate treatment of latent tuberculosis prior to administering STELARA^{®}. Consider anti-tuberculosis therapy prior to initiation of STELARA^{®} in patients with a past history of latent or active tuberculosis in whom an adequate course of treatment cannot be confirmed. Closely monitor patients receiving STELARA^{®} for signs and symptoms of active tuberculosis during and after treatment.

### 5.4 Malignancies

STELARA^{®} is an immunosuppressant and may increase the risk of malignancy. Malignancies were reported among subjects who received STELARA^{®} in clinical studies *[see Adverse Reactions (6.1)].* In rodent models, inhibition of IL-12/IL-23p40 increased the risk of malignancy *[see Nonclinical Toxicology (13)].*

The safety of STELARA^{®} has not been evaluated in patients who have a history of malignancy or who have a known malignancy.

There have been post-marketing reports of the rapid appearance of multiple cutaneous squamous cell carcinomas in patients receiving STELARA^{®} who had pre-existing risk factors for developing non-melanoma skin cancer. All patients receiving STELARA^{®} should be monitored for the appearance of non-melanoma skin cancer. Patients greater than 60 years of age, those with a medical history of prolonged immunosuppressant therapy and those with a history of PUVA treatment should be followed closely *[see Adverse Reactions (6.1)].*

### 5.5 Hypersensitivity Reactions

Hypersensitivity reactions, including anaphylaxis and angioedema, have been reported with STELARA^{®} *[see Adverse Reactions (6.1, 6.3)].* If an anaphylactic or other clinically significant hypersensitivity reaction occurs, institute appropriate therapy and discontinue STELARA^{®}.

### 5.6 Reversible Posterior Leukoencephalopathy Syndrome

One case of reversible posterior leukoencephalopathy syndrome (RPLS) was observed in clinical studies of psoriasis and psoriatic arthritis. The subject, who had received 12 doses of STELARA^{®} over approximately two years, presented with headache, seizures and confusion. No additional STELARA^{®} injections were administered and the subject fully recovered with appropriate treatment. No cases of RPLS were observed in clinical studies of Crohn's disease or ulcerative colitis.

RPLS is a neurological disorder, which is not caused by demyelination or a known infectious agent. RPLS can present with headache, seizures, confusion and visual disturbances. Conditions with which it has been associated include preeclampsia, eclampsia, acute hypertension, cytotoxic agents and immunosuppressive therapy. Fatal outcomes have been reported.

If RPLS is suspected, administer appropriate treatment and discontinue STELARA^{®}.

### 5.7 Immunizations

Prior to initiating therapy with STELARA^{®}, patients should receive all age-appropriate immunizations as recommended by current immunization guidelines. Patients being treated with STELARA^{®} should not receive live vaccines. BCG vaccines should not be given during treatment with STELARA^{®} or for one year prior to initiating treatment or one year following discontinuation of treatment. Caution is advised when administering live vaccines to household contacts of patients receiving STELARA^{®} because of the potential risk for shedding from the household contact and transmission to patient.

Non-live vaccinations received during a course of STELARA^{®} may not elicit an immune response sufficient to prevent disease.

### 5.8 Concomitant Therapies

In clinical studies of psoriasis the safety of STELARA^{®} in combination with other immunosuppressive agents or phototherapy was not evaluated. Ultraviolet-induced skin cancers developed earlier and more frequently in mice genetically manipulated to be deficient in both IL-12 and IL-23 or IL-12 alone *[see Nonclinical Toxicology (13.1)].*

### 5.9 Noninfectious Pneumonia

Cases of interstitial pneumonia, eosinophilic pneumonia and cryptogenic organizing pneumonia have been reported during post-approval use of STELARA^{®}. Clinical presentations included cough, dyspnea, and interstitial infiltrates following one to three doses. Serious outcomes have included respiratory failure and prolonged hospitalization. Patients improved with discontinuation of therapy and in certain cases administration of corticosteroids. If diagnosis is confirmed, discontinue STELARA^{®} and institute appropriate treatment *[see Postmarketing Experience (6.3)].*

### 6 ADVERSE REACTIONS

The following serious adverse reactions are discussed elsewhere in the label:
- Infections *[see Warnings and Precautions (5.1)]*
- Malignancies *[see Warnings and Precautions (5.4)]*
- Hypersensitivity Reactions *[see Warnings and Precautions (5.5)]*
- Reversible Posterior Leukoencephalopathy Syndrome *[see Warnings and Precautions (5.6)]*

### 6.1 Clinical Trials Experience

Because clinical trials are conducted under widely varying conditions, adverse reaction rates observed in the clinical trials of a drug cannot be directly compared to rates in the clinical trials of another drug and may not reflect the rates observed in practice.

### Adult Subjects with Plaque Psoriasis

The safety data reflect exposure to STELARA^{®} in 3117 adult psoriasis subjects, including 2414 exposed for at least 6 months, 1855 exposed for at least one year, 1653 exposed for at least two years, 1569 exposed for at least three years, 1482 exposed for at least four years and 838 exposed for at least five years.

Label Table 4 summarizes the adverse reactions that occurred at a rate of at least 1% and at a higher rate in the STELARA^{®} groups than the placebo group during the placebo-controlled period of Ps STUDY 1 and Ps STUDY 2 *[see Clinical Studies (14)].*

**Label Table 4: Adverse Reactions Reported by ≥1% of Subjects through Week 12 in Ps STUDY 1 and Ps STUDY 2**

| | | | **STELARA^{®}** | |
|---|---|---|---|---|
| | | **Placebo** | **45 mg** | **90 mg** |
| **Subjects treated** | | **665** | **664** | **666** |
| | Nasopharyngitis | 51 (8%) | 56 (8%) | 49 (7%) |
| | Upper respiratory tract infection | 30 (5%) | 36 (5%) | 28 (4%) |
| | Headache | 23 (3%) | 33 (5%) | 32 (5%) |
| | Fatigue | 14 (2%) | 18(3%) | 17 (3%) |
| | Diarrhea | 12 (2%) | 13 (2%) | 13 (2%) |
| | Back pain | 8 (1%) | 9 (1%) | 14 (2%) |
| | Dizziness | 8 (1%) | 8 (1%) | 14 (2%) |
| | Pharyngolaryngeal pain | 7 (1%) | 9 (1%) | 12 (2%) |
| | Pruritus | 9 (1%) | 10 (2%) | 9 (1%) |
| | Injection site erythema | 3 (<1%) | 6 (1%) | 13 (2%) |
| | Myalgia | 4 (1%) | 7 (1%) | 8 (1%) |
| | Depression | 3 (<1%) | 8 (1%) | 4 (1%) |

Adverse reactions that occurred at rates less than 1% in the controlled period of Ps STUDIES 1 and 2 through week 12 included: cellulitis, herpes zoster, diverticulitis and certain injection site reactions (pain, swelling, pruritus, induration, hemorrhage, bruising, and irritation).

One case of RPLS occurred during clinical studies *[see Warnings and Precautions (5.6)].*

### • Infections

In the placebo-controlled period of clinical studies of psoriasis subjects (average follow-up of 12.6 weeks for placebo-treated subjects and 13.4 weeks for STELARA^{®}-treated subjects), 27% of STELARA^{®}-treated subjects reported infections (1.39 per subject-year of follow-up) compared with 24% of placebo-treated subjects (1.21 per subject-year of follow-up). Serious infections occurred in 0.3% of STELARA^{®}-treated subjects (0.01 per subject-year of follow-up) and in 0.4% of placebo-treated subjects (0.02 per subject-year of follow-up) *[see Warnings and Precautions (5.1)].*

In the controlled and non-controlled portions of psoriasis clinical studies (median follow-up of 3.2 years), representing 8998 subject-years of exposure, 72.3% of STELARA^{®}-treated subjects reported infections (0.87 per subject-years of follow-up). Serious infections were reported in 2.8% of subjects (0.01 per subject-years of follow-up).

### • Malignancies

In the controlled and non-controlled portions of psoriasis clinical studies (median follow-up of 3.2 years, representing 8998 subject-years of exposure), 1.7% of STELARA^{®}-treated subjects reported malignancies excluding non-melanoma skin cancers (0.60 per hundred subject-years of follow-up). Non-melanoma skin cancer was reported in 1.5% of STELARA^{®}-treated subjects (0.52 per hundred subject-years of follow-up) *[see Warnings and Precautions (5.4)].* The most frequently observed malignancies other than non-melanoma skin cancer during the clinical studies were: prostate, melanoma, colorectal and breast. Malignancies other than non-melanoma skin cancer in STELARA^{®}-treated patients during the controlled and uncontrolled portions of studies were similar in type and number to what would be expected in the general U.S. population according to the SEER database (adjusted for age, gender and race).¹

### Adolescent Subjects with Plaque Psoriasis

The safety of STELARA^{®} was assessed in a study of 110 subjects 12 to 17 years of age with moderate to severe plaque psoriasis. The safety profile in these subjects through Week 60 was similar to the safety profile from studies in adults with plaque psoriasis.

### Psoriatic Arthritis

The safety of STELARA^{®} was assessed in 927 patients in two randomized, double-blind, placebo-controlled studies in adult patients with active psoriatic arthritis (PsA). The overall safety profile of STELARA^{®} in patients with PsA was consistent with the safety profile seen in adult psoriasis clinical studies. A higher incidence of arthralgia, nausea, and dental infections was observed in STELARA^{®}-treated patients when compared with placebo-treated patients (3% vs. 1% for arthralgia and 3% vs. 1% for nausea; 1% vs. 0.6% for dental infections) in the placebo-controlled portions of the PsA clinical studies.

### Crohn's Disease

The safety of STELARA^{®} was assessed in 1407 patients with moderately to severely active Crohn's disease (Crohn's Disease Activity Index [CDAI] greater than or equal to 220 and less than or equal to 450) in three randomized, double-blind, placebo-controlled, parallel-group, multicenter studies. These 1407 patients included 40 patients who received a prior investigational intravenous ustekinumab formulation but were not included in the efficacy analyses. In Studies CD-1 and CD-2 there were 470 patients who received STELARA^{®} 6 mg/kg as a weight-based single intravenous induction dose and 466 who received placebo *[see Dosage and Administration (2.3)].* Patients who were responders in either Study CD-1 or CD-2 were randomized to receive a subcutaneous maintenance regimen of either 90 mg STELARA^{®} every 8 weeks, or placebo for 44 weeks in Study CD-3. Patients in these 3 studies may have received other concomitant therapies including aminosalicylates, immunomodulatory agents [azathioprine (AZA), 6-mercaptopurine (6-MP), MTX], oral corticosteroids (prednisone or budesonide), and/or antibiotics for their Crohn's disease *[see Clinical Studies (14.4)].*

The overall safety profile of STELARA^{®} was consistent with the safety profile seen in the adult psoriasis and psoriatic arthritis clinical studies. Common adverse reactions in Studies CD-1 and CD-2 and in Study CD-3 are listed in Label Tables 5 and 6, respectively.

**Label Table 5: Common adverse reactions through Week 8 in Studies CD-1 and CD-2 occurring in ≥3% of STELARA^{®}-treated patients and higher than placebo**

| | **Placebo N=466** | **STELARA^{®} 6 mg/kg single intravenous induction dose N=470** |
|---|---|---|
| Vomiting | 3% | 4% |

Other less common adverse reactions reported in patients in Studies CD-1 and CD-2 included asthenia (1% vs 0.4%), acne (1% vs 0.4%), and pruritus (2% vs 0.4%).

**Label Table 6: Common adverse reactions through Week 44 in Study CD-3 occurring in ≥3% of STELARA^{®}-treated patients and higher than placebo**

| | **Placebo N=133** | **STELARA^{®} 90 mg subcutaneous maintenance dose every 8 weeks N=131** |
|---|---|---|
| Nasopharyngitis | 8% | 11% |
| Injection site erythema | 0 | 5% |
| Vulvovaginal candidiasis/mycotic infection | 1% | 5% |
| Bronchitis | 3% | 5% |
| Pruritus | 2% | 4% |
| Urinary tract infection | 2% | 4% |
| Sinusitis | 2% | 3% |

### • Infections

In patients with Crohn's disease, serious or other clinically significant infections included anal abscess, gastroenteritis, and pneumonia. In addition, listeria meningitis and ophthalmic herpes zoster were reported in one patient each *[see Warnings and Precautions (5.1)].*

### • Malignancies

With up to one year of treatment in the Crohn's disease clinical studies, 0.2% of STELARA^{®}-treated patients (0.36 events per hundred patient-years) and 0.2% of placebo-treated patients (0.58 events per hundred patient-years) developed non-melanoma skin cancer. Malignancies other than non-melanoma skin cancers occurred in 0.2% of STELARA^{®}-treated patients (0.27 events per hundred patient-years) and in none of the placebo-treated patients.

### • Hypersensitivity Reactions Including Anaphylaxis

In CD studies, two patients reported hypersensitivity reactions following STELARA^{®} administration. One patient experienced signs and symptoms consistent with anaphylaxis (tightness of the throat, shortness of breath, and flushing) after a single subcutaneous administration (0.1% of patients receiving subcutaneous STELARA^{®}). In addition, one patient experienced signs and symptoms consistent with or related to a hypersensitivity reaction (chest discomfort, flushing, urticaria, and increased body temperature) after the initial intravenous STELARA^{®} dose (0.08% of patients receiving intravenous STELARA^{®}). These patients were treated with oral antihistamines or corticosteroids and in both cases symptoms resolved within an hour.

### Ulcerative Colitis

The safety of STELARA^{®} was evaluated in two randomized, double-blind, placebo-controlled clinical studies (UC-1 [IV induction] and UC-2 [SC maintenance]) in 960 adult patients with moderately to severely active ulcerative colitis *[see Clinical Studies (14.5)].* The overall safety profile of STELARA^{®} in patients with ulcerative colitis was consistent with the safety profile seen across all approved indications. Adverse reactions reported in at least 3% of STELARA^{®}-treated patients and at a higher rate than placebo were:
- Induction (UC-1): nasopharyngitis (7% vs 4%).
- Maintenance (UC-2): nasopharyngitis (24% vs 20%), headache (10% vs 4%), abdominal pain (7% vs 3%), influenza (6% vs 5%), fever (5% vs. 4%), diarrhea (4% vs 1%), sinusitis (4% vs 1%), fatigue (4% vs 2%), and nausea (3% vs 2%).

### • Infections

In patients with ulcerative colitis, serious or other clinically significant infections included gastroenteritis and pneumonia. In addition, listeriosis and ophthalmic herpes zoster were reported in one patient each *[see Warnings and Precautions (5.1)].*

### • Malignancies

With up to one year of treatment in the ulcerative colitis clinical studies, 0.4% of STELARA^{®}-treated patients (0.48 events per hundred patient-years) and 0.0% of placebo-treated patients (0.00 events per hundred patient-years) developed non-melanoma skin cancer. Malignancies other than non-melanoma skin cancers occurred in 0.5% of STELARA^{®}-treated patients (0.64 events per hundred patient-years) and 0.2% of placebo-treated patients (0.40 events per hundred patient-years).

### 6.2 Immunogenicity

As with all therapeutic proteins, there is potential for immunogenicity. The detection of antibody formation is highly dependent on the sensitivity and specificity of the assay. Additionally, the observed incidence of antibody (including neutralizing antibody) positivity in an assay may be influenced by several factors, including assay methodology, sample handling, timing of sample collection, concomitant medications and underlying disease. For these reasons, comparison of the incidence of antibodies to ustekinumab in the studies described below with the incidence of antibodies to other products may be misleading.

Approximately 6 to 12.4% of subjects treated with STELARA^{®} in psoriasis and psoriatic arthritis clinical studies developed antibodies to ustekinumab, which were generally low-titer. In psoriasis clinical studies, antibodies to ustekinumab were associated with reduced or undetectable serum ustekinumab concentrations and reduced efficacy. In psoriasis studies, the majority of patients who were positive for antibodies to ustekinumab had neutralizing antibodies.

In Crohn's disease and ulcerative colitis clinical studies, 2.9% and 4.6% of patients, respectively, developed antibodies to ustekinumab when treated with STELARA^{®} for approximately one year. No apparent association between the development of antibodies to ustekinumab and the development of injection site reactions was seen.

### 6.3 Postmarketing Experience

The following adverse reactions have been reported during post-approval of STELARA^{®}. Because these reactions are reported voluntarily from a population of uncertain size, it is not always possible to reliably estimate their frequency or establish a causal relationship to STELARA^{®} exposure.

*Immune system disorders:* Serious hypersensitivity reactions (including anaphylaxis and angioedema), other hypersensitivity reactions (including rash and urticaria) *[see Warnings and Precautions (5.5)].*

*Respiratory, thoracic and mediastinal disorders:* Interstitial pneumonia, eosinophilic pneumonia and cryptogenic organizing pneumonia *[see Warnings and Precautions (5.9)].*

*Skin reactions:* Pustular psoriasis, erythrodermic psoriasis.

### 7 DRUG INTERACTIONS

### 7.1 Live Vaccines

Avoid use of live vaccines with STELARA^{®} *[see Warnings and Precautions (5.7)].*

### 7.2 Concomitant Therapies

In psoriasis studies the safety of STELARA^{®} in combination with immunosuppressive agents or phototherapy has not been evaluated *[see Warnings and Precautions (5.8)].* In psoriatic arthritis studies, concomitant MTX use did not appear to influence the safety or efficacy of STELARA^{®}. In Crohn's disease and ulcerative colitis induction studies, immunomodulators (6-NW, AZA, MTX) were used concomitantly in approximately 30% of patients and corticosteroids were used concomitantly in approximately 40% and 50% of Crohn's disease and ulcerative colitis patients, respectively. Use of these concomitant therapies did not appear to influence the overall safety or efficacy of STELARA^{®}.

### 7.3 CYP450 Substrates

The formation of CYP450 enzymes can be altered by increased levels of certain cytokines (e.g., IL-1, IL-6, IL-10, TNFα, IFN) during chronic inflammation. Thus, STELARA^{®}, an antagonist of IL-12 and IL-23, could normalize the formation of CYP450 enzymes. Upon initiation of STELARA^{®} in patients who are receiving concomitant CYP450 substrates, particularly those with a narrow therapeutic index, monitoring for therapeutic effect (e.g., for warfarin) or drug concentration (e.g., for cyclosporine) should be considered and the individual dose of the drug adjusted as needed *[see Clinical Pharmacology (12.3)].*

### 7.4 Allergen Immunotherapy

STELARA^{®} has not been evaluated in patients who have undergone allergy immunotherapy. STELARA^{®} may decrease the protective effect of allergen immunotherapy (decrease tolerance) which may increase the risk of an allergic reaction to a dose of allergen immunotherapy. Therefore, caution should be exercised in patients receiving or who have received allergen immunotherapy, particularly for anaphylaxis.

### 8 USE IN SPECIFIC POPULATIONS

### 8.1 Pregnancy

### Pregnancy Exposure Registry

There is a pregnancy registry that monitors pregnancy outcomes in women exposed to STELARA^{®} during pregnancy. Patients should be encouraged to enroll by calling 1-877-311-8972.

### Risk Summary

Limited data on the use of STELARA^{®} in pregnant women are insufficient to inform a drug associated risk *[see Data].* In animal reproductive and developmental toxicity studies, no adverse developmental effects were observed after administration of ustekinumab to pregnant monkeys at exposures greater than 100 times the human exposure at the maximum recommended human subcutaneous dose (MRHD).

All pregnancies have a background risk of birth defect, loss, or other adverse outcomes. The estimated background risk of major birth defects and miscarriage for the indicated population(s) are unknown. In the U.S. general population, the estimated background risk of major birth defects and miscarriage of clinically recognized pregnancies is 2% to 4% and 15% to 20%, respectively.

### Data

### • Human Data

Limited data on use of STELARA^{®} in pregnant women from observational studies, published case reports, and postmarketing surveillance are insufficient to inform a drug associated risk.

### • Animal Data

Ustekinumab was tested in two embryo-fetal development toxicity studies in cynomolgus monkeys. No teratogenic or other adverse developmental effects were observed in fetuses from pregnant monkeys that were administered ustekinumab subcutaneously twice weekly or intravenously weekly during the period of organogenesis. Serum concentrations of ustekinumab in pregnant monkeys were greater than 100 times the serum concentration in patients treated subcutaneously with 90 mg of ustekinumab weekly for 4 weeks.

In a combined embryo-fetal development and pre- and post-natal development toxicity study, pregnant cynomolgus monkeys were administered subcutaneous doses of ustekinumab twice weekly at exposures greater than 100 times the human subcutaneous exposure from the beginning of organogenesis to Day 33 after delivery. Neonatal deaths occurred in the offspring of one monkey administered ustekinumab at 22.5 mg/kg and one monkey dosed at 45 mg/kg. No ustekinumab-related effects on functional, morphological, or immunological development were observed in the neonates from birth through six months of age.

### 8.2 Lactation

### Risk Summary

There are no data on the presence of ustekinumab in human milk, the effects on the breastfed infant, or the effects on milk production. Ustekinumab was present in the milk of lactating monkeys administered ustekinumab. Due to species-specific differences in lactation physiology, animal data may not reliably predict drug levels in human milk. Maternal IgG is known to be present in human milk. Published data suggest that the systemic exposure to a breastfed infant is expected to be low because ustekinumab is a large molecule and is degraded in the gastrointestinal tract. However, if ustekinumab is transferred into human milk the effects of local exposure in the gastrointestinal tract are unknown.

The developmental and health benefits of breastfeeding should be considered along with the mother's clinical need for STELARA^{®} and any potential adverse effects on the breastfed child from STELARA^{®} or from the underlying maternal condition.

### 8.4 Pediatric Use

The safety and effectiveness of STELARA^{®} have been established in pediatric patients 12 to 17 years old with moderate to severe plaque psoriasis. Use of STELARA^{®} in this age group is supported by evidence from a multicenter, randomized, 60-week trial that included a 12-week, double-blind, placebo-controlled, parallel-group portion, in 110 pediatric subjects 12 years and older *[see Adverse Reactions (6.1), Clinical Studies (14.2)].* The safety and effectiveness of STELARA^{®} for pediatric patients less than 12 years of age with psoriasis have not been established.

The safety and effectiveness of STELARA^{®} have not been established in pediatric patients with psoriatic arthritis, Crohn's disease or ulcerative colitis.

### 8.5 Geriatric Use

Of the 6709 patients exposed to STELARA^{®}, a total of 340 were 65 years or older (183 patients with psoriasis, 65 patients with psoriatic arthritis, 58 patients with Crohn's disease and 34 patients with ulcerative colitis), and 40 patients were 75 years or older. Although no overall differences in safety or efficacy were observed between older and younger patients, the number of patients aged 65 and over is not sufficient to determine whether they respond differently from younger patients.

### 10 OVERDOSAGE

Single doses up to 6 mg/kg intravenously have been administered in clinical studies without dose-limiting toxicity. In case of overdosage, it is recommended that the patient be monitored for any signs or symptoms of adverse reactions or effects and appropriate symptomatic treatment be instituted immediately.

### 11 DESCRIPTION

Ustekinumab is a human IgG1κ monoclonal antibody against the p40 subunit of the IL-12 and IL-23 cytokines. Using DNA recombinant technology, ustekinumab is produced in a well characterized recombinant cell line and is purified using standard bio-processing technology. The manufacturing process contains steps for the clearance of viruses. Ustekinumab is comprised of 1326 amino acids and has an estimated molecular mass that ranges from 148,079 to 149,690 Daltons.

STELARA^{®} (ustekinumab) Injection is a sterile, preservative-free, colorless to light yellow solution and may contain a few small translucent or white particles with pH of 5.7- 6.3.

### STELARA^{®} for Subcutaneous Use

Available as 45 mg of ustekinumab in 0.5 mL and 90 mg of ustekinumab in 1 mL, supplied as a sterile solution in a single-dose prefilled syringe with a 27 gauge fixed ½ inch needle and as 45 mg of ustekinumab in 0.5 mL in a single-dose 2 mL Type I glass vial with a coated stopper. The syringe is fitted with a passive needle guard and a needle cover that contains dry natural rubber (a derivative of latex).

Each 0.5 mL prefilled syringe or vial delivers 45 mg ustekinumab, L-histidine and L-histidine monohydrochloride monohydrate (0.5 mg), Polysorbate 80 (0.02 mg), and sucrose (38 mg).

Each 1 mL prefilled syringe delivers 90 mg ustekinumab, L-histidine and L-histidine monohydrochloride monohydrate (1 mg), Polysorbate 80 (0.04 mg), and sucrose (76 mg).

### STELARA^{®} for Intravenous Infusion

Available as 130 mg of ustekinumab in 26 mL, supplied as a single-dose 30 mL Type I glass vial with a coated stopper.

Each 26 mL vial delivers 130 mg ustekinumab, EDTA disodium salt dihydrate (0.52 mg), L-histidine (20 mg), L-histidine hydrochloride monohydrate (27 mg), L-methionine (10.4 mg), Polysorbate 80 (10.4 mg) and sucrose (2210 mg).

### 12 CLINICAL PHARMACOLOGY

### 12.1 Mechanism of Action

Ustekinumab is a human IgG1κ monoclonal antibody that binds with specificity to the p40 protein subunit used by both the IL-12 and IL-23 cytokines. IL-12 and IL-23 are naturally occurring cytokines that are involved in inflammatory and immune responses, such as natural killer cell activation and CD4+ T-cell differentiation and activation. In *in vitro* models, ustekinumab was shown to disrupt IL-12 and IL-23 mediated signaling and cytokine cascades by disrupting the interaction of these cytokines with a shared cell-surface receptor chain, IL-12Rβ1. The cytokines IL-12 and IL-23 have been implicated as important contributors to the chronic inflammation that is a hallmark of Crohn's disease and ulcerative colitis. In animal models of colitis, genetic absence or antibody blockade of the p40 subunit of IL-12 and IL-23, the target of ustekinumab, was shown to be protective.

### 12.2 Pharmacodynamics

### Psoriasis

In a small exploratory study, a decrease was observed in the expression of mRNA of its molecular targets IL-12 and IL-23 in lesional skin biopsies measured at baseline and up to two weeks post-treatment in subjects with psoriasis.

### Ulcerative Colitis

In both study UC-1 (induction) and study UC-2 (maintenance), a positive relationship was observed between exposure and rates of clinical remission, clinical response, and endoscopic improvement. The response rate approached a plateau at the ustekinumab exposures associated with the recommended dosing regimen for maintenance treatment *[see Clinical Studies (14.5)].*

### 12.3 Pharmacokinetics

### Absorption

In adult subjects with psoriasis, the median time to reach the maximum serum concentration (Tₘₐₓ) was 13.5 days and 7 days, respectively, after a single subcutaneous administration of 45 mg (N=22) and 90 mg (N=24) of ustekinumab. In healthy subjects (N=30), the median Tₘₐₓ value (8.5 days) following a single subcutaneous administration of 90 mg of ustekinumab was comparable to that observed in subjects with psoriasis.

Following multiple subcutaneous doses of STELARA^{®} in adult subjects with psoriasis, steady-state serum concentrations of ustekinumab were achieved by Week 28. The mean (±SD) steady-state trough serum ustekinumab concentrations were 0.69 ± 0.69 mcg/mL for patients less than or equal to 100 kg receiving a 45 mg dose and 0.74 ± 0.78 mcg/mL for patients greater than 100 kg receiving a 90 mg dose. There was no apparent accumulation in serum ustekinumab concentration over time when given subcutaneously every 12 weeks.

Following the recommended intravenous induction dose, mean ±SD peak serum ustekinumab concentration was 125.2 ± 33.6 mcg/mL in patients with Crohn's disease, and 129.1 ± 27.6 mcg/mL in patients with ulcerative colitis. Starting at Week 8, the recommended subcutaneous maintenance dosing of 90 mg ustekinumab was administered every 8 weeks. Steady state ustekinumab concentration was achieved by the start of the second maintenance dose. There was no apparent accumulation in ustekinumab concentration over time when given subcutaneously every 8 weeks. Mean ±SD steady-state trough concentration was 2.5 ± 2.1 mcg/mL in patients with Crohn's disease, and 3.3 ± 2.3 mcg/mL in patients with ulcerative colitis for 90 mg ustekinumab administered every 8 weeks.

### Distribution

Population pharmacokinetic analyses showed that the volume of distribution of ustekinumab in the central compartment was 2.7 L (95% CI: 2.69, 2.78) in patients with Crohn's disease and 3.0 L (95% CI: 2.96, 3.07) in patients with ulcerative colitis. The total volume of distribution at steady-state was 4.6 L in patients with Crohn's disease and 4.4 L in patients with ulcerative colitis.

### Elimination

The mean (±SD) half-life ranged from 14.9 ± 4.6 to 45.6 ± 80.2 days across all psoriasis studies following subcutaneous administration. Population pharmacokinetic analyses showed that the clearance of ustekinumab was 0.19 L/day (95% CI: 0.185, 0.197) in patients with Crohn's disease and 0.19 L/day (95% CI: 0.179, 0.192) in patients with ulcerative colitis with an estimated median terminal half-life of approximately 19 days for both IBD (Crohn's disease and ulcerative colitis) populations.

These results indicate the pharmacokinetics of ustekinumab were similar between patients with Crohn's disease and ulcerative colitis.

### Metabolism

The metabolic pathway of ustekinumab has not been characterized. As a human IgG1κ monoclonal antibody, ustekinumab is expected to be degraded into small peptides and amino acids via catabolic pathways in the same manner as endogenous IgG.

### Specific Populations

### • Weight

When given the same dose, subjects with psoriasis or psoriatic arthritis weighing more than 100 kg had lower median serum ustekinumab concentrations compared with those subjects weighing 100 kg or less. The median trough serum concentrations of ustekinumab in subjects of higher weight (greater than 100 kg) in the 90 mg group were comparable to those in subjects of lower weight (100 kg or less) in the 45 mg group.

### • Age: Geriatric Population

A population pharmacokinetic analysis (N=106/1937 patients with psoriasis greater than or equal to 65 years old) was performed to evaluate the effect of age on the pharmacokinetics of ustekinumab. There were no apparent changes in pharmacokinetic parameters (clearance and volume of distribution) in subjects older than 65 years old.

### • Age: Pediatric Population

Following multiple recommended doses of STELARA^{®} in adolescent subjects 12 to 17 years of age with psoriasis, steady-state serum concentrations of ustekinumab were achieved by Week 28. At Week 28, the mean ±SD steady-state trough serum ustekinumab concentration was 0.54 ± 0.43 mcg/mL.

### • Drug Interaction Studies

The effects of IL-12 or IL-23 on the regulation of CYP450 enzymes were evaluated in an *in vitro* study using human hepatocytes, which showed that IL-12 and/or IL-23 at levels of 10 ng/mL did not alter human CYP450 enzyme activities (CYP1A2, 2B6, 2C9, 2C19, 2D6, or 3A4). However, the clinical relevance of *in vitro* data has not been established *[see Drug Interactions (7.3)].*

No *in vivo* drug interaction studies have been conducted with STELARA^{®}.

Population pharmacokinetic analyses indicated that the clearance of ustekinumab was not impacted by concomitant MTX, NSAIDs, and oral corticosteroids, or prior exposure to a TNF blocker in patients with psoriatic arthritis.

In patients with Crohn's disease and ulcerative colitis, population pharmacokinetic analyses did not indicate changes in ustekinumab clearance with concomitant use of corticosteroids or immunomodulators (AZA, 6-MP, or MTX); and serum ustekinumab concentrations were not impacted by concomitant use of these medications.

### 13 NONCLINICAL TOXICOLOGY

### 13.1 Carcinogenesis, Mutagenesis, Impairment of Fertility

Animal studies have not been conducted to evaluate the carcinogenic or mutagenic potential of STELARA^{®}. Published literature showed that administration of murine IL-12 caused an anti-tumor effect in mice that contained transplanted tumors and IL-12/IL-23p40 knockout mice or mice treated with anti-IL-12/IL-23p40 antibody had decreased host defense to tumors. Mice genetically manipulated to be deficient in both IL-12 and IL-23 or IL-12 alone developed UV-induced skin cancers earlier and more frequently compared to wild-type mice. The relevance of these experimental findings in mouse models for malignancy risk in humans is unknown.

No effects on fertility were observed in male cynomolgus monkeys that were administered ustekinumab at subcutaneous doses up to 45 mg/kg twice weekly (45 times the MRHD on a mg/kg basis) prior to and during the mating period. However, fertility and pregnancy outcomes were not evaluated in mated females.

No effects on fertility were observed in female mice that were administered an analogous IL-12/IL-23p40 antibody by subcutaneous administration at doses up to 50 mg/kg, twice weekly, prior to and during early pregnancy.

### 13.2 Animal Toxicology and/or Pharmacology

In a 26-week toxicology study, one out of 10 monkeys subcutaneously administered 45 mg/kg ustekinumab twice weekly for 26 weeks had a bacterial infection.

### 14 CLINICAL STUDIES

### 14.1 Psoriasis

Two multicenter, randomized, double-blind, placebo-controlled studies (Ps STUDY 1 and Ps STUDY 2) enrolled a total of 1996 subjects 18 years of age and older with plaque psoriasis who had a minimum body surface area involvement of 10%, and Psoriasis Area and Severity Index (PASI) score ≥12, and who were candidates for phototherapy or systemic therapy. Subjects with guttate, erythrodermic, or pustular psoriasis were excluded from the studies.

Ps STUDY 1 enrolled 766 subjects and Ps STUDY 2 enrolled 1230 subjects. The studies had the same design through Week 28. In both studies, subjects were randomized in equal proportion to placebo, 45 mg or 90 mg of STELARA^{®}. Subjects randomized to STELARA^{®} received 45 mg or 90 mg doses, regardless of weight, at Weeks 0, 4, and 16. Subjects randomized to receive placebo at Weeks 0 and 4 crossed over to receive STELARA^{®} (either 45 mg or 90 mg) at Weeks 12 and 16.

In both studies, the endpoints were the proportion of subjects who achieved at least a 75% reduction in PASI score (PASI 75) from baseline to Week 12 and treatment success (cleared or minimal) on the Physician's Global Assessment (PGA). The PGA is a 6-category scale ranging from 0 (cleared) to 5 (severe) that indicates the physician's overall assessment of psoriasis focusing on plaque thickness/induration, erythema, and scaling.

In both studies, subjects in all treatment groups had a median baseline PASI score ranging from approximately 17 to 18. Baseline PGA score was marked or severe in 44% of subjects in Ps STUDY 1 and 40% of subjects in Ps STUDY 2. Approximately two-thirds of all subjects had received prior phototherapy, 69% had received either prior conventional systemic or biologic therapy for the treatment of psoriasis, with 56% receiving prior conventional systemic therapy and 43% receiving prior biologic therapy. A total of 28% of subjects had a history of psoriatic arthritis.

### Clinical Response

The results of Ps STUDY 1 and Ps STUDY 2 are presented in Label Table 7 below.

**Label Table 7: Clinical Outcomes Ps STUDY 1 and Ps STUDY 2**

| **Week 12** | **Ps STUDY 1** | | | **Ps STUDY 2** | | |
|---|---|---|---|---|---|---|
| | **STELARA^{®}** | | | **STELARA^{®}** | | |
| | **Placebo** | **45 mg** | **90 mg** | **Placebo** | **45 mg** | **90 mg** |
| **Subjects randomized** | **255** | **255** | **256** | **410** | **409** | **411** |
| PASI 75 response | 8 (3%) | 171 (67%) | 170 (66%) | 15 (4%) | 273 (67%) | 311 (76%) |
| PGA of Cleared or Minimal | 10 (4%) | 151 (59%) | 156 (61%) | 18 (4%) | 277 (68%) | 300 (73%) |

Examination of age, gender, and race subgroups did not identify differences in response to STELARA^{®} among these subgroups.

In subjects who weighed 100 kg or less, response rates were similar with both the 45 mg and 90 mg doses; however, in subjects who weighed greater than 100 kg, higher response rates were seen with 90 mg dosing compared with 45 mg dosing (Label Table 8 below).

**Label Table 8: Clinical Outcomes by Weight Ps STUDY 1 and Ps STUDY 2**

| | **Ps STUDY 1** | | | **Ps STUDY 2** | | |
|---|---|---|---|---|---|---|
| | | **STELARA^{®}** | | | **STELARA^{®}** | |
| | **Placebo** | **45 mg** | **90 mg** | **Placebo** | **45 mg** | **90 mg** |
| **Subjects randomized** | **255** | **255** | **256** | **410** | **409** | **411** |

| **PASI 75 response at Week 12*** | | | | | | |
|---|---|---|---|---|---|---|
| ≤100 kg | 4% | 74% | 65% | 4% | 73% | 78% |
| | 6/166 | 124/168 | 107/164 | 12/290 | 218/297 | 225/289 |
| >100 kg | 2% | 54% | 68% | 3% | 49% | 71% |
| | 2/89 | 47/87 | 63/92 | 3/120 | 55/112 | 86/121 |

| **PGA of Cleared or Minimal at Week 12*** | | | | | | |
|---|---|---|---|---|---|---|
| ≤100 kg | 4% | 64% | 63% | 5% | 74% | 75% |
| | 7/166 | 108/168 | 103/164 | 14/290 | 220/297 | 216/289 |
| >100 kg | 3% | 49% | 58% | 3% | 51% | 69% |
| | 3/89 | 43/87 | 53/92 | 4/120 | 57/112 | 84/121 |

| | | | | | | |
|---|---|---|---|---|---|---|
| * Patients were dosed with study medication at Weeks 0 and 4. | | | | | | |

Subjects in Ps STUDY 1 who were PASI 75 responders at both Weeks 28 and 40 were re-randomized at Week 40 to either continued dosing of STELARA^{®} (STELARA^{®} at Week 40) or to withdrawal of therapy (placebo at Week 40). At Week 52, 89% (144/162) of subjects re-randomized to STELARA^{®} treatment were PASI 75 responders compared with 63% (100/159) of subjects re-randomized to placebo (treatment withdrawal after Week 28 dose). The median time to loss of PASI 75 response among the subjects randomized to treatment withdrawal was 16 weeks.

### 14.2 Adolescent Subjects with Plaque Psoriasis

A multicenter, randomized, double blind, placebo-controlled study (Ps STUDY 3) enrolled 110 adolescent subjects 12 to 17 years of age with a minimum BSA involvement of 10%, a PASI score greater than or equal to 12, and a PGA score greater than or equal to 3, who were candidates for phototherapy or systemic therapy and whose disease was inadequately controlled by topical therapy.

Subjects were randomized to receive placebo (n = 37), the recommended dose of STELARA^{®} (n = 36), or one-half the recommended dose of STELARA^{®} (n = 37) by subcutaneous injection at Weeks 0 and 4 followed by dosing every 12 weeks (q12w). The recommended dose of STELARA^{®} was 0.75 mg/kg for subjects weighing less than 60 kg, 45 mg for subjects weighing 60 kg to 100 kg, and 90 mg for subjects weighing greater than 100 kg. At Week 12, subjects who received placebo were crossed over to receive STELARA^{®} at the recommended dose or one-half the recommended dose.

Of the adolescent subjects, approximately 63% had prior exposure to phototherapy or conventional systemic therapy and approximately 11% had prior exposure to biologics.

The endpoints were the proportion of patients who achieved a PGA score of cleared (0) or minimal (1), PASI 75, and PASI 90 at Week 12. Subjects were followed for up to 60 weeks following first administration of study agent.

### Clinical Response

The efficacy results at Week 12 for Ps STUDY 3 are presented in Label Table 9.

**Label Table 9: Summary of Efficacy Endpoints in the Adolescent Psoriasis Study at Week 12**

| | **Ps STUDY 3** | |
|---|---|---|
| | **Placebo** | **STELARA^{®}*** |
| | **n (%)** | **n (%)** |
| **N** | **37** | **36** |

| **PGA** | | |
|---|---|---|
| PGA of cleared (0) or minimal (1) | 2 (5.4%) | 25 (69.4%) |

| **PASI** | | |
|---|---|---|
| PASI 75 responders | 4 (10.8%) | 29 (80.6%) |
| PASI 90 responders | 2 (5.4%) | 22 (61.1%) |

| | | |
|---|---|---|
| * Using the weight-based dosage regimen specified in Label Table 1 and Label Table 2. | | |

### 14.3 Psoriatic Arthritis

The safety and efficacy of STELARA^{®} was assessed in 927 patients (PsA STUDY 1, n=615; PsA STUDY 2, n=312), in two randomized, double-blind, placebo-controlled studies in adult patients 18 years of age and older with active PsA (≥5 swollen joints and ≥5 tender joints) despite non-steroidal anti-inflammatory (NSAID) or disease modifying antirheumatic (DMARD) therapy. Patients in these studies had a diagnosis of PsA for at least 6 months. Patients with each subtype of PsA were enrolled, including polyarticular arthritis with the absence of rheumatoid nodules (39%), spondylitis with peripheral arthritis (28%), asymmetric peripheral arthritis (21%), distal interphalangeal involvement (12%) and arthritis mutilans (0.5%). Over 70% and 40% of the patients, respectively, had enthesitis and dactylitis at baseline.

Patients were randomized to receive treatment with STELARA^{®} 45 mg, 90 mg, or placebo subcutaneously at Weeks 0 and 4 followed by every 12 weeks (q12w) dosing. Approximately 50% of patients continued on stable doses of MTX (≤25 mg/week). The primary endpoint was the percentage of patients achieving ACR 20 response at Week 24.

In PsA STUDY 1 and PsA STUDY 2, 80% and 86% of the patients, respectively, had been previously treated with DMARDs. In PsA STUDY 1, previous treatment with anti-tumor necrosis factor (TNF)-α agent was not allowed. In PsA STUDY 2, 58% (n=180) of the patients had been previously treated with TNF blocker, of whom over 70% had discontinued their TNF blocker treatment for lack of efficacy or intolerance at any time.

### Clinical Response

In both studies, a greater proportion of patients achieved ACR 20, ACR 50 and PASI 75 response in the STELARA^{®} 45 mg and 90 mg groups compared to placebo at Week 24 (see Label Table 10). ACR 70 responses were also higher in the STELARA^{®} 45 mg and 90 mg groups, although the difference was only numerical (p=NS) in STUDY 2. Responses were similar in patients regardless of prior TNFα exposure.

**Label Table 10: ACR 20, ACR 50, ACR 70 and PASI 75 responses in PsA STUDY 1 and PsA STUDY 2 at Week 24**

| | **PsA STUDY 1** | | | **PsA STUDY 2** | | |
|---|---|---|---|---|---|---|
| | | **STELARA^{®}** | | | **STELARA^{®}** | |
| | **Placebo** | **45 mg** | **90 mg** | **Placebo** | **45 mg** | **90 mg** |
| **Number of patients randomized** | **206** | **205** | **204** | **104** | **103** | **105** |
| ACR 20 response, N (%) | 47 (23%) | 87 (42%) | 101 (50%) | 21 (20%) | 45 (44%) | 46 (44%) |
| ACR 50 response, N (%) | 18 (9%) | 51 (25%) | 57 (28%) | 7 (7%) | 18 (17%) | 24 (23%) |
| ACR 70 response, N (%) | 5 (2%) | 25 (12%) | 29 (14%) | 3 (3%) | 7 (7%) | 9 (9%) |
| **Number of patients with ≥ 3% BSA^{a}** | **146** | **145** | **149** | **80** | **80** | **81** |
| PASI 75 response, N (%) | 16 (11%) | 83 (57%) | 93 (62%) | 4 (5%) | 41 (51%) | 45 (56%) |

| | | | | | | |
|---|---|---|---|---|---|---|
| ^{a} Number of patients with ≥ 3% BSA psonasis skin involvement at baseline | | | | | | |

The percent of patients achieving ACR 20 responses by visit is shown in Label Figure 1.

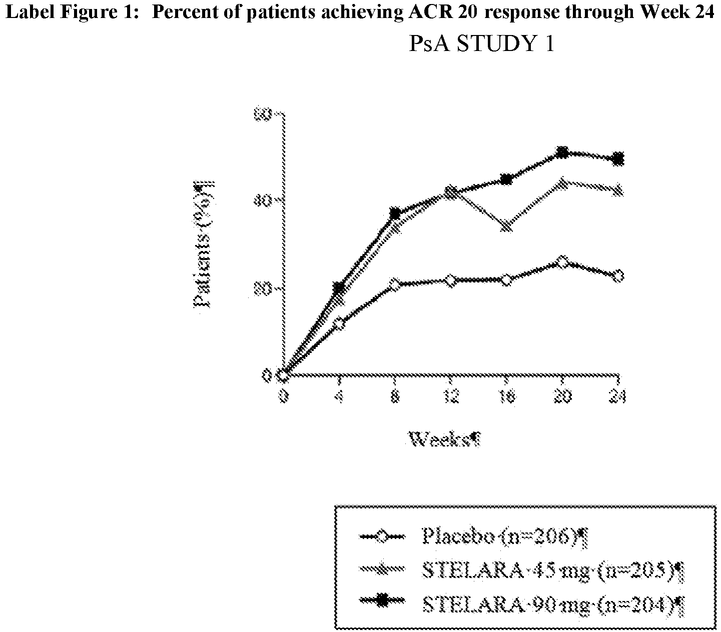

The results of the components of the ACR response criteria are shown in Label Table 11.

**Label Table 11: Mean change from baseline in ACR components at Week 24**

| | | **PsA STUDY 1** | | |
|---|---|---|---|---|
| | | | **STELARA^{®}** | |
| | | **Placebo (N=206)** | **45 mg (N= 205)** | **90 mg (N= 204)** |
| Number of swollen joints^{a} | | | | |
| | Baseline | 15 | 12 | 13 |
| | Mean Change at Week 24 | -3 | -5 | -6 |

| Number of tender joints^{b} | | | | |
|---|---|---|---|---|
| | Baseline | 25 | 22 | 23 |
| | Mean Change at Week 24 | -4 | -8 | -9 |

| Patient's assessment of pain^{c} | | | | |
|---|---|---|---|---|
| | Baseline | 6.1 | 6.2 | 6.6 |
| | Mean Change at Week 24 | -0.5 | -2.0 | -2.6 |

| Patient global assessment^{c} | | | | |
|---|---|---|---|---|
| | Baseline | 6.1 | 6.3 | 6.4 |
| | Mean Change at Week 24 | -0.5 | -2.0 | -2.5 |
| Physician global assessment^{c} | | | | |
| | Baseline | 5.8 | 5.7 | 6.1 |
| | Mean Change at Week 24 | -1.4 | -2.6 | -3.1 |
| Disability index (HAQ)^{d} | | | | |
| | Baseline | 1.2 | 1.2 | 1.2 |
| | Mean Change at Week 24 | -0.1 | -0.3 | -0.4 |
| CRP (mg/dL)^{e} | | | | |
| | Baseline | 1.6 | 1.7 | 1.8 |
| | Mean Change at Week 24 | 0.01 | -0.5 | -0.8 |

| | | | | |
|---|---|---|---|---|
| ^{a} Number of swollen joints counted (0-66) ^{b} Number of tender joints counted (0-68) ^{c} Visual analogue scale, 0= best, 10=worst. ^{d} Disability Index of the Health Assessment Questionnaire, 0 -- best, 3 -- worst, measures the patient's ability to perform the following dress/groom, arise, eat, walk, reach, gnp, maintain hygiene, and maintain daily activity. ^{e} CRP (Normal Range 0.0-1.0 mg/dL) | | | | |

An improvement in enthesitis and dactylitis scores was observed in each STELARA^{®} group compared with placebo at Week 24.

### Physical Function

STELARA^{®} treated patients showed improvement in physical function compared to patients treated with placebo as assessed by HAQ-DI at Week 24. In both studies, the proportion of HAQ-DI responders (≥0.3 improvement in HAQ-DI score) was greater in the STELARA^{®} 45 mg and 90 mg groups compared to placebo at Week 24.

### 14.4 Crohn's Disease

STELARA^{®} was evaluated in three randomized, double-blind, placebo-controlled clinical studies in adult patients with moderately to severely active Crohn's disease (Crohn's Disease Activity Index [CDAI] score of 220 to 450). There were two 8-week intravenous induction studies (CD-1 and CD-2) followed by a 44-week subcutaneous randomized withdrawal maintenance study (CD-3) representing 52 weeks of therapy. Patients in CD-1 had failed or were intolerant to treatment with one or more TNF blockers, while patients in CD-2 had failed or were intolerant to treatment with immunomodulators or corticosteroids, but never failed treatment with a TNF blocker.

### Studies CD-1 and CD-2

In studies CD-1 and CD-2, 1409 patients were randomized, of whom 1368 (CD-1, n=741; CD-2, n=627) were included in the final efficacy analysis. Induction of clinical response (defined as a reduction in CDAI score of greater than or equal to 100 points or CDAI score of less than 150) at Week 6 and clinical remission (defined as a CDAI score of less than 150) at Week 8 were evaluated. In both studies, patients were randomized to receive a single intravenous administration of STELARA^{®} at either approximately 6 mg/kg, placebo (see Label Table 3), or 130 mg (a lower dose than recommended).

In Study CD-1, patients had failed or were intolerant to prior treatment with a TNF blocker: 29% patients had an inadequate initial response (primary non-responders), 69% responded but subsequently lost response (secondary non-responders) and 36% were intolerant to a TNF blocker. Of these patients, 48% failed or were intolerant to one TNF blocker and 52% had failed 2 or 3 prior TNF blockers. At baseline and throughout the study, approximately 46% of the patients were receiving corticosteroids and 31% of the patients were receiving immunomodulators (AZA, 6-MP, MTX). The median baseline CDAI score was 319 in the STELARA^{®} approximately 6 mg/kg group and 313 in the placebo group.

In Study CD-2, patients had failed or were intolerant to prior treatment with corticosteroids (81% of patients), at least one immunomodulator (6-MP, AZA, MTX; 68% of patients), or both (49% of patients). Additionally, 69% never received a TNF blocker and 31% previously received but had not failed a TNF blocker. At baseline, and throughout the study, approximately 39% of the patients were receiving corticosteroids and 35% of the patients were receiving immunomodulators (AZA, 6-MP, MTX). The median baseline CDAI score was 286 in the STELARA^{®} and 290 in the placebo group.

In these induction studies, a greater proportion of patients treated with STELARA^{®} (at the recommended dose of approximately 6 mg/kg dose) achieved clinical response at Week 6 and clinical remission at Week 8 compared to placebo (see Label Table 12 for clinical response and remission rates). Clinical response and remission were significant as early as Week 3 in STELARA^{®}-treated patients and continued to improve through Week 8.

**Label Table 12: Induction of Clinical Response and Remission in CD-1* and CD-2****

| | **CD-1 n=741** | | | **CD-2 n=627** | | |
|---|---|---|---|---|---|---|
| | **Placebo N=247** | **STELARA ^{®}† N=249** | **Treatment difference and 95% CI** | **Placebo N=209** | **STELARA ^{®}† N=209** | **Treatment difference and 95% CI** |
| Clinical Response (100 point), | 53 (21%) | 84 (34%)^{a} | 12% (4%, 20%) | 60 (29%) | 116 (56%)^{b} | 27% (18%, 36%) |
| Week 6 Clinical Remission, Week 8 | 18 (7%) | 52 (21%)^{b} | 14% (8%, 20%) | 41 (20%) | 84 (40%)^{b} | 21% (12%, 29%) |
| Clinical Response (100 point), Week 8 | 50 (20%) | 94 (38%)^{b} | 18% (10%, 25%) | 67 (32%) | 121 (58%)^{b} | 26% (17%, 35%) |
| 70 Point Response, Week 6 | 75 (30%) | 109 (44%)^{a} | 13% (5%, 22%) | 81 (39%) | 135 (65%)^{b} | 26% (17%, 35%) |
| 70 Point Response, Week 3 | 67 (27%) | 101 (41%)^{a} | 13% (5%, 22%) | 66 (32%) | 106 (51%)^{b} | 19% (10%, 28%) |

| | | | | | | |
|---|---|---|---|---|---|---|
| Clinical remission is defined as CDAI score < 150; Clinical response is defined as reduction in CDAI score by at least 100 points or being in clinical remission 70 point response is defined as reduction in CDAI score by at least 70 points ^{*} Patient population consisted of patients who failed or were intolerant to TNF blocker therapy ^{**} Patient population consisted of patients who failed or were intolerant to corticosteroids or immunomodulators (e.g., 6-MP, AZA, MTX) and previously received but not failed a TNF blocker or were never treated with a TNF blocker. ^{†} Infusion dose of STELARA^{®} using the weight-based dosage regimen specified in Label Table 3. ^{a} 0.001≤p < 0.01 ^{b} p < 0.001 | | | | | | |

### Study CD-3

The maintenance study (CD-3), evaluated 388 patients who achieved clinical response (≥100 point reduction in CDAI score) at Week 8 with either induction dose of STELARA^{®} in studies CD-1 or CD-2. Patients were randomized to receive a subcutaneous maintenance regimen of either 90 mg STELARA^{®} every 8 weeks or placebo for 44 weeks (see Label Table 13).

**Label Table 13: Clinical Response and Remission in CD-3 (Week 44; 52 weeks from initiation of the induction dose)**

| | **Placebo* N=131**^{†} | **90 mg STELARA^{®} every 8 weeks N=128^{†}** | **Treatment difference and 95% CI** |
|---|---|---|---|
| Clinical Remission | 47 (36%) | 68 (53%)^{a} | 17% (5%, 29%) |
| Clinical Response | 58 (44%) | 76 (59%)^{b} | 15% (3%, 27%) |
| Clinical Remission in patients in remission at the start of maintenance therapy** | 36/79 (46%) | 52/78 (67%)^{a} | 21% (6%, 36%) |

| | | | |
|---|---|---|---|
| Clinical remission is defined as CDAI score < 150; Clinical response is defined as reduction in CDAI of at least 100 points or being in clinical remission ^{*} The placebo group consisted of patients who were in response to STELARA^{®} and were randomized to receive placebo at the start of maintenance therapy. ^{**} Patients in remission at the end of maintenance therapy who were in remission at the start of maintenance therapy. This does not account for any other time point during maintenance therapy. ^{†} Patients who achieved clinical response to STELARA^{®} at the end of the induction study. ^{a} p < 0.01 ^{b} 0.01≤p<0.05 | | | |

At Week 44, 47% of patients who received STELARA^{®} were corticosteroid-free and in clinical remission, compared to 30% of patients in the placebo group.

At Week 0 of Study CD-3, 34/56 (61%) STELARA^{®} treated patients who previously failed or were intolerant to TNF blocker therapies were in clinical remission and 23/56 (41%) of these patients were in clinical remission at Week 44. In the placebo arm, 27/61 (44%) patients were in clinical remission at Week 0 while 16/61 (26%) of these patients were in remission at Week 44.

At Week 0 of Study CD-3, 46/72 (64%) STELARA^{®} treated patients who had previously failed immunomodulator therapy or corticosteroids (but not TNF blockers) were in clinical remission and 45/72 (63%) of these patients were in clinical remission at Week 44. In the placebo arm, 50/70 (71%) of these patients were in clinical remission at Week 0 while 31/70 (44%) were in remission at Week 44. In the subset of these patients who were also naive to TNF blockers, 34/52 (65%) of STELARA^{®} treated patients were in clinical remission at Week 44 as compared to 25/51 (49%) in the placebo arm.

Patients who were not in clinical response 8 weeks after STELARA^{®} induction were not included in the primary efficacy analyses for Study CD-3; however, these patients were eligible to receive a 90 mg subcutaneous injection of STELARA^{®} upon entry into Study CD-3. Of these patients, 102/219 (47%) achieved clinical response eight weeks later and were followed for the duration of the study.

### 14.5 Ulcerative Colitis

STELARA^{®} was evaluated in two randomized, double-blind, placebo-controlled clinical studies [UC-1 and UC-2 (NCT02407236)] in adult patients with moderately to severely active ulcerative colitis who had an inadequate response to or failed to tolerate a biologic (i.e., TNF blocker and/or vedolizumab), corticosteroids, and/or 6-MP or AZA therapy. The 8-week intravenous induction study (UC-1) was followed by the 44-week subcutaneous randomized withdrawal maintenance study (UC-2) for a total of 52 weeks of therapy.

Disease assessment was based on the Mayo score, which ranged from 0 to 12 and has four subscores that were each scored from 0 (normal) to 3 (most severe): stool frequency, rectal bleeding, findings on centrally-reviewed endoscopy, and physician global assessment. Moderately to severely active ulcerative colitis was defined at baseline (Week 0) as Mayo score of 6 to 12, including a Mayo endoscopy subscore ≥2. An endoscopy score of 2 was defined by marked erythema, absent vascular pattern, friability, erosions; and a score of 3 was defined by spontaneous bleeding, ulceration. At baseline, patients had a median Mayo score of 9, with 84% of patients having moderate disease (Mayo score 6-10) and 15% having severe disease (Mayo score 11-12).

Patients in these studies may have received other concomitant therapies including aminosalicylates, immunomodulatory agents (AZA, 6-MP, or MTX), and oral corticosteroids (prednisone).

### Study UC-1

In UC-1, 961 patients were randomized at Week 0 to a single intravenous administration of STELARA^{®} of approximately 6 mg/kg, 130 mg (a lower dose than recommended), or placebo. Patients enrolled in UC-1 had to have failed therapy with corticosteroids, immunomodulators or at least one biologic. A total of 51% had failed at least one biologic and 17% had failed both a TNF blocker and an integrin receptor blocker. Of the total population, 46% had failed corticosteroids or immunomodulators but were biologic-naive and an additional 3% had previously received but had not failed a biologic. At induction baseline and throughout the study, approximately 52% patients were receiving oral corticosteroids, 28% patients were receiving immunomodulators (AZA, 6-MP, or MTX) and 69% patients were receiving aminosalicylates.

The primary endpoint was clinical remission at Week 8. Clinical remission with a definition of: Mayo stool frequency subscore of 0 or 1, Mayo rectal bleeding subscore of 0 (no rectal bleeding), and Mayo endoscopy subscore of 0 or 1 (Mayo endoscopy subscore of 0 defined as normal or inactive disease and Mayo subscore of 1 defined as presence of erythema, decreased vascular pattern and no friability) is provided in Label Table 14.

The secondary endpoints were clinical response, endoscopic improvement, and histolologic-endoscopic mucosal improvement. Clinical response with a definition of (≥ 2 points and ≥ 30% decrease in modified Mayo score, defined as 3-component Mayo score without the Physician's Global Assessment, with either a decrease from baseline in the rectal bleeding subscore ≥1 or a rectal bleeding subscore of 0 or 1), endoscopic improvement with a definition of Mayo endoscopy subscore of 0 or 1, and histologic-endoscopic mucosal improvement with a definition of combined endoscopic improvement and histologic improvement of the colon tissue [neutrophil infiltration in <5% of crypts, no crypt destruction, and no erosions, ulcerations, or granulation tissue]) are provided in Label Table 14.

In UC-1, a significantly greater proportion of patients treated with STELARA^{®} (at the recommended dose of approximately 6 mg/kg dose) were in clinical remission and response and achieved endoscopic improvement and histologic-endoscopic mucosal improvement compared to placebo (see Label Table 14).

**Label Table 14: Proportion of Patients Meeting Efficacy Endpoints at Week 8 in UC-1**

| **Endpoint** | | **Placebo** N = 319 | | **STELARA^{®†}** N = 322 | | **Treatment difference and 97.5% CI^{a}** |
|---|---|---|---|---|---|---|
| | | **N** | **%** | **N** | **%** | |
| **Clinical Remission*** | | **22** | **7%** | **62** | **19%** | 12% (7%, 18%)^{b} |
| | Bio-naïve 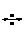 | 14/151 | 9% | 36/147 | 24% | |
| | Prior biologic failure | 7/161 | 4% | 24/166 | 14% | |
| **Endoscopic Improvement^{§}** | | **40** | **13%** | **80** | **25%** | 12% (6%, 19%) ^{b} |
| | Bio-naïve 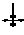 | 28/151 | 19% | 43/147 | 29% | |
| | Prior biologic failure | 11/161 | 7% | 34/166 | 20% | |
| **Clinical Response^{†}** | | **99** | **31%** | **186** | **58%** | 27% (18%, 35%) ^{b} |
| | Bio-naïve 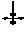 | 55/151 | 36% | 94/147 | 64% | |
| | Prior biologic failure | 42/161 | 26% | 86/166 | 52% | |
| **Histologic-Endoscopic Mucosal Improvement** | | **26** | **8%** | **54** | **17%** | 9% (3%, 14%) ^{b} |
| | Bio-naïve 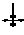 | 19/151 | 13% | 30/147 | 20% | |
| | Prior biologic failure | 6/161 | 4% | 21/166 | 13% | |

| | | | | | | |
|---|---|---|---|---|---|---|
| ^{†}Infusion dose of STELARA^{®} using the weight-based dosage regimen specified in Label Table 3. 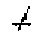 An additional 7 patients on placebo and 9 patients on STELARA^{®} (6 mg/kg) had been exposed to, but had not failed, biologics. ^{*}Clinical remission was defined as Mayo stool frequency subscore of 0 or 1, Mayo rectal bleeding subscore of 0, and Mayo endoscopy subscore of 0 or 1 (modified so that 1 does not include friability). ^{§} Endoscopic improvement was defined as Mayo endoscopy subscore of 0 or 1 (modified so that 1 does not include friability). ^{†} Clinical response was defined as a decrease from baseline in the modified Mayo score by ≥30% and ≥2 points, with either a decrease from baseline in the rectal bleeding subscore ≥1 or a rectal bleeding subscore of 0 or 1. ^{‡} Histologic-endoscopic mucosal improvement was defined as combined endoscopic improvement (Mayo endoscopy subscore of 0 or 1) and histologic improvement of the colon tissue (neutrophil infiltration in <5% of crypts, no crypt destruction, and no erosions, ulcerations, or granulation tissue). ^{a} Adjusted treatment difference (97.5% Cl) ^{b} p < 0.001 | | | | | | |

The relationship of histologic-endoscopic mucosal improvement, as defined in UC-1, at Week 8 to disease progression and long-term outcomes was not evaluated during UC-1.

### Rectal Bleeding and Stool Frequency Subscores

Decreases in rectal bleeding and stool frequency subscores were observed as early as Week 2 in STELARA^{®} treated patients.

### Study UC-2

The maintenance study (UC-2) evaluated 523 patients who achieved clinical response 8 weeks following the intravenous administration of either induction dose of STELARA^{®} in UC-1. These patients were randomized to receive a subcutaneous maintenance regimen of either 90 mg STELARA^{®} every 8 weeks, or every 12 weeks (a lower dose than recommended), or placebo for 44 weeks.

The primary endpoint was the proportion of patients in clinical remission at Week 44. The secondary endpoints included the proportion of patients maintaining clinical response at Week 44, the proportion of patients with endoscopic improvement at Week 44, the proportion of patients with corticosteroid-free clinical remission at Week 44, and the proportion of patients maintaining clinical remission at Week 44 among patients who achieved clinical remission 8 weeks after induction.

Results of the primary and secondary endpoints at Week 44 in patients treated with STELARA^{®} at the recommended dosage (90 mg every 8 weeks) compared to the placebo are shown in Label Table 15.

**Label Table 15: Efficacy Endpoints of Maintenance at Week 44 in UC-2 (52 Weeks from Initiation of the Induction Dose)**

| **Endpoint** | | **Placebo^{*}** N = 175^{†} | | **90 mg STELARA^{®} every 8 weeks** N = 176 | | **Treatment difference and 95% CI** |
|---|---|---|---|---|---|---|
| | | **N** | **%** | **N** | **%** | |
| **Clinical Remission^{**}** | | **46** | **26%** | **79** | **45%** | 19% (9%, 28%)^{a} |
| | Bio-naïve 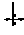 | 30/84 | 36% | 39/79 | 49% | |
| | Prior biologic failure | 16/88 | 18% | 37/91 | 41% | |
| **Maintenance of Clinical Response at Week 44^{†}** | | **84** | **48%** | **130** | **74%** | 26% (16%, 36%)^{a} |
| | Bio-naïve 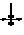 | 49/84 | 58% | 62/79 | 78% | |
| | Prior biologic failure | 35/88 | 40% | 64/91 | 70% | |
| **Endoscopic Improvement^{§}** | | **47** | **27%** | **83** | **47%** | 20% (11%, 30%)^{a} |
| | Bio-naïve 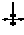 | 29/84 | 35% | 42/79 | 53% | |
| | Prior biologic failure | 18/88 | 20% | 38/91 | 42% | |
| **Corticosteroid-free Clinical Remission^{‡}** | | **45** | **26%** | **76** | **43%** | 17% (8%, 27%) ^{a} |
| | Bio-naïve 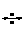 | 30/84 | 36% | 38/79 | 48% | |
| | Prior biologic failure | 15/88 | 17% | 35/91 | 38% | |
| **Maintenance of Clinical Remission at Week 44 in patients who achieved clinical remission 8 weeks after induction** | | **18/50** | **36%** | **27/41** | **66%** | 31% (12%, 50%)^{b} |
| | Bio-naïve 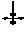 | 12/27 | 44% | 14/20 | 70% | |
| | Prior biologic failure | 6/23 | 26% | 12/18 | 67% | |

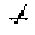 An additional 3 patients on placebo and 6 patients on STELARA^{®} had been exposed to, but had not failed, biologics.
^{*}The placebo group consisted of patients who were in response to STELARA^{®} and were randomized to receive placebo at the start of maintenance therapy.
^{**}Clinical remission was defined as Mayo stool frequency subscore of 0 or 1, Mayo rectal bleeding subscore of 0, and Mayo endoscopy subscore of 0 or 1 (modified so that 1 does not include friability).
^{†} Clinical response was defined as a decrease from baseline in the modified Mayo score by ≥30% and ≥2 points, with either a decrease from baseline in the rectal bleeding subscore ≥1 or a rectal bleeding subscore of 0 or 1.
^{§} Endoscopic improvement was defined as Mayo endoscopy subscore of 0 or 1 (modified so that 1 does not include friability).
^{‡} Corticosteroid-free clinical remission was defined as patients in clinical remission and not receiving corticosteroids at Week 44.
^{a} p =<0.001
^{b} p=0.004

### Other Endpoints

### Week 16 Responders to Ustekinumab Induction

Patients who were not in clinical response 8 weeks after induction with STELARA^{®} in UC-1 were not included in the primary efficacy analyses for Study UC-2; however, these patients were eligible to receive a 90 mg subcutaneous injection of STELARA^{®} at Week 8. Of these patients, 55/101 (54%) achieved clinical response eight weeks later (Week 16) and received STELARA^{®} 90 mg subcutaneously every 8 weeks during the UC-2 trial. At Week 44, there were 97/157 (62%) patients who maintained clinical response and there were 51/157 (32%) who achieved clinical remission.

### Histologic-Endoscopic Mucosal Improvement at Week 44

The proportion of patients achieving histologic-endoscopic mucosal improvement during maintenance treatment in UC-2 was 75/172 (44%) among patients on STELARA^{®} and 40/172 (23%) in patients on placebo at Week 44. The relationship of histologic-endoscopic mucosal improvement, as defined in UC-2, at Week 44 to progression of disease or long-term outcomes was not evaluated in UC-2.

### Endoscopic Normalization

Normalization of endoscopic appearance of the mucosa was defined as a Mayo endoscopic subscore of 0. At Week 8 in UC-1, endoscopic normalization was achieved in 25/322 (8%) of patients treated with STELARA and 12/319 (4%) of patients in the placebo group. At Week 44 of UC-2, endoscopic normalization was achieved in 51/176 (29%) of patients treated with STELARA^{®} and in 32/175 (18%) of patients in placebo group.

### 15 References

¹ Surveillance, Epidemiology, and End Results (SEER) Program (www.seer.cancer.gov) SEER* Stat Database: Incidence - SEER 6.6.2 Regs Research Data, Nov 2009 Sub (1973-2007) - Linked To County Attributes - Total U.S., 1969-2007 Counties, National Cancer Institute, DCCPS, Surveillance Research Program, Surveillance Systems Branch, released April 2010, based on the November 2009 submission.

### 16 HOW SUPPLIED/STORAGE AND HANDLING

STELARA^{®} (ustekinumab) Injection is a sterile, preservative-free, colorless to light yellow solution and may contain a few small translucent or white particles. It is supplied as individually packaged, single-dose prefilled syringes or single-dose vials.

For Subcutaneous Use

### • Prefilled Syringes

- 45 mg/0.5 mL (NDC 57894-060-03)
- 90 mg/mL (NDC 57894-061-03)

Each prefilled syringe is equipped with a 27 gauge fixed ½ inch needle, a needle safety guard, and a needle cover that contains dry natural rubber.

### • Single-dose Vial

- 45 mg/0.5 mL (NDC 57894-060-02)

For Intravenous Infusion

### • Single-dose Vial

- 130 mg/26 mL (5 mg/mL) (NDC 57894-054-27)

### Storage and Stability

STELARA^{®} vials and prefilled syringes must be refrigerated at 2°C to 8°C (36°F to 46°F). Store STELARA^{®} vials upright. Keep the product in the original carton to protect from light until the time of use. Do not freeze. Do not shake.

### 17 PATIENT COUNSELING INFORMATION

Advise the patient and/or caregiver to read the FDA-approved patient labeling (Medication Guide and Instructions for Use).

### Infections

Inform patients that STELARA^{®} may lower the ability of their immune system to fight infections and to contact their healthcare provider immediately if they develop any signs or symptoms of infection *[see Warnings and Precautions (5.1)].*

Malignancies

Inform patients of the risk of developing malignancies while receiving STELARA^{®} *[see Warnings and Precautions (5.4)].*

### Hypersensitivity Reactions

- Advise patients to seek immediate medical attention if they experience any signs or symptoms of serious hypersensitivity reactions and discontinue STELARA^{®} *[see Warnings and Precautions (5.5)].*
- Inform patients the needle cover on the prefilled syringe contains dry natural rubber (a derivative of latex), which may cause allergic reactions in individuals sensitive to latex *[see Dosage and Administration (2.4)]*

### Immunizations

Inform patients that STELARA^{®} can interfere with the usual response to immunizations and that they should avoid live vaccines *[see Warnings and Precautions (5.7)].*

### Pregnancy Registry

Inform patients that there is a pregnancy registry to monitor fetal outcomes of pregnant women exposed to STELARA^{®} *[see Use in Specific Populations (8.1)].*

### Administration

Instruct patients to follow sharps disposal recommendations, as described in the Instructions for Use.
Prefilled Syringe Manufactured by: Janssen Biotech, Inc., Horsham, PA 19044, US License No. 1864 at Baxter Pharmaceutical Solutions, Bloomington, IN 47403 and at Cilag AG, Schaffhausen, Switzerland
Vial Manufactured by: Janssen Biotech, Inc., Horsham, PA 19044, US License No. 1864 at Cilag AG, Schaffhausen, Switzerland
^{©} 2012, 2016, 2019 Janssen Pharmaceutical Companies

### LIST OF EMBODIMENTS

1. A pharmaceutical composition of an anti-IL-12/IL-23p40 antibody, comprising:
   A. an antibody comprising: (i) a heavy chain variable region and a light chain variable region, the heavy chain variable region comprising: a complementarity determining region heavy chain 1 (CDRH1) amino acid sequence of SEQ ID NO:1; a CDRH2 amino acid sequence of SEQ ID NO:2; and a CDRH3 amino acid sequence of SEQ ID NO:3; and the light chain variable region comprising: a complementarity determining region light chain 1 (CDRL1) amino acid sequence of SEQ ID NO:4; a CDRL2 amino acid sequence of SEQ ID NO:5; and a CDRL3 amino acid sequence of SEQ ID NO:6; (ii) a heavy chain variable region of the amino acid sequence of SEQ ID NO:7 and a light chain variable region of the amino acid sequence of SEQ ID NO:8; or (iii) a heavy chain of the amino acid sequence of SEQ ID NO:10 and a light chain of the amino acid sequence of SEQ ID NO:11; and
   B. packaging comprising one or more drug product label elements disclosed in Annex I including data from a randomized, double-blind, placebo-controlled, clinical study in adult men and women with moderately to severely active ulcerative colitis (UC).
2. A pharmaceutical composition of an anti-IL-12/IL-23p40 antibody, comprising:
   an antibody comprising: (i) a heavy chain variable region and a light chain variable region, the heavy chain variable region comprising: a complementarity determining region heavy chain 1 (CDRH1) amino acid sequence of SEQ ID NO:1; a CDRH2 amino acid sequence of SEQ ID NO:2; and a CDRH3 amino acid sequence of SEQ ID NO:3; and the light chain variable region comprising: a complementarity determining region light chain 1 (CDRL1) amino acid sequence of SEQ ID NO:4; a CDRL2 amino acid sequence of SEQ ID NO:5; and a CDRL3 amino acid sequence of SEQ ID NO:6; (ii) a heavy chain variable region of the amino acid sequence of SEQ ID NO:7 and a light chain variable region of the amino acid sequence of SEQ ID NO:8; or (iii) a heavy chain of the amino acid sequence of SEQ ID NO:10 and a light chain of the amino acid sequence of SEQ ID NO:11; wherein the antibody is provided in packaging comprising one or more drug product label elements disclosed in Annex I including data from a randomized, double-blind, placebo-controlled, clinical study in adult men and women with moderately to severely active ulcerative colitis (UC).
3. The pharmaceutical composition of embodiment 2, wherein the pharmaceutical composition is for intravenous administration and comprises a solution comprising 10 mM L-histidine, 8.5% (w/v) sucrose, 0.04% (w/v) polysorbate 80, 0.4 mg/mL L-methionine, and 20 µg/mL EDTA disodium salt, dehydrate, at pH 6.0.
4. The pharmaceutical composition of embodiment 2, wherein the pharmaceutical composition is for subcutaneous administration and comprises a solution comprising 6.7 mM L-histidine, 7.6% (w/v) sucrose, 0.004% (w/v) polysorbate 80, at pH 6.0.
5. A method of treating moderately to severely active ulcerative colitis (UC) in a subject in need thereof, comprising administering to the subject the pharmaceutical composition of embodiment 2 in a clinically proven safe and clinically proven effective amount, wherein after treating with the antibody, the subject is a responder to treatment.
6. The method of embodiment 5, wherein the antibody is administered intravenously to the subject at week 0 of the treatment, at a dosage of about 6.0 mg/kg body weight of the subject or 130 mg per administration.
7. The method of embodiment 6, wherein the antibody is further administered subcutaneously to the subject at week 8 of the treatment, at a dosage of about 90 mg per administration.
8. The method of embodiment 7, wherein the subject had previously failed or were intolerant of at least one therapy selected from the group consisting of an anti-TNF, vedolizumab, corticosteroids, azathioprine (AZA), and 6 mercaptopurine (6 MP), or the subject had demonstrated corticosteroid dependence.
9. The method of embodiment 7, wherein the antibody is administered in a maintenance dose every 8 weeks after the treatment at week 8 or every 12 weeks after the treatment at week 8.
10. The method of embodiment 9, wherein the subject is identified as having a clinical remission based on at least one of the global definition and the US definition by week 16, preferably by week 8 of the treatment and the clinical remission continues at least 44 weeks after week 0.
11. The method of embodiment 9, wherein the subject is in corticosteroid-free clinical remission at least 44 weeks after week 0.
12. The method of embodiment 9, wherein the subject is identified as having an endoscopic healing continuing at least 44 weeks after week 0.
13. The method of embodiment 9, wherein the subject is identified as achieving a clinical response based on the Mayo endoscopy subscore continuing at least 44 weeks after week 0.
14. The method of embodiment 9, wherein the subject is identified as having a change from baseline in Inflammatory Bowel Disease Questionnaire (IBDQ) score continuing at least 44 weeks after week 0.
15. The method of embodiment 9, wherein the subject is identified as having a mucosal healing continuing at least 44 weeks after week 0.
16. The method of embodiment 9, wherein the subject identified as having a decrease from baseline in Mayo score continuing at least 44 weeks after week 0.
17. The method of embodiment 9, wherein the subject is identified as having a normalization of one or more biomarkers selected from the group consisting of C-reactive protein, fecal lactoferrin and fecal calprotectin continuing at least 44 weeks after week 0.
18. The method of embodiment 9, wherein the subject is in clinical response as determined by a decrease from baseline in the Mayo score by ≥30% and ≥3 points and a decrease from baseline in the rectal bleeding subscore ≥1 points or a rectal bleeding subscore of 0 or 1 continuing at least 44 weeks after week 0.
19. A method of treating moderately to severely active ulcerative colitis (UC) in a subject in need thereof, comprising:
   A. intravenously administering to the subject at a dosage of about 6.0 mg/kg body weight of the subject or 130 mg per administration at week 0 of the treatment, a pharmaceutical composition of an antibody comprising: (i) a heavy chain variable region and a light chain variable region, the heavy chain variable region comprising: a complementarity determining region heavy chain 1 (CDRH1) amino acid sequence of SEQ ID NO:1; a CDRH2 amino acid sequence of SEQ ID NO:2; and a CDRH3 amino acid sequence of SEQ ID NO:3; and the light chain variable region comprising: a complementarity determining region light chain 1 (CDRL1) amino acid sequence of SEQ ID NO:4; a CDRL2 amino acid sequence of SEQ ID NO:5; and a CDRL3 amino acid sequence of SEQ ID NO:6; (ii) a heavy chain variable region of the amino acid sequence of SEQ ID NO:7 and a light chain variable region of the amino acid sequence of SEQ ID NO:8; or (iii) a heavy chain of the amino acid sequence of SEQ ID NO:10 and a light chain of the amino acid sequence of SEQ ID NO:11; wherein the pharmaceutical composition is provided in packaging comprising one or more drug product label elements disclosed in Annex I including data from a randomized, double-blind, placebo-controlled, clinical study in adult men and women with moderately to severely active ulcerative colitis (UC); and
   B. subcutaneously administering to the subject the pharmaceutical composition at a dosage of 90 mg per administration at week 8 of the treatment; and
   wherein the subject is a responder to treatment and had previously failed or was intolerant of at least one therapy selected from the group consisting of: an anti-TNF, vedolizumab, corticosteroids, azathioprine (AZA), and 6 mercaptopurine (6 MP), or the subject had demonstrated corticosteroid dependence.
20. The method of embodiment 19, wherein the pharmaceutical composition for intravenous administration comprises a solution comprising 10 mM L-histidine, 8.5% (w/v) sucrose, 0.04% (w/v) polysorbate 80, 0.4 mg/mL L-methionine, and 20 µg/mL EDTA disodium salt, dehydrate, at pH 6.0.
21. The method of embodiment 19, wherein the pharmaceutical composition for subcutaneous administration comprises a solution comprising 6.7 mM L-histidine, 7.6% (w/v) sucrose, 0.004% (w/v) polysorbate 80, at pH 6.0.
22. The method of embodiment 19, wherein the subject is identified as having a clinical remission based on at least one of the global definition and the US definition by week 16 of the treatment.
23. The method of embodiment 19, wherein the subject is identified as having an endoscopic healing by week 16 of the treatment.
24. The method of embodiment 19, wherein the subject is identified as achieving a clinical response based on the Mayo endoscopy subscore by week 16 of the treatment.
25. The method of embodiment 19, wherein the subject is identified as having a change from baseline in Inflammatory Bowel Disease Questionnaire (IBDQ) score by week 16 of the treatment.
26. The method of embodiment 19, wherein the subject is identified as having a mucosal healing by week 16 of the treatment.
27. The method of embodiment 19, wherein the subject is identified as having a decrease from baseline in Mayo score by week 16 of the treatment.
28. The method of embodiment 19, wherein the subject is identified as having a normalization of one or more biomarkers selected from the group consisting of C-reactive protein, fecal lactoferrin and fecal calprotectin by week 16 of the treatment.
29. The method of embodiment 19, wherein the subject is in clinical response as determined by a decrease from baseline in the Mayo score by ≥30% and ≥3 points and a decrease from baseline in the rectal bleeding subscore ≥1 points or a rectal bleeding subscore of 0 or 1 by week 16 of the treatment.
30. The method of embodiment 19, wherein the subject is not a responder to the treatment with the antibody by week 8 and is a responder to the treatment by week 16 of the treatment.
31. A method of treating moderately to severely active ulcerative colitis (UC) in a subject in need thereof, comprising:
   A. intravenously administering to the subject at a dosage of about 6.0 mg/kg body weight of the subject or 130 mg per administration at week 0 of the treatment, a pharmaceutical composition of an antibody comprising: (i) a heavy chain variable region and a light chain variable region, the heavy chain variable region comprising: a complementarity determining region heavy chain 1 (CDRH1) amino acid sequence of SEQ ID NO:1; a CDRH2 amino acid sequence of SEQ ID NO:2; and a CDRH3 amino acid sequence of SEQ ID NO:3; and the light chain variable region comprising: a complementarity determining region light chain 1 (CDRL1) amino acid sequence of SEQ ID NO:4; a CDRL2 amino acid sequence of SEQ ID NO:5; and a CDRL3 amino acid sequence of SEQ ID NO:6; (ii) a heavy chain variable region of the amino acid sequence of SEQ ID NO:7 and a light chain variable region of the amino acid sequence of SEQ ID NO:8; or (iii) a heavy chain of the amino acid sequence of SEQ ID NO: 10 and a light chain of the amino acid sequence of SEQ ID NO: 11, wherein the pharmaceutical composition is provided in packaging comprising one or more drug product label elements disclosed in Annex I including data from a randomized, double-blind, placebo-controlled, clinical study in adult men and women with moderately to severely active ulcerative colitis (UC); and
   B. subcutaneously administering to the subject a maintenance therapy of the pharmaceutical composition at a dosage of 90 mg per administration at week 8 of the treatment once every 8 weeks or once every 12 weeks after the administration at week 8, wherein the maintenance therapy is provided for 44 weeks and the subject is a responder to treatment.

## Claims

1. A pharmaceutical composition of an anti-IL-12/IL-23p40 antibody, comprising:
an antibody comprising: (i) a heavy chain variable region and a light chain variable region, the heavy chain variable region comprising: a complementarity determining region heavy chain 1 (CDRH1) amino acid sequence of SEQ ID NO: 1; a CDRH2 amino acid sequence of SEQ ID NO:2; and a CDRH3 amino acid sequence of SEQ ID NO:3; and the light chain variable region comprising: a complementarity determining region light chain 1 (CDRL1) amino acid sequence of SEQ ID NO:4; a CDRL2 amino acid sequence of SEQ ID NO:5; and a CDRL3 amino acid sequence of SEQ ID NO:6; (ii) a heavy chain variable region of the amino acid sequence of SEQ ID NO:7 and a light chain variable region of the amino acid sequence of SEQ ID NO:8; or (iii) a heavy chain of the amino acid sequence of SEQ ID NO: 10 and a light chain of the amino acid sequence of SEQ ID NO: 11; wherein the antibody is provided in packaging comprising one or more drug product label elements disclosed in Annex I including data from a randomized, double-blind, placebo-controlled, clinical study in adult men and women with moderately to severely active ulcerative colitis (UC).

2. The pharmaceutical composition of claim 1, wherein the antibody is a human IgG antibody; optionally wherein the antibody is a fully human IgG1κ monoclonal antibody.

3. The pharmaceutical composition of claim 1, wherein the antibody is ustekinumab.

4. The pharmaceutical composition of any preceding claim, wherein the pharmaceutical composition:
a) is for intravenous administration and comprises a solution comprising 10 mM L-histidine, 8.5% (w/v) sucrose, 0.04% (w/v) polysorbate 80, 0.4 mg/mL L-methionine, and 20 µg/mL EDTA disodium salt, dehydrate, at pH 6.0; or
b) is for subcutaneous administration and comprises a solution comprising 6.7 mM L-histidine, 7.6% (w/v) sucrose, 0.004% (w/v) polysorbate 80, at pH 6.0.

5. The pharmaceutical composition of any preceding claim, for use in a method of treating moderately to severely active ulcerative colitis (UC) in a subject in need thereof, wherein the method comprises administering to the subject the pharmaceutical composition in a clinically proven safe and clinically proven effective amount, wherein after treating with the antibody, the subject is a responder to treatment.

6. The pharmaceutical composition for use of claim 5, wherein the antibody is administered intravenously to the subject at week 0 of the treatment, at a dosage of about 6.0 mg/kg body weight of the subject or 130 mg per administration.

7. The pharmaceutical composition for use of claim 6, wherein the antibody is further administered subcutaneously to the subject at week 8 of the treatment, at a dosage of about 90 mg per administration.

8. The pharmaceutical composition for use of any one of claims 5-7, wherein the subject had previously failed or were intolerant of at least one therapy selected from the group consisting of an anti-TNF, vedolizumab, corticosteroids, azathioprine (AZA), and 6 mercaptopurine (6 MP), or the subject had demonstrated corticosteroid dependence.

9. The pharmaceutical composition for use of claim 7 or 8, wherein the antibody is administered in a maintenance dose every 8 weeks after the treatment at week 8 or every 12 weeks after the treatment at week 8.

10. The pharmaceutical composition for use of any one of claims 5-9, wherein the subject is:
A. identified as having a clinical remission based on at least one of the global definition and the US definition by week 16, preferably by week 8 of the treatment and the clinical remission continues at least 44 weeks after week 0;
B. in corticosteroid-free clinical remission at least 44 weeks after week 0;
C. identified as having an endoscopic healing continuing at least 44 weeks after week 0;
D. identified as achieving a clinical response based on the Mayo endoscopy subscore continuing at least 44 weeks after week 0;
E. identified as having a change from baseline in Inflammatory Bowel Disease Questionnaire (IBDQ) score continuing at least 44 weeks after week 0;
F. identified as having a mucosal healing continuing at least 44 weeks after week 0;
G. identified as having a decrease from baseline in Mayo score continuing at least 44 weeks after week 0;
H. identified as having a normalization of one or more biomarkers selected from the group consisting of C-reactive protein, fecal lactoferrin and fecal calprotectin continuing at least 44 weeks after week 0; or
I. in clinical response as determined by a decrease from baseline in the Mayo score by ≥30% and ≥3 points and a decrease from baseline in the rectal bleeding subscore ≥1 points or a rectal bleeding subscore of 0 or 1 continuing at least 44 weeks after week 0.

11. The pharmaceutical composition of any one of claims 1-4, for use in a method of treating moderately to severely active ulcerative colitis (UC) in a subject in need thereof, wherein the method comprises:
A. intravenously administering the pharmaceutical composition to the subject at a dosage of about 6.0 mg/kg body weight of the subject or 130 mg per administration at week 0 of the treatment; and
B. subcutaneously administering to the subject the pharmaceutical composition at a dosage of 90 mg per administration at week 8 of the treatment;
wherein the subject is a responder to treatment and had previously failed or was intolerant of at least one therapy selected from the group consisting of: an anti-TNF, vedolizumab, corticosteroids, azathioprine (AZA), and 6 mercaptopurine (6 MP), or the subject had demonstrated corticosteroid dependence.

12. The pharmaceutical composition for use of claim 11, wherein:
a) the pharmaceutical composition for intravenous administration comprises a solution comprising 10 mM L-histidine, 8.5% (w/v) sucrose, 0.04% (w/v) polysorbate 80, 0.4 mg/mL L-methionine, and 20 µg/mL EDTA disodium salt, dehydrate, at pH 6.0; and/or
b) the pharmaceutical composition for subcutaneous administration comprises a solution comprising 6.7 mM L-histidine, 7.6% (w/v) sucrose, 0.004% (w/v) polysorbate 80, at pH 6.0.

13. The pharmaceutical composition for use of claim 11 or 12, wherein the subject is:
A. identified as having a clinical remission based on at least one of the global definition and the US definition by week 16 of the treatment;
B. identified as having an endoscopic healing by week 16 of the treatment;
C. identified as achieving a clinical response based on the Mayo endoscopy subscore by week 16 of the treatment;
D. identified as having a change from baseline in Inflammatory Bowel Disease Questionnaire (IBDQ) score by week 16 of the treatment;
E. identified as having a mucosal healing by week 16 of the treatment;
F. identified as having a decrease from baseline in Mayo score by week 16 of the treatment;
G. identified as having a normalization of one or more biomarkers selected from the group consisting of C-reactive protein, fecal lactoferrin and fecal calprotectin by week 16 of the treatment;
H. in clinical response as determined by a decrease from baseline in the Mayo score by ≥30% and ≥3 points and a decrease from baseline in the rectal bleeding subscore ≥1 points or a rectal bleeding subscore of 0 or 1 by week 16 of the treatment; or
I. not a responder to the treatment with the antibody by week 8 and is a responder to the treatment by week 16 of the treatment.

14. The pharmaceutical composition of any one of claims 1-4, for use in a method of treating moderately to severely active ulcerative colitis (UC) in a subject in need thereof, wherein the method comprises:
A. intravenously administering the pharmaceutical composition to the subject at a dosage of about 6.0 mg/kg body weight of the subject or 130 mg per administration at week 0 of the treatment,; and
B. subcutaneously administering to the subject a maintenance therapy of the pharmaceutical composition at a dosage of 90 mg per administration at week 8 of the treatment once every 8 weeks or once every 12 weeks after the administration at week 8, wherein the maintenance therapy is provided for 44 weeks and the subject is a responder to treatment.

15. The antibody for use of any of claims 5-14, wherein the intravenous dose is 260 mg for subjects with body weight ≥35 kg and ≤55 kg, 390 mg for subjects with body weight >55 kg and ≤85 kg, and 520 mg for subjects with body weight >85 kg.
